# EUROPEAN PATENT SPECIFICATION

(11) **EP 3 093 343 B1**
(45) Date of publication and mention of the grant of the patent: **01.01.2020**
(21) Application number: 15735080.2
(22) Date of filing: 09.01.2015
(51) Int. Cl.: C12Q 1/6886

(54) **METHOD FOR ASSESSING LYMPH NODE METASTATIC POTENTIAL OF ENDOMETRIAL CANCER**
VERFAHREN ZUR BEURTEILUNG DES METASTATISCHEN POTENZIALS VON LYMPHKNOTEN VON ENDOMETRIUMKREBS
MÉTHODE D'ÉVALUATION DU POTENTIEL MÉTASTATIQUE DU CANCER DE L'ENDOMÈTRE EN DIRECTION DES GANGLIONS LYMPHATIQUES

(30) Priority: 10.01.2014 JP 2014003191
(43) Date of publication of application: 16.11.2016
(73) Proprietor: Juntendo Educational Foundation, Tokyo 113-8421 (JP); Kanagawa Prefectural Hospital Organization, Yokohama-shi, Kanagawa 2310005 (JP)
(72) Inventor: TERAO, Yasuhisa, Tokyo 113-8421 (JP); TAKEDA, Satoru, Tokyo 113-8421 (JP); HAYASHIZAKI, Yoshihide, Wako-shi Saitama 351-0198 (JP); ITO, Masayoshi, Wako-shi Saitama 351-0198 (JP); KAWAJI, Hideya, Wako-shi Saitama 351-0198 (JP); OOMIYA, Hiroko, Wako-shi Saitama 351-0198 (JP); KATO, Hisamori, Yokohama-shi Kanagawa 241-8515 (JP); MIYAGI, Yohei, Yokohama-shi Kanagawa 241-8515 (JP); OHTSU, Takashi, Yokohama-shi Kanagawa 241-8515 (JP)
(74) Representative: Schiener, Jens
(86) International application number: PCT/JP2015/050551
(87) International publication number: WO 2015/105190

(56) References cited:
- WO-A1-2005/061725
- WO-A1-2006/016110
- WO-A1-2012/019300
- WO-A2-03/029273
- WO-A2-2005/024064
- WO-A2-2011/039734
- DE-A1- 10 260 928
- US-A1- 2005 221 398
- SATOKO SUDO ET AL.: 'Shikyu Taigan Lymph Setsu Ten'i Biomarker no Tansaku' THE JOURNAL OF THE JAPAN SOCIETY OF GYNECOLOGIC ONCOLOGY vol. 29, no. 3, 2011, page 500, XP008184278
- SATOKO SUDO ET AL.: 'Shikyu Taigan Lymph Setsu Ten'i Biomarker no Tansaku' ACTA OBSTETRICA ET GYNAECOLOGIA JAPONICA vol. 63, no. 2, 2011, page 793, XP008184118
- SATOKO SUDO ET AL.: 'Shikyu Taigan Lymph Setsu Ten'i Biomarker no Tansaku' THE JOURNAL OF THE JAPAN SOCIETY OF GYNECOLOGIC ONCOLOGY vol. 27, no. 3, 2009, pages 313 , P 5 - 8, XP008184277
- TROVIK J. ET AL.: 'Stathmin overexpression identifies high-risk patients and lymph node metastasis in endometrial cancer.' CLIN. CANCER RES. vol. 17, no. 10, 2011, pages 3368 - 3377, XP055020515
- FISHMAN A. ET AL.: 'Detection of micrometastasis by cytokeratin-20 (reverse transcription polymerase chain reaction) in lymph nodes of patients with endometrial cancer.' GYNECOL.ONCOL. vol. 77, no. 3, 2000, pages 399 - 404, XP055356963

## Description

### Field of the Invention

### (Related Application)

The present application claims the priority of Japanese Patent Application No. 2014-003191 filed on January 10, 2014.

The present invention relates to an approach for discriminating at a molecular level between endometrial cancer with lymph node metastasis and endometrial cancer without metastasis.

### Background of the Invention

The difference between the presence and absence of lymph node metastasis in early endometrial cancer (well-differentiated adenocarcinoma) cases is considered to be brought about by abnormal transcription in tumors at primary lesions. Some cases of endometrial cancer type 1 well-differentiated adenocarcinoma are positive to metastasis to the lymph node, in spite of early cancer without muscle invasion. However, a great majority of cases of well-differentiated endometrial adenocarcinoma are negative to lymph node metastasis, even if muscle invasion is found. Nonetheless, unnecessary lymph node dissection is practiced under the current circumstance.

Heretofore, lymph node metastatic potential of endometrial cancer has been examined only by actually practicing lymph node dissection and pathologically confirming the presence or absence of a cancer tissue that has metastasized in the harvested lymph nodes. An approach for determining whether or not endometrial cancer is with lymph node metastasis without lymph node dissection has been totally unknown so far.

Meanwhile, in recent years, an approach for gene expression analysis has been developed which involves comprehensively analyzing genes expressed in cells in a certain state by the comparison of the expression statuses of the genes, and comparing their types or expression levels among the cells. For example, RNA-seq (Non Patent Literature 1) and CAGE (cap analysis gene expression; Non Patent Literature 2) are known to comprehensively analyze the expression statuses of genes at transcription start sites as sequence information. Of these methods, CAGE is characterized in that this method is capable of comprehensively quantifying the activity of transcription start points by selecting long capped RNAs such as mRNA and sequencing their 5' ends at random and at a large scale.

However, none of the previous reports mention the relation of the expression level of a transcription start site in the human genome to a particular disease.

### Citation List

### Non Patent Literature

Non Patent Literature 1: Nature Reviews Genetics 10 (1): 57-63
Non Patent Literature 2: Genome Res. 2011 Jul; 21 (7): 1150-9

Trovik J. et al. Clin. Cancer Res., 2011, 17 (10): 3368-3377 discloses that stathmin overexpression identifies high-risk patients and lymph node metastasis in endometrial cancer.

### Summary of the Invention

### Problems to be Solved by the Invention

The present invention relates to provide an approach for discriminating at a molecular level between endometrial cancer with lymph node metastasis and endometrial cancer without metastasis.

### Means for Solving the Problems

The present inventors have extracted RNA from cancer tissues with lymph node metastasis and cancer tissues without lymph node metastasis collected from endometrial cancer patients, and comprehensively analyzed their expression statuses near transcription start sites (TSSs) as sequence information by the CAGE analysis method. As a result, the present inventors have found that the expression level of DNA containing a particular transcription start site significantly differ between the group with lymph node metastasis and the group without lymph node metastasis, and this difference can be used as an index to discriminate between endometrial cancer with lymph node metastasis and endometrial cancer without metastasis.

The present invention relates to the subject matter of claims 1 to 9. Specifically, the present invention relates to the following 1) to 4):
1) A method for assessing lymph node metastasis or lymph node metastatic potential of endometrial cancer, comprising the step of measuring an expression level of an expression product of at least one DNA comprising a transcription start site in a biological sample derived from a cancer tissue isolated from an endometrial cancer patient, wherein
   the DNA comprises a base at an arbitrary position of a region, wherein both ends of said region are defined by the first base in the nucleotide sequence represented by SEQ ID NO: 264 and the 101st base counted from the 3' end of SEQ ID NO: 264, and 20 or more bases located immediately downstream thereof.
2) Use of a testing kit for assessing lymph node metastasis or lymph node metastatic potential of endometrial cancer in the method according to 1), the testing kit comprising an oligonucleotide specifically hybridizing to a transcription product of the DNA, or an antibody recognizing a translation product of the DNA.
3) Use of an expression product of at least one DNA as a marker for assessing lymph node metastasis or lymph node metastatic potential of endometrial cancer, wherein the DNA comprises a base at an arbitrary position of a region, wherein both ends of said region are defined by the first base in the nucleotide sequence represented by SEQ ID NO: 264 and the 101st base counted from the 3' end of SEQ ID NO: 264, and 20 or more bases located immediately downstream thereof.
4) A method for assessing lymph node metastasis or lymph node metastatic potential of endometrial cancer, comprising the step of measuring an expression level of TACC2 RNA or TACC2 protein in a biological sample derived from a cancer tissue isolated from an endometrial cancer patient.

### Effects of the Invention

According to the present invention, an endometrial cancer specimen having no chance to metastasize to the lymph nodes can be selected. This suggests a policy regarding operative procedures, also including the avoidance of unnecessary lymph node dissection. Provided that the lymph node dissection can be avoided, shortening of operative duration, mitigation of operative invasion, and prevention of postoperative lymphedema or the like can be achieved. Also, use of the present invention allows lymph node metastasis or lymph node metastatic potential of endometrial cancer to be objectively classified at a level equivalent to or higher than the subjectivity of specialists such as well-trained clinical laboratory technicians. The present invention can also be suitably used in point of care testing (POCT) from the collection of specimens from patients to the analysis thereof.

### Brief Description of the Drawings

[Figure 1] Figure 1 shows immunohistochemical images. (Left image) With metastasis, dark brown: TACC2-expressing cells, magnification: ×400, (Right image) Without metastasis, magnification: ×400.
[Figure 2] Figure 2 is a graph showing the RNA expression level of TACC2.

### Modes for Carrying out the Invention

In the present invention, the endometrial cancer means a cancer which develops in the uterine corpus among uterine cancers. The endometrial cancer arises from the endometrium, which is an epithelial tissue lining the cavity of the uterus, and is also called uterine body cancer or uterine corpus cancer, etc. The stages of the endometrial cancer are classified into I to IV according to the Japan Society of Obstetrics and Gynecology (JSOG) classification of clinical stages (2011) in view of the tumor size, spread, and invasion or metastasis statuses of the cancer. In the present invention, the endometrial cancer to be assessed is not particularly limited by its stage and is preferably well-differentiated adenocarcinoma of endometrioid adenocarcinoma.

In the present invention, the lymph node metastasis means that the endometrial cancer grows in, for example, the intrapelvic or para-aortic lymph nodes.

In the present invention, the assessment of lymph node metastasis means that the endometrial cancer is evaluated or assayed for the presence or absence of its lymph node metastasis. The assessment of lymph node metastatic potential (lymph node metastatic property) means that the endometrial cancer is evaluated or assayed for whether or not to have the ability to metastasize to the lymph nodes and grow therein.

The biological sample used in the present invention is an endometrial cancer tissue isolated before or during operation from an endometrial cancer patient to be assessed. The biological sample is appropriately prepared and/or treated for assay. For example, in the case of assaying the sample at a nucleic acid level, RNA extracts are prepared. In the case of assaying the sample at a protein level, protein extracts are prepared.

Any method known in the art can be used as a method for extracting RNA from the biological sample. Specific examples thereof can include Ambion RiboPure kit (manufactured by Life Technologies Corp.), miRNeasy (manufactured by Qiagen N.V.), and RNeasy (manufactured by Qiagen N.V.). Of them, miRNeasy kit manufactured by Qiagen N.V. is preferably used.

In the present specification, the term "nucleic acid" or "polynucleotide" means DNA or RNA. The "DNA" encompasses not only double-stranded DNA but each single-stranded DNA as a sense strand and an antisense strand constituting the double-stranded DNA. Thus, the DNA encompasses, for example, double-stranded genomic DNA, single-stranded cDNA, and single-stranded DNA having a sequence complementary to the DNA. The "RNA" includes all of total RNA, mRNA, rRNA, and synthetic RNA.

In the present invention, transcription products of DNAs consisting of nucleotide sequences represented by SEQ ID NOs: 1 to 277 (human genomic DNAs each consisting of a transcription start site and 100 bases located immediately downstream thereof) have been confirmed, as shown in Examples, to significantly differ in their expression levels (transcriptional activity) between endometrial cancer with lymph node metastasis and endometrial cancer without metastasis, as a result of comprehensively analyzing the expression statuses of DNAs each comprising a transcription start site and 100 or more downstream bases on the genome by use of the CAGE (cap analysis gene expression) analysis method on endometrial cancer specimens without lymph node metastasis and endometrial cancer specimens with lymph node metastasis. Specifically, these transcription products were extracted by differential analysis on the transcriptional activity of RNA between a profile group derived from clinical specimens obtained from subjects "with lymph node metastasis" and a profile group derived from clinical specimens obtained from subjects "without lymph node metastasis" using R/Bioconductor edgeR package (Bioinformatics. 2010 Jan 1; 26 (1): 139-40) with a threshold set to FDR (false discovery rate) of 5%.

Thus, an expression product of (or encoded by) DNA comprising a base at an arbitrary position (transcription start point) in the transcription start site in any of the nucleotide sequences represented by SEQ ID NOs: 1 to 277 and one or more bases located immediately downstream thereof (hereinafter, this DNA is referred to as "DNA containing a transcription start point in SEQ ID NOs: 1 to 277") (hereinafter, this expression product is referred to as the "expression product of the present invention") can serve as a biomarker for discriminating between endometrial cancer with lymph node metastasis and endometrial cancer without metastasis. The expression product of the DNA containing a transcription start point in SEQ ID NOs: 1 to 275 is a marker whose expression level is increased in the case of having lymph node metastasis. The expression product of the DNA containing a transcription start point in SEQ ID NOs: 276 to 277 is a marker whose expression level is decreased in the case of having lymph node metastasis.

In the present invention, the "transcription start site" refers to a region containing transcription start points. The transcription start points from a particular promoter are not limited to single bases and may be bases located at a plurality of positions downstream of the promoter on the genome. In the present specification, the region containing these plurality of transcription start points is referred to as a transcription start site. More specifically, the transcription start site is a region between a transcription start point positioned closest to the 5' end and a transcription start point positioned closest to the 3' end, among the plurality of transcription start points. In each of the nucleotide sequences represented by SEQ ID NOs: 1 to 277, the transcription start site is a 5'-terminal base region which corresponds to a region wherein both ends thereof are defined by a base at position 1 (5' end) and the 101st base counted from the 3' end. In other words, each of the nucleotide sequences represented by SEQ ID NOs: 1 to 277 is indicated by the transcription start site and 100 bases following the transcription start point positioned closest to the 3' end in the transcription start site. In the present specification, such a transcription start site is also referred to as a "transcription start site shown in SEQ ID NOs: 1 to 277".

The position of the transcription start site shown in SEQ ID NOs: 1 to 277 on the genome, and gene information related thereto, etc., are as shown later in Tables 1-1 to 1-12.

In the present invention, the DNA to be assayed for the expression level of the expression product comprises a base at an arbitrary position of a region, wherein both ends of said region are defined by the first base in the nucleotide sequence represented by SEQ ID NO: 264 and the 101st base counted from the 3' end of SEQ ID NO: 264, and 20 or more bases located immediately downstream thereof.

In this context, the number of bases in the nucleotide sequence immediately downstream thereof can be 20 or more bases, 25 or more bases, 30 or more bases, 40 or more bases, and 50 or more bases. Also, examples of the number of the bases include 25 or less bases, 30 or less bases, 40 or less bases, 50 or less bases, and 100 or less bases.

The downstream bases can be any downstream moiety up to approximately 100 bases for securing the accuracy of assay based on hybridization or PCR, though this is not particularly required for CAGE assay. A length of at least 20 or more bases in the DNA consisting of the transcription start site and 100 bases downstream thereof can be identified with high probability even in an experimental system targeting the whole genome.

The DNA also encompasses DNA having a nucleotide sequence substantially identical to the nucleotide sequence of the DNA as long as its expression product can serve as a biomarker for discriminating between endometrial cancer with lymph node metastasis and endometrial cancer without metastasis. In this context, the substantially identical nucleotide sequence means that the nucleotide sequence has 90% or higher, preferably 95% or higher, more preferably 98% or higher identity with nucleotide sequence 264 when searched using, for example, a homology calculation algorithm NCBI BLAST under conditions involving expected value = 10, gap accepted, filtering = ON, match score = 1, and mismatch score = -3.

The expression product of the present invention is capable of discriminating between endometrial cancer with lymph node metastasis and endometrial cancer without metastasis by determining the expression level of this expression product alone or combined with the other expression product(s) of the present invention. Among others, the expression products of DNAs containing transcription start points in SEQ ID NO: 208, SEQ ID NO: 264, SEQ ID NO: 237, SEQ ID NO: 270, SEQ ID NO: 57, SEQ ID NO: 274, SEQ ID NO: 108, and SEQ ID NO: 182 permit classification with 100% specificity and 100% sensitivity when the thresholds shown in Table 2 are established. Specifically, the expression level of even only one of these expression products achieves reliable discrimination.

In the case of confirming the expression levels of a plurality of expression products in combination, the number thereof and the contents regarding the combination can be appropriately selected. Preferred combinations (434 sets) of at least two DNA expression products used are shown later in Table 3. These combinations were extracted as follows: as for every combination of two transcription start sites selected from the 277 transcription start sites described above, logistic regression models for estimating the presence or absence of lymph node metastasis were constructed for samples for transcription start site extraction with their expression levels as explanatory variables; and the extraction was performed such that both of the samples for transcription start site extraction and samples for validation can be classified with 100% specificity and 100% sensitivity. Two sets or 3 or more sets of these combinations can be appropriately used in combination for the purpose of further improving accuracy. In addition to the combination(s) of these two DNA expression products, the expression product of the DNA containing a transcription start point in SEQ ID NOs: 1 to 277 may be combined with the expression product of DNA other than its partner shown in Tables 3-1 to 3-6. Alternatively, the expression product of the present invention may be combined with an expression product of DNA consisting of any of the other nucleotide sequences as long as this combination can contribute to the assessment of the present invention.

Examples of the expression product of the present invention include a transcription product and a translation product expressed from the DNA. Specific examples of the transcription product include RNA transcribed from the DNA, preferably mRNA. Specific examples of the translation product include a protein encoded by the RNA. For example, a protein expressed from DNA containing a transcription start point in SEQ ID NO: 264, among the expression products of the DNAs containing a transcription start point as shown in Table 2, has been identified as "TACC2" (transforming, acidic coiled-coil containing protein 2; UniProtKB/Swiss-Prot: TACC2_HUMAN, 095359).

The target in the assay or detection of the expression product also encompasses, for example, cDNA artificially synthesized from the RNA, DNA encoding the RNA, a protein encoded by the RNA, a molecule interacting with the protein, a molecule interacting with the RNA, or a molecule interacting with the DNA. In this context, examples of the molecule interacting with the RNA, the DNA, or the protein include DNA, RNA, proteins, polysaccharides, oligosaccharides, monosaccharides, lipids, fatty acids, and phosphorylation products, alkylation products, or glycosylation products thereof, and complexes of any of these molecules.

The expression level collectively means the expression amount and activity of the expression product.

The method for measuring the expression level of RNA, cDNA, or DNA to be assayed can be selected from nucleic acid amplification methods typified by PCR using DNA primers hybridizing thereto, real-time RT-PCR, SmartAmp, and LAMP, hybridization methods (DNA chips, DNA microarrays, dot blot hybridization, slot blot hybridization, Northern blot hybridization, etc.) using nucleic acid probes hybridizing thereto, sequencing methods, and combinations of these methods.

In this context, the probe or the primer for use in the assay corresponds to a primer for specifically recognizing and amplifying the expression product of the present invention (transcription product) or a nucleic acid derived therefrom, or a probe for specifically detecting the RNA or a nucleic acid derived therefrom. These can be designed on the basis of the nucleotide sequence represented by SEQ ID NO: 264. In this context, the phrase "specifically recognizing" means that substantially only the expression product of the present invention (transcription product) or a nucleic acid derived therefrom can be detected, for example, in Northern blot, and substantially the detected matter or the product can be determined as the transcription product or a nucleic acid derived therefrom, for example, in RT-PCR, in such a way that substantially only the nucleic acid is formed.

Specifically, an oligonucleotide comprising a given number of nucleotides complementary to the DNA comprising any of the nucleotide sequence represented by SEQ ID NO: 264 or a complementary strand thereof can be used. In this context, the "complementary strand" refers to another strand against one strand of double-stranded DNA composed of A:T (U for RNA) and G:C base pairs. The term "complementary" is not limited by a completely complementary sequence in a region with the given number of consecutive nucleotides and may only have preferably 80% or higher, more preferably 90% or higher, even more preferably 95% or higher nucleotide sequence identity. The nucleotide sequence identity can be determined by an algorithm such as BLAST described above.

For use as a primer, such an oligonucleotide is not particularly limited as long as the oligonucleotide is capable of specific annealing and strand elongation. Examples thereof include oligonucleotides usually having a chain length of, for example, 10 or more bases, preferably 15 or more bases, more preferably 20 or more bases, and, for example, 100 or less bases, preferably 50 or less bases, more preferably 35 or less bases. For use as a probe, the oligonucleotide is not particularly limited as long as the oligonucleotide is capable of specific hybridization. An oligonucleotide having at least a portion or the whole sequence of the DNA comprising any of the nucleotide sequence represented by SEQ ID NO: 264 (or a complementary strand thereof) and having a chain length of, for example, 10 or more bases, preferably 15 or more bases, and, for example, 100 or less bases, preferably 50 or less bases, more preferably 25 or less bases is used.

In this context, the "oligonucleotide" can be DNA or RNA and may be synthetic or natural. The probe for use in hybridization is usually labeled and then used.

For example, in the case of utilizing Northern blot hybridization, first, probe DNA is labeled with a radioisotope, a fluorescent material, or the like, and the obtained labeled DNA is subsequently hybridized with biological sample-derived RNA transferred to a nylon membrane or the like according to a routine method.
Then, the formed double strand of the labeled DNA and the RNA can be confirmed by use of a method for detecting and measuring a signal derived from the label.

In the case of utilizing RT-PCR, first, cDNA is prepared from biological sample-derived RNA according to a routine method. This cDNA is used as a template and hybridized with a pair of primers (a forward strand binding to the cDNA (- strand) and a reverse strand binding to the + strand) prepared so as to be capable of amplifying the target expression product of the present invention (in this case, a transcription product). Then, PCR is performed according to a routine method, and the obtained amplified double-stranded DNA is detected. The detection of the amplified double-stranded DNA can employ, for example, a method which involves detecting labeled double-stranded DNA produced by PCR described above using primers labeled in advance with RI, a fluorescent material, or the like.

In the case of measuring the expression level of mRNA in a specimen using a DNA microarray, an array in which at least one nucleic acid (cDNA or DNA) derived from the expression product of the present invention (in this case, a transcription product) is immobilized on a support is used. Labeled cDNA or cRNA prepared from the mRNA is allowed to bind onto the microarray. The mRNA expression level can be measured by detecting the label on the microarray.

The nucleic acid to be immobilized on the array can be a nucleic acid capable of specific hybridization (i.e., hybridization substantially only to the nucleic acid of interest) under stringent conditions and may be, for example, a nucleic acid having the whole sequence of the expression product of the present invention (transcription product) or may be a nucleic acid consisting of a partial sequence thereof. In this context, examples of the "partial sequence" include a nucleic acid consisting of at least 15 to 25 bases.

In this context, examples of the stringent conditions can typically include washing conditions on the order of "1 × SSC, 0.1% SDS, 37°C" and can include more stringent hybridization conditions on the order of "0.5 × SSC, 0.1% SDS, 42°C" and further stringent hybridization conditions on the order of "0.1 × SSC, 0.1% SDS, 65°C". The hybridization conditions are described in, for example, J. Sambrook et al., Molecular Cloning: A Laboratory Manual, Third Edition, Cold Spring Harbor Laboratory Press (2001).

Examples of the sequencing method include CAGE, TSS-seq, RNA-seq, DGE, and SAGE. CAGE is preferred.

In the case of measuring the expression level by use of CAGE, this measurement can be carried out according to a method described later in Examples.

In the case of assaying the protein (translation product) encoded by the DNA containing a transcription start point in SEQ ID NO: 264, the molecule interacting with the protein, the molecule interacting with the RNA, or the molecule interacting with the DNA, a method such as protein chip analysis, immunoassay (e.g., ELISA), one-hybrid method (PNAS 100, 12271-12276 (2003)), or two-hybrid method (Biol. Reprod. 58, 302-311 (1998)) can be used and can be appropriately selected according to the target.

In the case of assaying, for example, the protein used as a target, this assay is carried out by contacting an antibody against the expression product of the present invention (in this case, a translation product) with the biological sample, detecting the antibody-bound polypeptide in the sample, and measuring the level thereof. According to, for example, Western blot, the antibody described above is used as a primary antibody. Then, for example, radioisotope-, fluorescent material- or enzyme-labeled antibody binding to the primary antibody is used as a secondary antibody to label the primary antibody. A signal derived from such a labeling material is measured using a radiation counter, a fluorescence detector, or the like.

The antibody against the translation product may be a polyclonal antibody or may be a monoclonal antibody. These antibodies can be produced according to methods known in the art. Specifically, the polyclonal antibody can be obtained according to a routine method from the serum of an immunized animal obtained by immunizing a nonhuman animal (e.g., a rabbit) with a protein expressed in *E. coli* or the like and purified according to a routine method or with a partial polypeptide of the protein synthesized according to a routine method.

On the other hand, the monoclonal antibody can be obtained from hybridoma cells prepared by immunizing a nonhuman animal (e.g., a mouse) with a protein expressed in E. coli or the like and purified according to a routine method or with a partial polypeptide of the protein and fusing the obtained spleen cells with myeloma cells. Alternatively, the monoclonal antibody may be prepared by use of phage display (Griffiths, A.D.; Duncan, A.R., Current Opinion in Biotechnology, Volume 9, Number 1, February 1998, pp. 102-108 (7)).

In the case of conducting an immunohistochemical analysis method, the biological sample isolated from a patient is fixed in formalin by a routine method, then embedded in paraffin, and sliced into a tissue section, which is attached to slide glass. The resultant is preferably used as a section sample. An antibody labeled with an enzyme such as alkaline phosphatase or peroxidase can be used as the secondary antibody. Highly sensitive detection is preferably performed using, for example, a three-step method such as ABC or LSAB, or DAKO EnVision detection system.

In this way, the expression level of the expression product of the present invention in the biological sample derived from a cancer tissue isolated from an endometrial cancer patient is measured. The presence or absence of lymph node metastasis or lymph node metastatic potential is assessed on the basis of the expression level. Specifically, the detected expression level of the expression product of the present invention is compared with a control level for the assessment.

In this context, examples of the "control level" include the expression level of the expression product in an endometrial cancer tissue isolated from endometrial cancer patient without lymph node metastasis or in a normal tissue isolated from the endometrial cancer patient, and the expression level of the expression product in a healthy individual group having no endometrial cancer.

For example, when the expression level of the expression product in the cancer tissue of the subject patient is close to the expression level in an endometrial cancer tissue isolated from an endometrial cancer patient without lymph node metastasis, a normal tissue, or a tissue derived from a healthy individual, when the expression level of the expression product in the cancer tissue of the subject patient belongs to within the range of this expression level, or when the expression level of the expression product in the cancer tissue of the subject patient is significantly higher (or lower) than this expression level, the endometrial cancer of the patient can be assessed as having no lymph node metastasis or low lymph node metastatic potential.

The assessment of lymph node metastasis or lymph node metastatic potential of endometrial cancer according to the present invention can also be conducted on the basis of increase or decrease in the expression level of the expression product of the present invention. In this case, a reference value (threshold level) is established on the basis of the control level, for example, the expression level of the expression product derived from a normal tissue, an endometrial cancer tissue isolated from an endometrial cancer patient without lymph node metastasis, or a tissue of a healthy individual. The assessment can be conducted by comparing the expression level of the expression product in the patient-derived biological sample with the reference value (e.g., a range of ± 2S.D. is used as a tolerance). For example, when the expression level of the expression product in the patient-derived biological sample is higher or lower than the threshold level, the endometrial cancer of the patient can be assessed as having no lymph node metastasis or low lymph node metastatic potential.

The possibility of lymph node metastasis or lymph node metastatic potential is determined on the basis of information provided according to the method of the present invention, if necessary combined with an additional method (CT, MRI, PET-CT, etc.). Although criteria for determining whether to perform lymph node biopsy or lymph node dissection are left to physician's judgment, for example, lymph node dissection can be performed for a patient with endometrial cancer confirmed to have the possibility of lymph node metastasis or high lymph node metastatic potential. On the other hand, it is considered that lymph node dissection does not have to be performed for a patient with endometrial cancer confirmed to have no possibility of lymph node metastasis or low lymph node metastatic potential.

The testing kit for assessing lymph node metastasis or lymph node metastatic potential of endometrial cancer according to the present invention comprises a testing reagent for measuring the expression level of the expression product of the present invention in the biological sample isolated from a patient. Specific examples thereof include a reagent for nucleic acid amplification or hybridization comprising an oligonucleotide specifically binding (hybridizing) to the expression product of the present invention (transcription product) or the like, and a reagent for immunoassay comprising an antibody recognizing the expression product of the present invention (translation product). The oligonucleotide, the antibody, or the like included in the kit can be obtained by a method known in the art as mentioned above.

The testing kit can further comprise a labeling reagent, a buffer solution, a chromogenic substrate, a secondary antibody, a blocking agent, and equipment or a control necessary for the test, in addition to the antibody or the nucleic acid.

### Examples

### Example 1 Extraction and validation of transcription start site which permits discrimination between endometrial cancer metastatic to lymph nodes and endometrial cancer without metastasis

### (1) Acquisition of test sample

5 mm each of endometrial tumor lesions was collected from operatively harvested specimens of patients with endometrial cancer (well-differentiated adenocarcinoma) having 1/2 or less myometrial invasion, under their comprehensive consent before operation. The samples used were 10 specimens (3 specimens with lymph node metastasis and 7 specimens without lymph node metastasis) as samples for transcription start site extraction and 5 specimens (2 specimens with lymph node metastasis and 3 specimens without lymph node metastasis) as samples for validation.

### (2) Preservation and preparation of sample

Each harvested tissue section was appropriately frozen and preserved at -80°C. The preserved tissue section was placed in a 2 mL microtube such that the amount of the tissue section was 50 mg or less. QIAzol (manufactured by Qiagen N.V.) was added to the microtube, and one zirconia bead was placed therein. After hermetically sealing of the tube, the tissue section was lysed by penetration treatment using TissueLyser (manufactured by Qiagen N.V.).

### (3) Preparation of RNA

Each sample thus treated by lysis and extraction was subjected to RNA preparation using miRNeasy mini kit (manufactured by Qiagen N.V.) according to the protocol included in the kit. The RNA thus prepared was assayed for ultraviolet absorption (230, 260, and 280 nm) using a spectrophotometer, and 260/230 and 260/280 ratios were calculated to test the quality of the RNA. Furthermore, the RNA was electrophoresed using BioAnalyzer RNA nano chip (manufactured by Agilent Technologies Inc.), and RIN values indicating the degree of RNA degradation were calculated to test the degree of degradation of the RNA.

### (4) Preparation of CAGE library

5 µg of each purified RNA was used to prepare a CAGE library by no-amplification non-tagging CAGE (see "Cell Technology, suppl. Purpose-specific advanced methods of next-generation sequencers", edited by Sumio Sugano and Yutaka Suzuki, Gakken Medical Shujunsha Co., Ltd., issued on September 19, 2012), Part 3-3, "Comprehensive promoter analysis (no-amplification CAGE using Illumina sequencer)"). Specifically, the purified RNA was subjected to reverse transcription reaction. After purification, diol in the ribose was oxidized with sodium periodate for conversion to aldehyde. The aldehyde group was biotinylated by the addition of biotin hydrazide. After digestion of the single-stranded RNA moiety with RNase I and purification, only the biotinylated RNA/cDNA double strand was allowed to bind to the surface of avidin magnetic beads, and cDNA was released by RNase H digestion and heat treatment and recovered. Both ends of the recovered cDNA were linked to adaptors necessary for sequencing, followed by sequencing using HiSeq 2500 (manufactured by Illumina, Inc.). The standard conditions of AMPure XP (manufactured by Beckman Coulter, Inc.) used in purification, buffer solution replacement, and the like in this step are conditions under which, in the case of double strand, nucleic acids of 100 or more bases long are recovered. The CAGE library produced by this step which adopted the conditions consisted of double-stranded DNAs each having a chain length of 100 or more bases.

### (5) RNA expression analysis

### i) Preparation of reference transcription start site

The reference transcription start sites were set to approximately 180,000 transcription start sites defined on the human reference genome hg19 among the transcription start sites identified in the profiling project "FANTOM5" (paper submitted) assaying in a genome-wide manner the activity of transcription start points as to human samples as many as approximately 1,000 samples in total including human primary cultured cells, cell lines, and tissues, etc.

### ii) Quantification of transcriptional activity

Reads obtained by sequencing were aligned against the human reference genome (hg19) using bwa (Bioinformatics. 2009 Jul 15; 25 (14): 1754-60).

Alignments were selected such that the mapping quality was 20 or more and the alignment starting position was located within the reference transcription start sites. The number of reads per transcription start site was counted. Counts per million were calculated using the total number of reads in each library and a library size predicted by RLE (Genome Biol. 2010; 11 (10): R106).

### (6) Results

### (A) Extraction of transcription start sites differing in activity

Differential analysis was conducted on the thus-quantified transcriptional activity of each sample for transcription start site extraction between a profile group derived from clinical specimens obtained from subjects "with lymph node metastasis" and a profile group derived from clinical specimens obtained from subjects "without lymph node metastasis" using R/Bioconductor edgeR package (Bioinformatics. 2010 Jan 1; 26 (1): 139-40). In short, this analysis is to statistically test whether an average expression level differs between two groups (equality of the average expression level is defined as null hypothesis, and assuming that this null hypothesis is true, the probability of producing assay results accidentally is calculated). The threshold was set to FDR (false discovery rate) of 5%. As a result, 277 DNAs containing transcription start sites having values smaller than this threshold were identified (Tables 1-1 to 1-12). This criterion is based on the statistical presumption that 95% of candidates extracted by the corresponding threshold truly have expression difference, and is stricter than the P value (probability of occurring accidentally provided that there is no expression difference) of 5% usually used widely.

**[Table 1-1]**

| SEQ ID NO | Transcription start site (TSS) | | | | | Expression ratio [with metastasis/with out metastasis] | Average expression | Average expression level of group without metastasis | Average expression level of group with metastasis | Pvalue | FDR |
|---|---|---|---|---|---|---|---|---|---|---|---|
| | Chromosome number | Start position | Termination position | Strand | Gene name | | | | | | |
| 1 | chr8 | 61406495 | 61406499 | + | | 270.6714 | 80.90141 | 0 | 269.6714 | 1.38E-11 | 6.87E-07 |
| 2 | chr11 | 5248294 | 5248308 | - | HBB | 223.8821 | 1945.916 | 27.68823 | 6421.78 | 1.46E-10 | 7.25E-06 |
| 3 | chr4 | 74606277 | 74606293 | + | IL8 | 174.0954 | 1183.507 | 21.37932 | 3895.137 | 1.72E-08 | 0.000856 |
| 4 | chr2 | 149639329 | 149639360 | - | | 166.3905 | 64.16637 | 0.287437 | 213.2172 | 5.96E-10 | 2.97E-05 |
| 5 | chr2 | 113885165 | 113885181 | + | IL1RN | 163.5748 | 150.4722 | 2.043294 | 496.8062 | 2.5E-09 | 0.000124 |
| 6 | chr6 | 13272941 | 13272965 | + | PHACTR1 | 127.7184 | 86.8597 | 1.251916 | 286.6112 | 4.88E-09 | 0.000243 |
| 7 | chr2 | 37844467 | 37844481 | + | | 126.1014 | 39.07528 | 0.040095 | 130.1574 | 1.6E-09 | 7.98E-05 |
| 8 | chr3 | 71834290 | 71834306 | - | PROK2 | 124.6753 | 38.90223 | 0.047231 | 129.5639 | 1.64E-12 | 8.16E-08 |
| 9 | chr2 | 40680676 | 40680690 | - | SLC8A1 | 123.5894 | 48.92918 | 0.321688 | 162.3467 | 1.36E-07 | 0.00677 |
| 10 | chr1 | 209848749 | 209848764 | + | G0S2 | 118.1868 | 371.9691 | 9.315539 | 1218.161 | 9.74E-10 | 4.84E05 |
| 11 | chr5 | 169724725 | 169724744 | - | LCP2 | 114.8027 | 247.636 | 6.07542 | 811.2773 | 2.13E-09 | 0.000106 |
| 12 | chr15 | 80263506 | 80263553 | - | BCL2A1 | 107.8252 | 164.1653 | 3.997807 | 537.8894 | 8.09E-09 | 0.000402 |
| 13 | chr10 | 95360985 | 95360999 | - | RBP4 | 101.5621 | 72.45639 | 1.356741 | 238.3556 | 6.02E-07 | 0.02979 |
| 14 | chr2 | 167467988 | 167467993 | - | | 95.61781 | 51.09954 | 0.772977 | 168.5282 | 5.6E-09 | 0.000278 |
| 15 | chr15 | 34447023 | 34447038 | - | C15orf29 | 94.22973 | 29.72195 | 0.060514 | 98.93198 | 1.39E-08 | 0.00069 |
| 16 | chr22 | 30662832 | 30662847 | - | OSM | 90.66984 | 182.4083 | 5.573551 | 595.0228 | 5.76E-09 | 0.000286 |
| 17 | chr20 | 48892848 | 48892884 | + | ENST00000422459 | 89.85826 | 51.74744 | 0.907168 | 170.3748 | 1.28E-08 | 0.000637 |
| 18 | chr7 | 105926308 | 105926320 | - | NAMPT | 84.84781 | 28.21935 | 0.117189 | 93.79106 | 7.87E-09 | 0.000391 |
| 19 | chr12 | 123201337 | 123201364 | - | HCAR3 | 78.20847 | 24.79804 | 0.067687 | 82.5022 | 6.39E-10 | 3.18E-05 |
| 20 | chr2 | 70142232 | 70142251 | + | MXD1 | 77.7548 | 267.4314 | 10.17233 | 867.7025 | 1.76E-09 | 8.76E-05 |
| 21 | chr5 | 138882825 | 138882831 | + | | 77.72697 | 38.80787 | 0.657412 | 127.8256 | 1.01E-08 | 0.000502 |
| 22 | chr22 | 39353606 | 39353623 | + | APOBEC3A | 74.26265 | 28.23778 | 0.272381 | 93.49036 | 1.66E-09 | 8.26E-05 |
| 23 | chr1 | 183559575 | 183559602 | - | NCF2 | 73.05665 | 63.13777 | 1.835822 | 206.1756 | 2.73E-08 | 0.001355 |
| 24 | chr14 | 24105566 | 24105579 | + | DHRS2 | 71.49518 | 47.10029 | 1.171713 | 154.267 | 1.22E-09 | 6.08E-05 |
| 25 | chr4 | 175204510 | 175204534 | + | CEP44 | 68.59673 | 27.64014 | 0.345933 | 91.32663 | 2.73E-07 | 0.013547 |

**[Table 1-2]**

| SEQ ID NO | Transcription start site (TSS) | | | | | Expression ratio [with metastasis/with out metastasis] | Average expression | Average expression level of group without metastasis | Average expression level of group with metastasis | Pvalue | FDR |
|---|---|---|---|---|---|---|---|---|---|---|---|
| | Chromosome number | Start position | Termination position | Strand | Gene name | | | | | | |
| 26 | chr6 | 41254410 | 41254441 | - | TREM1 | 68.14648 | 113.4603 | 4.413383 | 367.903 | 4.24E-07 | 0.021007 |
| 27 | chr11 | 57227981 | 57228020 | + | RTN4RL2 | 62.26013 | 79.29082 | 3.143393 | 256.9682 | 1.71E-08 | 0.000849 |
| 28 | chr5 | 1494968 | 1495042 | - | LPCAT1 | 59.92638 | 29.78347 | 0.648122 | 97.76596 | 2,33E-08 | 0.001155 |
| 29 | chr16 | 57702210 | 57702233 | + | GPR97 | 59.16715 | 23.46849 | 0.326195 | 77.46719 | 1.59E-08 | 0.000787 |
| 30 | chr15 | 45722800 | 45722816 | + | C15orf48 | 53.9488 | 167.2811 | 8.96652 | 536.6818 | 1.93E-07 | 0.009572 |
| 31 | chr2 | 102608415 | 102608430 | + | IL1R2 | 53.83476 | 39.69222 | 1.414907 | 129.006 | 1.25E-08 | 0.000622 |
| 32 | chr1 | 161600942 | 161600975 | - | FCGR3B | 53.6243 | 18.2662 | 0.147666 | 60.54278 | 2,05E-09 | 0.000102 |
| 33 | chr1 | 161494328 | 161494332 | + | HSPA6 | 52.73901 | 34.33503 | 1.138704 | 111.7931 | 6.99E-07 | 0.034605 |
| 34 | chrX | 101035626 | 101035631 | - | ENST00000328462 | 52.70185 | 69.76278 | 3.285912 | 224.8755 | 5.97E-10 | 2.97E-05 |
| 35 | chr11 | 117695449 | 117695466 | - | FXYD2 | 50.49336 | 24.88004 | 0.633015 | 81.45644 | 4.99E-07 | 0.024705 |
| 36 | chr7 | 45928079 | 45928095 | + | IGFBP1 | 49.04065 | 15.28568 | 0.056675 | 50.82001 | 2.69E-10 | 1.34E-05 |
| 37 | chr2 | 85921473 | 85921488 | + | GNLY | 48.27424 | 75.93858 | 4.067659 | 243.6374 | 5.76E-08 | 0.00286 |
| 38 | chr13 | 100634031 | 100634045 | + | ZIC2 | 47.62113 | 16.81088 | 0.188474 | 55.59649 | 7.81E-12 | 3.89E-07 |
| 39 | chr19 | 55385682 | 55385717 | + | FCAR | 47.33784 | 19.82158 | 0.397294 | 65.1449 | 3.23E-07 | 0.016008 |
| 40 | chr19 | 52264423 | 52264439 | + | FPR2 | 46.11272 | 17.08299 | 0.244201 | 56.37351 | 4.64E-07 | 0.023 |
| 41 | chr8 | 26247896 | 26247981 | + | BNIP3L | 45.91816 | 17.37907 | 0.269672 | 57.301 | 9.05E-08 | 0.004489 |
| 42 | chr5 | 1480422 | 1480489 | - | | 45.4815 | 14.3759 | 0.071906 | 47.75189 | 5.44E-11 | 2.71E-06 |
| 43 | chr20 | 36974877 | 36974892 | + | LBP | 44.8291 | 13.62732 | 0.033826 | 45.34547 | 9.78E-10 | 4.86E-05 |
| 44 | chr11 | 102668879 | 102668898 | - | MMP1 | 43.88456 | 30.6879 | 1.285399 | 99.29374 | 3.28E-07 | 0.01627 |
| 45 | chr2 | 95691445 | 95691461 | + | MAL | 43.24674 | 34.46018 | 1.593252 | 111.1497 | 2.76E-08 | 0.001373 |
| 46 | chr17 | 6899366 | 6899371 | + | ALOX12 | 42.77858 | 12.53357 | 0 | 41.77858 | 4.7E-10 | 2.34E-05 |
| 47 | chr9 | 19127478 | 19127497 | - | PLIN2 | 42.20767 | 344.8787 | 24.88467 | 1091.532 | 1.78E-07 | 0.00881 |
| 48 | chrM | 3226 | 3246 | + | AK074482 | 41.08689 | 80.85846 | 5.284204 | 257.1984 | 3.87E-09 | 0.000192 |
| 49 | chr2 | 114461747 | 114461752 | - | ENST00000424612 | 40.79368 | 12.56893 | 0.048757 | 41.78266 | 5.37E-12 | 2.67E-07 |
| 50 | chr19 | 7741968 | 7742004 | + | C19orf59 | 40.77247 | 13.38689 | 0.112526 | 44.36042 | 7.27E-08 | 0.003608 |

**[Table 1-3]**

| SEQ ID NO | Transcription start site (TSS) | | | | | Expression ratio [with metastasis/with out metastasis] | Average expression | Average expression level of group without metastasis | Average expression level of group with metastasis | Pvalue | FDR |
|---|---|---|---|---|---|---|---|---|---|---|---|
| | Chromosome number | Start position | Termination position | Strand | Gene name | | | | | | |
| 51 | chr2 | 218766698 | 218766769 | - | TNS1 | 40.12586 | 22.24344 | 0.824767 | 72.22035 | 5.22E-07 | 0.025876 |
| 52 | chr20 | 2310953 | 2310974 | + | | 39.99858 | 14.2966 | 0.204497 | 47.17816 | 2.58E-07 | 0.012768 |
| 53 | chr1 | 185286466 | 185286482 | - | IVNS1ABP | 39.57347 | 169.1156 | 12.53126 | 534.4791 | 2.75E-11 | 1.37E-06 |
| 54 | chr14 | 62217779 | 62217824 | - | ENST00000554254 | 39.41585 | 18.02228 | 0.518774 | 58.86378 | 4.13E-10 | 2.05E-05 |
| 55 | chr1 | 66797919 | 66797935 | + | PDE4B | 39.32486 | 68.72843 | 4.579409 | 218.4095 | 3.25E-08 | 0.001614 |
| 56 | chr22 | 39348723 | 39348754 | + | APOBEC3A | 35.88999 | 11.76839 | 0.11349 | 38.96315 | 2.37E-08 | 0.001175 |
| 57 | chr6 | 10482197 | 10482203 | - | | 35.67032 | 12.40798 | 0.176025 | 40.9492 | 3.06E-09 | 0.000152 |
| 58 | chr4 | 56502455 | 56502471 | - | NMU | 35.62994 | 45.16947 | 3.053872 | 143.4392 | 5.75E-09 | 0.000286 |
| 59 | chr22 | 30667064 | 30667080 | + | | 33.91904 | 11.02919 | 0.10606 | 36.51648 | 4.16E-07 | 0.020629 |
| 60 | chr3 | 187463154 | 187463172 | - | | 33.61904 | 53.01154 | 4.007694 | 167.3539 | 1.84E-07 | 0.009106 |
| 61 | chr10 | 75670882 | 75670898 | + | PLAU | 33.00066 | 294.9354 | 26.91791 | 920.3094 | 1.68E-08 | 0.000834 |
| 62 | chr17 | 8868991 | 8869059 | - | PIK3R5 | 31.16839 | 79.44338 | 7.003905 | 248.4688 | 1.45E-07 | 0.007184 |
| 63 | chr6 | 72313654 | 72313701 | + | BC038189 | 30.63997 | 9.093983 | 0.02042 | 30.26563 | 5.41E-09 | 0.000269 |
| 64 | chr7 | 150549595 | 150549614 | + | ABP1 | 30.46883 | 27.92291 | 1.939126 | 88.55173 | 5.73E-07 | 0.028376 |
| 65 | chr22 | 18260337 | 18260359 | + | LINC00528 | 30.12836 | 9.327828 | 0.060514 | 30.95156 | 3.18E-07 | 0.015772 |
| 66 | chr3 | 10206585 | 10206609 | + | IRAK2 | 29.89898 | 21.19192 | 1.294998 | 67.61809 | 3.52E-08 | 0.001749 |
| 67 | chr11 | 46940074 | 46940135 | - | LRP4 | 29.77837 | 37.53972 | 3.00059 | 118.131 | 5.83E-10 | 2.9E-05 |
| 68 | chr14 | 96968802 | 96968817 | + | PAPOLA | 29.77175 | 15.81505 | 0.745835 | 50.97656 | 2.67E-12 | 1.33E-07 |
| 69 | chr1 | 27961629 | 27961665 | - | FGR | 29.45049 | 21.65399 | 1.375841 | 68.96968 | 9.27E-07 | 0.045844 |
| 70 | chr1 | 6086424 | 6086442 | + | KCNAB2 | 29.18244 | 44.02312 | 3.761967 | 137.9658 | 5.45E-07 | 0.02701 |
| 71 | chr3 | 46448742 | 46448764 | + | CCRL2 | 28.30933 | 19.92255 | 1.275971 | 63.43122 | 1.79E-07 | 0.008884 |
| 72 | chr7 | 106065713 | 106065747 | + | | 27.8147 | 9.7371 | 0.187153 | 32.02031 | 2.41E-07 | 0.011959 |
| 73 | chr22 | 18923796 | 18923814 | - | PRODH | 27.71689 | 22.78725 | 1.638611 | 72.13408 | 1.09E-07 | 0.005417 |
| 74 | chrM | 16213 | 16268 | - | | 27.6057 | 10.6746 | 0.299819 | 34.88243 | 6.7E-07 | 0.033174 |
| 75 | chr10 | 114132153 | 114132160 | + | | 27.2599 | 8.638282 | 0.08564 | 28.59445 | 2.94E-07 | 0.014591 |

**[Table 1-4]**

| SEQ ID NO | Transcription start site (TSS) | | | | | Expression ratio [with metastasis/with out metastasis] | Average expression | Average expression level of group without metastasis | Average expression level of group with metastasis | Pvalue | FDR |
|---|---|---|---|---|---|---|---|---|---|---|---|
| | Chromosome number | Start position | Termination position | Strand | Gene name | | | | | | |
| 76 | chr4 | 40244302 | 40244315 | + | RHOH | 26.96965 | 7.790894 | 0 | 25.96965 | 1.99E-07 | 0.009878 |
| 77 | chrM | 11250 | 11434 | - | | 26.89866 | 26.29557 | 2.112522 | 82.72269 | 8.42E-10 | 4.19E-05 |
| 78 | chr5 | 178259821 | 178259898 | - | | 26.87124 | 8.661217 | 0.102706 | 28.63108 | 6.92E-15 | 3.44E-10 |
| 79 | chr5 | 169724839 | 169724855 | - | LCP2 | 26.64255 | 11.55189 | 0.443947 | 37.47043 | 1.92E-07 | 0.009519 |
| 80 | chr7 | 93520056 | 93520072 | - | TFPI2 | 26.62237 | 193.2154 | 21.35776 | 594.2166 | 1.31E-08 | 0.000649 |
| 81 | chr16 | 31366486 | 31366531 | + | ITGAX | 26.50419 | 100.119 | 10.68836 | 308.7905 | 2.47E-07 | 0.012242 |
| 82 | chr22 | 50925279 | 50925294 | + | MIOX | 25.76009 | 7.600125 | 0.02042 | 25.2861 | 9.62E-09 | 0.000478 |
| 83 | chr9 | 137809718 | 137809778 | - | FCN1 | 25.7304 | 21.67469 | 1.693238 | 68.29809 | 8.19E-07 | 0.040554 |
| 84 | chr1 | 36948482 | 36948504 | - | CSF3R | 25.5693 | 16.17168 | 1.051382 | 51.45239 | 2.46E-08 | 0.001224 |
| 85 | chr10 | 31288398 | 31288455 | - | ZNF438 | 25.54095 | 18.27317 | 1.304773 | 57.86608 | 5.85E-08 | 0.002903 |
| 86 | chr11 | 65779425 | 65779509 | + | CST6 | 25.4396 | 39.98184 | 3.91868 | 124.1292 | 3.8E-07 | 0.01882 |
| 87 | chrM | 10862 | 10891 | - | | 25.01632 | 9.589638 | 0.290649 | 31.28728 | 7.09E-08 | 0.003521 |
| 88 | chrM | 16274 | 16381 | - | | 24.9048 | 11.97311 | 0.587616 | 38.53925 | 3.96E-08 | 0.001966 |
| 89 | chr21 | 39601840 | 39601861 | + | KCNJ15 | 24.87552 | 7.759962 | 0.073176 | 25.6958 | 8.08E-07 | 0.039992 |
| 90 | chr1 | 36948505 | 36948540 | - | CSF3R | 24.64381 | 71.75813 | 7.990096 | 220.5502 | 6.05E-07 | 0.029972 |
| 91 | chrM | 10910 | 10956 | - | | 24.62878 | 10.91507 | 0.473064 | 35.27976 | 1.59E-08 | 0.000791 |
| 92 | chr16 | 56703703 | 56703733 | + | MT1H | 24.14092 | 15.52818 | 1.081038 | 49.23818 | 2.83E-07 | 0.014028 |
| 93 | chr20 | 1876009 | 1876075 | + | SIRPA | 23.98613 | 29.89243 | 2.912494 | 92.84561 | 8.52E-07 | 0.042165 |
| 94 | chr15 | 69075358 | 69075384 | - | | 23.93173 | 9.438595 | 0.324776 | 30.70417 | 1.48E-07 | 0.007365 |
| 95 | chr11 | 104972182 | 104972190 | - | CARD17, CARD16, CASP1 | 23.07024 | 7.334369 | 0.093595 | 24.22951 | 7.9E-07 | 0.039106 |
| 96 | chr16 | 31366536 | 31366558 | + | ITGAX | 22.56698 | 10.22567 | 0.502748 | 32.91248 | 8.74E-07 | 0.043235 |
| 97 | chrX | 30671504 | 30671547 | + | GK | 22.52082 | 30.40091 | 3.211356 | 93.8432 | 4.53E-08 | 0.002251 |
| 98 | chr16 | 57702180 | 57702202 | + | GPR97 | 22.45251 | 7.454874 | 0.137057 | 24.52978 | 1.88E-07 | 0.009307 |
| 99 | chrM | 5348 | 5405 | - | AK000561 | 22.28772 | 13.92442 | 1.02055 | 44.03346 | 9.35E-09 | 0.000465 |
| 100 | chr12 | 8088773 | 8088789 | - | SLC2A3 | 21.89953 | 310.8749 | 41.89972 | 938.4837 | 3.98E-07 | 0.019741 |

**[Table 1-5]**

| SEQ ID NO | Transcription start site (TSS) | | | | | Expression ratio [with metastasis/with out metastasis] | Average expression | Average expression level of group without metastasis | Average expression level of group with metastasis | Pvalue | FDR |
|---|---|---|---|---|---|---|---|---|---|---|---|
| | Chromosome number | Start position | Termination position | Strand | Gene name | | | | | | |
| 101 | chr1 | 185287239 | 185287298 | + | | 21.8019 | 9.174118 | 0.405154 | 29.63503 | 1.14E-07 | 0.005676 |
| 102 | chr8 | 90770191 | 90770203 | + | RIPK2 | 21.61072 | 10.60394 | 0.615425 | 33.91049 | 3.14E-08 | 0,001559 |
| 103 | chr2 | 108905063 | 108905076 | - | | 21.55379 | 6.166136 | 0 | 20.55379 | 4.28E-09 | 0.000213 |
| 104 | chr16 | 66620833 | 66620903 | + | CMTM2 | 21.42476 | 6.26766 | 0.019675 | 20.84629 | 9.35E-09 | 0.000465 |
| 105 | chr11 | 57194550 | 57194617 | - | SLC43A3 | 21.29333 | 83.95968 | 10.98641 | 254.2306 | 5.8E-13 | 2.89E-08 |
| 106 | chr4 | 165798317 | 165798356 | + | ENST00000515275,uc003iqv.1 | 21.17571 | 6.637359 | 0.082897 | 21.9311 | 7.34E-08 | 0.003643 |
| 107 | chr10 | 81107216 | 81107237 | + | PPIF | 20.99054 | 234.5886 | 32.66916 | 705.7339 | 4.86E.08 | 0.002411 |
| 108 | chr7 | 23509977 | 23510032 | - | IGF2BP3 | 20.97957 | 6.462664 | 0.067029 | 21.38581 | 1.06E-10 | 5.25E-06 |
| 109 | chr3 | 46152659 | 46152674 | - | | 20.66025 | 6.09355 | 0.028337 | 20.24571 | 2.11E-07 | 0.010455 |
| 110 | chr3 | 134314104 | 134314110 | + | | 20.65381 | 5.896144 | 0 | 19.65381 | 4.71E-08 | 0.00234 |
| 111 | chr11 | 57228029 | 57228040 | + | RTN4RL2 | 20.26062 | 6.376224 | 0.08823 | 21.04821 | 8.22E-08 | 0.004081 |
| 112 | chr17 | 76411049 | 76411071 | + | | 19.85891 | 5.657673 | 0 | 18.85891 | 7.87E07 | 0.038936 |
| 113 | chr10 | 43187153 | 43187192 | - | ENST00000411439 | 19.83134 | 5.738542 | 0.013406 | 19.09719 | 1.34E-10 | 6.67E-06 |
| 114 | chr14 | 73930324 | 73930350 | - | NUMB | 19.60412 | 6.722403 | 0.173397 | 22.00342 | 2.52E-07. | 0.012493 |
| 115 | chrM | 5421 | 5456 | - | AK000561 | 19.40974 | 15.09599 | 1.467603 | 46.89555 | 4.8E-07 | 0.023793 |
| 116 | chrM | 3533 | 3576 | - | chrM:3533..3576 | 19.39823 | 9.845331 | 0.66353 | 31.26953 | 7.63E-08 | 0.003786 |
| 117 | chrM | 4909 | 4987 | - | AK000561 | 19.33235 | 7.381412 | 0.289506 | 23.92919 | 5.38E-09 | 0.000267 |
| 118 | chr19 | 2625676 | 2625690 | + | | 19.24058 | 5.472175 | 0 | 18.24058 | 9.01E-07 | 0.044596 |
| 119 | chr5 | 138930120 | 138930127 | + | | 19.1949 | 7,863084 | 0.37232 | 25.34153 | 2.59E-10 | 1.29E-05 |
| 120 | chr7 | 104581389 | 104581394 | + | ENST00000415513 | 19.16205 | 41.34516 | 5.566551 | 124.8286 | 8.86E-15 | 4.41E-10 |
| 121 | chr9 | 5231413 | 5231425 | + | INSL4 | 19.10382 | 5.431146 | 0 | 18.10382 | 2.76E-11 | 1.37E-06 |
| 122 | chr5 | 169739679 | 169739733 | - | ENST00000511921 | 18.93142 | 5.379426 | 0 | 17.93142 | 9.8E-07 | 0.048469 |
| 123 | chrX | 150732134 | 150732184 | + | PASD1 | 18.84006 | 5.532019 | 0.028337 | 18.37394 | 1.84E-07 | 0.009128 |
| 124 | chr19 | 14785622 | 14785667 | - | EMR3 | 18.72648 | 6.367507 | 0.166124 | 20.8374 | 8.61E-08 | 0.004271 |
| 125 | chr8 | 142318379 | 142318394 | - | SLC45A4 | 18.67466 | 94.43721 | 14.143 | 281.7904 | 1.49E-10 | 7.41E-06 |

**[Table 1-6]**

| SEQ ID NO | Transcription start site (TSS) | | | | | Expression ratio [with metastasis/with out metastasis] | Average expression | Average expression level of group without metastasis | Average expression level of group with metastasis | Pvalue | FDR |
|---|---|---|---|---|---|---|---|---|---|---|---|
| | Chromosome number | Start position | Termination position | Strand | Gene name | | | | | | |
| 126 | chr21 | 39644395 | 39644454 | + | KCNJ15 | 18.65757 | 5.297272 | 0 | 17.65757 | 3.84E-07 | 0.019 |
| 127 | chr12 | 8693475 | 8693507 | - | CLEC4E | 18.62185 | 10.11369 | 0.767851 | 31.92065 | 2.25E-07 | 0.011172 |
| 128 | chr6 | 87923426 | 87923456 | + | ZNF292 | 18.54366 | 5.698325 | 0.069491 | 18.83227 | 4.18E-07 | 0.020716 |
| 129 | chrM | 9060 | 9111 | - | AF271776 | 18.4288 | 7.933243 | 0.43422 | 25.43096 | 6.01E-06 | 0.003976 |
| 130 | chr5 | 36066975 | 36067019 | - | UGT3A2 | 18.31299 | 5.74694 | 0.089289 | 18.94813 | 5.36E-07 | 0.026528 |
| 131 | chr2 | 136875712 | 136875725 | - | CXCR4 | 18.06314 | 233.4361 | 37.31317 | 691.0562 | 2.78E-07 | 0.013784 |
| 132 | chrM | 15354 | 15374 | - | BC006482 | 17.73559 | 10.70116 | 0.943497 | 33.46905 | 3.11E-07 | 0.015401 |
| 133 | chr4 | 185747114 | 185747128 | - | ACSL1 | 17.71645 | 78.47877 | 12.21357 | 233.0976 | 4.65E-09 | 0.000231 |
| 134 | chr20 | 1569332 | 1569349 | - | SIRPB1 | 17.61528 | 4.984585 | 0 | 16.61528 | 1.89E-07 | 0.009348 |
| 135 | chr17 | 48072574 | 48072597 | - | DLX3 | 17.50316 | 8.384514 | 0.576978 | 26.6021 | 8.01E-08 | 0.003973 |
| 136 | chr10 | 98480243 | 98480297 | - | PIK3AP1 | 17.40359 | 60.41224 | 9.371804 | 179.5066 | 8.41E-07 | 0.041612 |
| 137 | chrM | 8667 | 8702 | - | | 17.39875 | 7.601996 | 0.453132 | 24.28268 | 3.05E-08 | 0.001512 |
| 138 | chr4 | 6675172 | 6675178 | + | ENST00000307533, ENST00000444232 | 17.35572 | 19.23904 | 2.426445 | 58.46844 | 6.43E-08 | 0.003189 |
| 139 | chr8 | 19615538 | 19615541 | - | CSGALNACT1 | 17.24864 | 5.381643 | 0.086313 | 17.73741 | 2.74E-08 | 0.001361 |
| 140 | chr7 | 48128854 | 48128871 | + | UPP1 | 17.21653 | 57.05072 | 8.897892 | 169.4073 | 4.41E-08 | 0.002188 |
| 141 | chr8 | 128231080 | 128231222 | - | ENST00000500112, uc003ysa.1 | 17.17313 | 5.935715 | 0.185199 | 19.35358 | 2.06E-10 | 1.02E-05 |
| 142 | chr13 | 100633445 | 100633468 | + | AK298232 | 17.1168 | 5.58311 | 0.128203 | 18.31123 | 2.54E-09 | 0.000126 |
| 143 | chr19 | 35939154 | 35939175 | + | FFAR2 | 17.07516 | 5.660319 | 0.143884 | 18.532 | 4.35E-07 | 0.021566 |
| 144 | chr19 | 42300512 | 42300542 | + | CEACAM3 | 17.07448 | 5.657331 | 0.143411 | 18.52314 | 1.96E-07 | 0.009713 |
| 145 | chr2 | 28618532 | 28618610 | + | FOSL2 | 17.06395 | 13.31481 | 1.459934 | 40.97618 | 1.01E-06 | 0.049759 |
| 146 | chrM | 6528 | 6569 | - | | 16.7295 | 6.872642 | 0.376613 | 22.03004 | 1.16E-08 | 0.000574 |
| 147 | chr12 | 28122899 | 28122916 | - | PTHLH | 16.54224 | 10.80003 | 1.083827 | 33.47117 | 2.09E-07 | 0.01036 |
| 148 | chr2 | 149639361 | 149639365 | - | | 16.15992 | 4.705191 | 0.028337 | 15.61785 | 3.6E-07 | 0.017819 |

**[Table 1-7]**

| SEQ ID NO | Transcription start site (TSS) | | | | | Expression ratio [with metastasis/with out metastasis] | Average expression | Average expression level of group without metastasis | Average expression level of group with metastasis | Pvalue | FDR |
|---|---|---|---|---|---|---|---|---|---|---|---|
| | Chromosome number | Start position | Termination position | Strand | Gene name | | | | | | |
| 149 | chr1 | 209848561 | 209848582 | - | ENST00000445272 | 15.87511 | 4.462534 | 0 | 14.87511 | 8.88E-07 | 0.043928 |
| 150 | chr15 | 23841916 | 23841926 | - | | 15.47947 | 4.343842 | 0 | 14.47947 | 1.46E-08 | 0.000723 |
| 151 | chr2 | 152214098 | 152214115 | + | TNFAIP6 | 15.34292 | 13.12939 | 1.664477 | 39.88086 | 9.91E-08 | 0.004918 |
| 152 | chr1 | 119530930 | 119530971 | - | | 15.27647 | 4.28294 | 0 | 14.27647 | 2.25E-09 | 0.000112 |
| 153 | chr2 | 71680778 | 71680825 | + | DYSF | 15.12668 | 6.007967 | 0.337908 | 19.23811 | 7.62E-07 | 0.037717 |
| 154 | chrM | 10660 | 10715 | - | | 14.64976 | 5.777522 | 0.330249 | 18.48783 | 1.9E-07 | 0.009413 |
| 155 | chrM | 13640 | 13698 | - | | 14.63985 | 40.21251 | 7.09365 | 117.4898 | 1.26E-08 | 0.000626 |
| 156 | chr22 | 39350005 | 39350109 | - | | 14.53735 | 4.228634 | 0.033081 | 14.01826 | 1.01E-06 | 0.049892 |
| 157 | chr16 | 56701968 | 56701983 | - | MT1G | 14.35805 | 126.4655 | 24.45534 | 364.4891 | 1.43E-07 | 0.007104 |
| 158 | chrM | 11850 | 11887 | - | | 14.21507 | 7.39475 | 0.690949 | 23.03695 | 5.93E-07 | 0.029379 |
| 159 | chrM | 12425 | 12491 | - | | 14.20551 | 6.346583 | 0.480673 | 20.03371 | 7.04E-07 | 0.034828 |
| 160 | chrM | 8717 | 8734 | - | AF271776 | 14.14642 | 4.044878 | 0.02042 | 13.43528 | 1.51E07 | 0.007505 |
| 161 | chr20 | 62103939 | 62104035 | - | KCNQ2 | 14.08484 | 4.092057 | 0.033826 | 13.56126 | 5.86E-08 | 0.002908 |
| 162 | chrM | 3880 | 3914 | - | | 13.97222 | 6.094845 | 0.450394 | 19.26523 | 9.6E-07 | 0.047501 |
| 163 | chrM | 583 | 594 | + | | 13.85105 | 7.853463 | 0.823458 | 24.25681 | 5.95E-08 | 0.002954 |
| 164 | chrM | 9833 | 9922 | - | | 13.81721 | 5.877016 | 0.419357 | 18.61155 | 8.11E-07 | 0.040119 |
| 165 | chr8 | 23386444 | 23386524 | + | SLC25A37 | 13.80477 | 107.6573 | 21.44322 | 308.8235 | 2.74E-07 | 0.013569 |
| 166 | chrX | 23710342 | 23710348 | - | | 13.74656 | 3.922471 | 0.02042 | 13.02726 | 2.07E-11 | 1.03E-06 |
| 167 | chr16 | 11680767 | 11680787 | - | LITAF | 13.52465 | 59.49426 | 11.71584 | 170.9773 | 1.55E-07 | 0.007689 |
| 168 | chr13 | 100623375 | 100623425 | - | ZIC5 | 13.49612 | 3.883406 | 0.028337 | 12.87857 | 1.56E-09 | 7.77E-05 |
| 169 | chr19 | 929027 | 929051 | + | ARID3A | 13.37004 | 3.807209 | 0.02042 | 12.64305 | 1.13E-07 | 0.005583 |
| 170 | chr14 | 104394770 | 104394820 | + | TDRD9 | 13.36687 | 7.998435 | 0.910471 | 24.53702 | 2.48E-07 | 0.012283 |
| 171 | chr7 | 150499392 | 150499424 | - | | 13.31597 | 3.790658 | 0.02042 | 12.58788 | 1.48E-07 | 0.007337 |
| 172 | chr21 | 43767753 | 43767789 | - | | 13.18064 | 3.654193 | 0 | 12.18064 | 6.15E-08 | 0.003053 |
| 173 | chrM | 5795 | 5816 | + | uc011mfh.1 | 13.15753 | 39.98469 | 7.819111 | 115.0377 | 1.77E-07 | 0.008801 |

**[Table 1-8]**

| SEQ ID NO | Transcription start site (TSS) | | | | | Expression ratio [with metastasis/with out metastasis] | Average expression | Average expression level of group without metastasis | Average expression level of group with metastasis | Pvalue | FDR |
|---|---|---|---|---|---|---|---|---|---|---|---|
| | Chromosome number | Start position | Termination position | Strand | Gene name | | | | | | |
| 174 | chr19 | 33191049 | 33191054 | - | | 13.05238 | 3.615714 | 0 | 12.05238 | 1.12E-11 | 5.55E-07 |
| 175 | chr1 | 153348067 | 153348103 | - | S100A12 | 12.92288 | 4.083995 | 0.110803 | 13.35478 | 3.43E-09 | 0.00017 |
| 176 | chr9 | 95896516 | 95896543 | - | NINJ1 | 12.91969 | 128.1483 | 27.22353 | 363.6393 | 2.37E-07 | 0.011771 |
| 177 | chr4 | 175184180 | 175184194 | - | FBXO8 | 12.85693 | 3.860751 | 0.066638 | 12.71368 | 3.66E-07 | 0.018152 |
| 178 | chr1 | 119530706 | 119530736 | - | TBX15 | 12.72484 | 3.517452 | 0 | 11.72484 | 3.15E-08 | 0.001563 |
| 179 | chr2 | 192542879 | 192542892 | + | OBFC2A | 12.67121 | 12.36553 | 1.969219 | 36.62359 | 1.37E-07 | 0.006808 |
| 180 | chr9 | 132472174 | 132472192 | + | | 12.6681 | 3.677521 | 0.03935 | 12.16659 | 1.99E-08 | 0.000986 |
| 181 | chr20 | 32150185 | 32150230 | + | CBFA2T2 | 12.64399 | 4.042793 | 0.122318 | 13.19057 | 6.94E-07 | 0.034347 |
| 182 | chr8 | 71368695 | 71368709 | - | | 12.62677 | 3.576334 | 0.019675 | 11.8752 | 1.55E-10 | 7.73E-06 |
| 183 | chr4 | 97091624 | 97091635 | + | | 12.58358 | 3.475073 | 0 | 11.58358 | 3.27E-08 | 0.001622 |
| 184 | chr6 | 107235249 | 107235267 | - | LOC100422737 | 12.51295 | 3.453884 | 0 | 11.51295 | 3.32E-08 | 0.001648 |
| 185 | chr8 | 104512123 | 104512154 | + | | 12.47184 | 4.033988 | 0.133385 | 13.1354 | 9.26E-07 | 0.045812 |
| 186 | chr1 | 95007302 | 95007322 | - | F3 | 12.44798 | 74.18679 | 15.95538 | 210.0601 | 5.87E-09 | 0.000292 |
| 187 | chrM | 14747 | 14817 | - | | 12.34811 | 16.19091 | 2.903093 | 47.19583 | 1.87E-07 | 0.009252 |
| 188 | chr21 | 44027711 | 44027715 | - | | 12.30994 | 3.392983 | 0 | 11.30994 | 5.54E-09 | 0.000276 |
| 189 | chr11 | 117695415 | 117695426 | - | FXYD2 | 12.2662 | 3.737602 | 0.081679 | 12.26809 | 5.02E-08 | 0.002494 |
| 190 | chrM | 15464 | 15549 | - | BC006482 | 12.21739 | 40.01678 | 8.396272 | 113.798 | 3.86E-08 | 0.001917 |
| 191 | chr12 | 65153154 | 65153171 | - | GNS | 11.9824 | 101.8805 | 22.95511 | 286.0396 | 5.38E-07 | 0.026642 |
| 192 | chr7 | 40174437 | 40174476 | - | C7orf11 | 11.93461 | 4.703914 | 0.332571 | 14.90372 | 1.12E-07 | 0.00554 |
| 193 | chr8 | 86376226 | 86376243 | + | CA2 | 11.82427 | 22.88107 | 4.622672 | 65.484 | 2E-07 | 0.009936 |
| 194 | chr22 | 21011215 | 21011224 | - | SMPD4P1 | 11.5772 | 3.17316 | 0 | 10.5772 | 3.3E-09 | 0.000164 |
| 195 | chr13 | 41363739 | 41363753 | + | SLC25A15 | 11.54151 | 11.11705 | 1.911036 | 32.59777 | 4.21E-07 | 0.02085 |
| 196 | chr4 | 175205407 | 175205421 | - | FBXO8 | 11.53705 | 3.885829 | 0.174164 | 12.54638 | 1.13E-07 | 0.005616 |
| 197 | chr11 | 57193937 | 57193966 | - | SLC43A3 | 11.50955 | 3.234572 | 0.019675 | 10.736 | 2E-07 | 0.009926 |
| 198 | chr2 | 70142189 | 70142223 | + | MXD1 | 11.493 | 71.48147 | 16.47425 | 199.8317 | 6.98E-07 | 0.034572 |

**[Table 1-9]**

| SEQ ID NO | Transcription start site (TSS) | | | | | Expression ratio [with metastasis/with out metastasis] | Average expression | Average expression level of group without metastasis | Average expression level of group with metastasis | Pvalue | FDR |
|---|---|---|---|---|---|---|---|---|---|---|---|
| | Chromosome number | Start position | Termination position | Strand | Gene name | | | | | | |
| 199 | chrM | 2343 | 2365 | - | | 11.45056 | 5.090463 | 0.472845 | 15.86491 | 6.17E-07 | 0.030546 |
| 200 | chr6 | 13925318 | 13925342 | + | RNF182 | 11.24698 | 3.152114 | 0.01915 | 10.46236 | 6.55E-09 | 0.000326 |
| 201 | chr10 | 43867077 | 43867098 | + | FXYD4 | 10.86414 | 3.142326 | 0.046242 | 10.36652 | 4.64E-10 | 2.31E-05 |
| 202 | chrM | 9036 | 9071 | + | uc011mfi.1 | 10.85356 | 54.88917 | 13.12746 | 152.3332 | 6.61E-07 | 0.032733 |
| 203 | chr4 | 155484155 | 155484177 | + | FGB | 10.8531 | 2.955931 | 0 | 9.853105 | 5.84E-08 | 0.002901 |
| 204 | chrM | 14366 | 14421 | - | | 10.80179 | 25.05371 | 5.611718 | 70,41836 | 3.76E-07 | 0.018617 |
| 205 | chr1 | 46955501 | 46955514 | + | | 10.77379 | 2.932138 | 0 | 9.773793 | 1E-08 | 0.000499 |
| 206 | chrM | 262 | 309 | - | uc004coq.3 | 10.66471 | 71.12642 | 17.49674 | 196.2623 | 1.1E-08 | 0.000548 |
| 207 | chr8 | 58055238 | 58055251 | + | ENST00000519314 | 10.51691 | 3.043035 | 0.048757 | 10.02968 | 6.7E-07 | 0.033163 |
| 208 | chr1 | 224370589 | 224370611 | + | DEGS1 | 10.30814 | 4.435322 | 0.433198 | 13.77361 | 7.3E-08 | 0.003624 |
| 209 | chr8 | 38239967 | 38239987 | - | WHSC1L1 | 10.14116 | 6.81111 | 1.087221 | 20.16685 | 2.65E-07 | 0.013114 |
| 210 | chrM | 11666 | 11689 | - | | 10.07977 | 5.122786 | 0.644173 | 15.57288 | 9.34E-07 | 0.04623 |
| 211 | chr7 | 150500382 | 150500396 | + | TMEM176A | 10.03012 | 2.854987 | 0.03935 | 9.424805 | 1.91E-08 | 0.00095 |
| 212 | chr8 | 9009045 | 9009059 | - | PPP1R3B | 10.00359 | 4.593518 | 0.511322 | 14.11864 | 2.44E-07 | 0.012091 |
| 213 | chr14 | 38678329 | 38678340 | + | SSTR1 | 9.953455 | 2.888417 | 0.054905 | 9.499947 | 8.65E-07 | 0.042828 |
| 214 | chr8 | 90770100 | 90770114 | + | RIPK2 | 9.865486 | 6.314098 | 0.998581 | 18.71697 | 5.26E-07 | 0.026041 |
| 215 | chrM | 9134 | 9163 | + | uc011mfi.1 | 9.843044 | 17.87006 | 4.165757 | 49.84677 | 2.89E-07 | 0.014298 |
| 216 | chr17 | 42447158 | 42447165 | + | | 9.799139 | 3.052816 | 0.11349 | 9.911243 | 3.13E-07 | 0.015514 |
| 217 | chr16 | 1524969 | 1525015 | - | CLCN7 | 9.772483 | 94.82909 | 25.38651 | 256.8618 | 6.69E-07 | 0.033121 |
| 218 | chr12 | 64784522 | 64784543 | - | C12orf56 | 9.617052 | 2.585116 | 0 | 8.617052 | 1.03E-07 | 0.005115 |
| 219 | chr8 | 127570706 | 127570729 | - | FAM84B | 9.494464 | 21.58584 | 5.365186 | 59.43403 | 6.8E-08 | 0.003373 |
| 220 | chr8 | 71368674 | 71368687 | - | | 9.405157 | 2.521547 | 0 | 8.405157 | 1.12E-07 | 0.005559 |
| 221 | chr8 | 9046503 | 9046517 | + | uc003wsp.1 | 9.369842 | 2.510952 | 0 | 8.369842 | 1.16E-07 | 0.005739 |
| 222 | chr9 | 119601047 | 119601058 | + | uc004bjy.2 | 9.369842 | 2.510952 | 0 | 8.369842 | 1.16E-07 | 0.005739 |
| 223 | chrX | 15682921 | 15682937 | - | TMEM27 | 9.22149 | 2.910546 | 0.128114 | 9.402889 | 2.95E-07 | 0.0146 |

**[Table 1-10]**

| SEQ ID NO | Transcription start site (TSS) | | | | | Expression ratio [with metastasis/with out metastasis] | Average expression | Average expression level of group without metastasis | Average expression level of group with metastasis | Pvalue | FDR |
|---|---|---|---|---|---|---|---|---|---|---|---|
| | Chromosome number | Start position | Termination position | Strand | Gene name | | | | | | |
| 224 | chr2 | 42722039 | 42722056 | - | | 9.127175 | 3.125019 | 0.199778 | 9.950582 | 1.69E-07 | 0.008373 |
| 225 | chr6 | 107235331 | 107235345 | - | ENST00000433965 | 8.981368 | 2.39441 | 0 | 7.981368 | 1.37E-07 | 0.006819 |
| 226 | chr4 | 97091584 | 97091602 | + | | 8.910736 | 2.373221 | 0 | 7.910736 | 1.42E-07 | 0.007048 |
| 227 | chr10 | 135160851 | 135160869 | + | PRAP1 | 8.902859 | 2.62928 | 0.076664 | 8.585386 | 5.88E-08 | 0.002917 |
| 228 | chrM | 14471 | 14537 | - | | 8.869242 | 27.58482 | 7.505431 | 74.43673 | 1.46E-07 | 0.007259 |
| 229 | chr17 | 67114473 | 67114480 | - | ABCA6 | 8.804789 | 2.341437 | 0 | 7.804789 | 1.51E-07 | 0.007468 |
| 230 | chr10 | 96443204 | 96443217 | + | CYP2C18 | 8.725478 | 2.317643 | 0 | 7.725478 | 2.8E-08 | 0.001392 |
| 231 | chr14 | 104398167 | 104398182 | + | | 8.716787 | 2.597848 | 0.085312 | 8.460431 | 2.07E-08 | 0.001026 |
| 232 | chrM | 13741 | 13762 | - | | 8.460537 | 11.74289 | 2.935224 | 32.29411 | 5.73E-07 | 0.028351 |
| 233 | chr11 | 11833036 | 11833048 | - | | 8.416315 | 2.224895 | 0 | 7.416315 | 1.85E-07 | 0.009177 |
| 234 | chr10 | 123779269 | 123779314 | + | TACC2 | 8.256719 | 3.306575 | 0.355541 | 10.19232 | 2.99E-08 | 0.001483 |
| 235 | chrM | 8112 | 8200 | + | uc011mfi.1 | 8.170829 | 20.3849 | 5.786168 | 54.44861 | 9.76E-07 | 0.048295 |
| 236 | chr2 | 131058236 | 131058275 | - | | 8.169105 | 2.150731 | 0 | 7.169105 | 2.1E-07 | 0.010429 |
| 237 | chr12 | 112443767 | 112443827 | - | TMEM116 | 8.076762 | 2.24428 | 0.038825 | 7.390343 | 1.07E-09 | 5.31E-05 |
| 238 | chr3 | 186435192 | 186435205 | + | KNG1 | 7.851263 | 2.055379 | 0 | 6.851263 | 2.56E-07 | 0.012677 |
| 239 | chr3 | 186435140 | 186435158 | + | KNG1 | 7.842344 | 2.111163 | 0.01915 | 6.992526 | 2.05E-07 | 0.010176 |
| 240 | chr2 | 192542850 | 192542872 | + | OBFC2A | 7.134746 | 146.6953 | 50.99764 | 369.99 | 4.09E-07 | 0.020263 |
| 241 | chr11 | 46740744 | 46740786 | + | F2 | 7.003684 | 1.801105 | 0 | 6.003684 | 4.28E-07 | 0.02118 |
| 242 | chr4 | 160521424 | 160521437 | - | | 7.003684 | 1.801105 | 0 | 6.003684 | 4.28E-07 | 0.021184 |
| 243 | chr10 | 135160870 | 135160906 | + | PRAP1 | 6.935729 | 1.780719 | 0 | 5.935729 | 3.88E-08 | 0.001926 |
| 244 | chr13 | 100634004 | 100634025 | + | ZIC2 | 6.901095 | 1.844606 | 0.026812 | 6.086125 | 6.4E-07 | 0.031708 |
| 245 | chr4 | 128703425 | 128703496 | + | HSPA4L | 6.886614 | 12.77834 | 3.98135 | 33.30464 | 4.55E-07 | 0.022512 |
| 246 | chr19 | 8444480 | 8444485 | + | | 6.821242 | 2.18727 | 0.160538 | 6.916311 | 6.69E-07 | 0.033103 |
| 247 | chr19 | 39313478 | 39313484 | + | | 6.774253 | 1.732276 | 0 | 5.774253 | 4.81E-08 | 0.002386 |
| 248 | chr22 | 37420056 | 37420068 | + | MPST | 6.544579 | 1.663374 | 0 | 5.544579 | 5.93E-07 | 0.029348 |

**[Table 1-11]**

| SEQ ID NO | Transcription start site (TSS) | | | | | Expression ratio [with metastasis/with out metastasis] | Average expression | Average expression level of group without metastasis | Average expression level of group with metastasis | Pvalue | FDR |
|---|---|---|---|---|---|---|---|---|---|---|---|
| | Chromosome number | Start position | Termination position | Strand | Gene name | | | | | | |
| 249 | chr10 | 3305882 | 3305893 | + | | 6.527042 | 1.658113 | 0 | 5.527042 | 5.85E-08 | 0.002902 |
| 250 | chr1 | 47407140 | 47407154 | - | CYP4A11 | 6.509263 | 1.652779 | 0 | 5.509263 | 6.24E-07 | 0.030869 |
| 251 | chr11 | 57193922 | 57193931 | - | SLC43A3 | 6.403315 | 1.620995 | 0 | 5.403315 | 6.78E-07 | 0.033544 |
| 252 | chr20 | 45336944 | 45336978 | + | | 6.184964 | 1.555489 | 0 | 5.184964 | 1.18E-07 | 0.005848 |
| 253 | chr10 | 96443542 | 96443563 | + | CYP2C18 | 6.156105 | 1.546831 | 0 | 5.156105 | 8.38E-07 | 0.041483 |
| 254 | chr7 | 138816862 | 138816869 | - | | 6.141002 | 1.542301 | 0 | 5.141002 | 4.79E-07 | 0.023712 |
| 255 | chr9 | 139873342 | 139873374 | + | PTGDS | 6.13502 | 2.000075 | 0.180897 | 6.244825 | 3.28E-07 | 0.016257 |
| 256 | chr7 | 87856280 | 87856298 | - | SRI | 6.133058 | 2.182229 | 0.252887 | 6.684027 | 7.91E-07 | 0.039167 |
| 257 | chr4 | 155511887 | 155511903 | - | FGA | 6.014842 | 1.504453 | 0 | 5.014842 | 9.21E-07 | 0.045587 |
| 258 | chr2 | 78768976 | 78769030 | - | uc002snv.3 | 5.936474 | 1.528452 | 0.01915 | 5.050158 | 7.72E-07 | 0.038202 |
| 259 | chr19 | 33184431 | 33184435 | - | | 5.905156 | 1.846915 | 0.151876 | 5.802006 | 2.72E-07 | 0.013477 |
| 260 | chr8 | 90770008 | 90770092 | + | RIPK2 | 5.858349 | 103.7222 | 41.61323 | 248.6432 | 4.23E-07 | 0.020934 |
| 261 | chr12 | 64784476 | 64784503 | - | C12orf56 | 5.803598 | 1.489108 | 0.019675 | 4.917784 | 1.85E-07 | 0.009162 |
| 262 | chr20 | 25320373 | 25320394 | - | ABHD12 | 5.69808 | 1.667344 | 0.107046 | 5.308038 | 1.99E-09 | 9.91 E-05 |
| 263 | chr7 | 151791137 | 151791153 | + | GALNT11 | 5.656486 | 1.429079 | 0.013406 | 4.732315 | 9.6E-07 | 0.047514 |
| 264 | chr10 | 123779316 | 123779341 | + | TACC2 | 5.625802 | 1.617269 | 0.096128 | 5.166598 | 1.01E-08 | 0.0005 |
| 265 | chr13 | 100634130 | 100634143 | + | ZIC2 | 5.336456 | 1.377054 | 0.033081 | 4.512991 | 1.53E-07 | 0.007593 |
| 266 | chr12 | 122107309 | 122107329 | - | MORN3 | 4.97329 | 1.281506 | 0.040839 | 4.176396 | 8.37E-07 | 0.041443 |
| 267 | chr2 | 10232601 | 10232606 | + | | 4.946689 | 1.184007 | 0 | 3.946689 | 6.13E-07 | 0.030346 |
| 268 | chrB | 103075001 | 103075005 | - | | 4.600114 | 1.120959 | 0.019675 | 3.690621 | 3.59E-07 | 0.017797 |
| 269 | chr5 | 1380178 | 1380183 | - | ENST00000504989,uc003jcj.1 | 4.513995 | 1.094615 | 0.019675 | 3.602808 | 6.05E-07 | 0.029979 |
| 270 | chr12 | 47473904 | 47473917 | - | AMIGO2 | 4.473497 | 1.238205 | 0.096058 | 3.903213 | 2.78E-07 | 0.013789 |
| 271 | chr2 | 162137741 | 162137745 | + | | 4.275899 | 0.98277 | 0 | 3.275899 | 1.62E-07 | 0.008046 |
| 272 | chr4 | 164647015 | 184647018 | + | | 4.097746 | 1.005243 | 0.03935 | 3.258993 | 9.1E-07 | 0.045029 |
| 273 | chr22 | 21011504 | 21011510 | + | | 3.887358 | 0.904315 | 0.02042 | 2.966736 | 3.85E-07 | 0.019098 |

**[Table 1-12]**

| SEQ ID NO | Transcription start site (TSS) | | | | | Expression ratio [with metastasis/with out metastasis | Average expression | Average expression level of group without metastasis | Average expression level of group with metastasis | Pvalue | FDR |
|---|---|---|---|---|---|---|---|---|---|---|---|
| | Chromosome number | Start position | Termination position | Strand | Gene name | | | | | | |
| 274 | chr6 | 18277503 | 18277537 | + | | 3.777658 | 0.976795 | 0.078273 | 3.073347 | 6.02E-07 | 0.029788 |
| 275 | chr6 | 31820606 | 31820612 | + | | 3.156812 | 0.669124 | 0.013406 | 2.199132 | 9.27E-07 | 0.045861 |
| 276 | chr1 | 204135454 | 204135486 | - | REN | 0.089136 | 7.441099 | 10.61501 | 0.035316 | 6.29E-07 | 0.031144 |
| 277 | chr7 | 83278414 | 83278504 | - | SEMA3E | 0.040516 | 16.57716 | 23.68166 | 0 | 4.37E-07 | 0.021658 |

### (B) Selection of transcription start site for highly accurate prediction - (1)

The transcription start sites identified in the preceding step (A) were examined for whether to be able to classify the presence or absence of lymph node metastasis using only one expression level. It was confirmed that both of the samples for transcription start site extraction and the samples for validation can be classified with 100% specificity and 100% sensitivity by setting some threshold for each transcription start site. Examples of the threshold are shown in Table 2 (when the largest value for a certain group is smaller than the smallest value for the other groups, an average thereof is shown in Table 2).

**[Table 2]**

| SEQ ID NO | Threshold (threshold_cpm) |
|---|---|
| 208 | 0.663483912 |
| 264 | 0.593907293 |
| 237 | 0.183136313 |
| 270 | 0.24217261 |
| 57 | 0.828673443 |
| 274 | 1.011158188 |
| 108 | 0.493392643 |
| 182 | 0.231203537 |

### (C) Selection of transcription start site for highly accurate prediction - (2)

The 277 transcription start sites identified above were examined for whether to be able to classify the presence or absence of lymph node metastasis using a plurality of expression levels. As an example, the construction of logistic regression models, which are one of the generalized linear models, is discussed here. This is one of the simplest models in the case of assuming that a dependent variable (Y) indicating the presence or absence of lymph node metastasis is stochastically predicted using the expression levels of the transcription start sites as explanatory variables (Xi; logarithm of the counts per million).

As for every combination (38,226 sets) of two transcription start sites selected from the 277 transcription start sites described above, logistic regression models for estimating the presence or absence of lymph node metastasis were constructed for the samples for transcription start site extraction with their expression levels as explanatory variables, and combinations were selected such that both of the samples for transcription start site extraction and the samples for validation can be classified with 100% specificity and 100% sensitivity (Tables 3-1 to 3-6).

Although the logistic regression models, one of the simplest machine learning systems, were adopted here, more robust prediction is possible through the proper use of other mathematical models by a method for assaying the expression of the TSS, combinations with the expression levels or genotypes of other genes or the like, etc.

**[Table 3-1]**

| | |
|---|---|
| 1) SEQ ID NO: 178 * SEQ ID NO: 67 | 41) SEQ ID NO: 175 * SEQ ID NO: 206 |
| 2) SEQ ID NO: 152 * SEQ ID NO: 175 | 42) SEQ ID NO: 175 * SEQ ID NO: 116 |
| 3) SEQ ID NO: 152 * SEQ ID NO: 67 | 43) SEQ ID NO: 175 * SEQ ID NO: 99 |
| 4) SEQ ID NO: 152 * SEQ ID NO: 180 | 44) SEQ ID NO: 175 * SEQ ID NO: 173 |
| 5) SEQ ID NO: 175 * SEQ ID NO: 23 | 45) SEQ ID NO: 175 * SEQ ID NO: 146 |
| 6) SEQ ID NO: 175 * SEQ ID NO: 84 | 46) SEQ ID NO: 175 * SEQ ID NO: 235 |
| 7) SEQ ID NO: 175 * SEQ ID NO: 90 | 47) SEQ ID NO: 175 * SEQ ID NO: 160 |
| 8) SEQ ID NO: 175 * SEQ ID NO: 70 | 48) SEQ ID NO: 175 * SEQ ID NO: 215 |
| 9) SEQ ID NO: 175 * SEQ ID NO: 55 | 49) SEQ ID NO: 175 * SEQ ID NO: 166 |
| 10) SEQ ID NO: 175 * SEQ ID NO: 243 | 50) SEQ ID NO: 175 * SEQ ID NO: 97 |
| 11) SEQ ID NO: 175 * SEQ ID NO: 249 | 51) SEQ ID NO: 33 * SEQ ID NO: 19 |
| 12) SEQ ID NO: 175 * SEQ ID NO: 113 | 52) SEQ ID NO: 32 * SEQ ID NO: 93 |
| 13) SEQ ID NO: 175 * SEQ ID NO: 168 | 53) SEQ ID NO: 32 * SEQ ID NO: 166 |
| 14) SEQ ID NO: 175 * SEQ ID NO: 142 | 54) SEQ ID NO: 53 * SEQ ID NO: 81 |
| 15) SEQ ID NO: 175 * SEQ ID NO: 38 | 55) SEQ ID NO: 53 * SEQ ID NO: 91 |
| 16) SEQ ID NO: 175 * SEQ ID NO: 265 | 56) SEQ ID NO: 53 * SEQ ID NO: 158 |
| 17) SEQ ID NO: 175 * SEQ ID NO: 24 | 57) SEQ ID NO: 53 * SEQ ID NO: 206 |
| 18) SEQ ID NO: 175 * SEQ ID NO: 114 | 58) SEQ ID NO: 101 * SEQ ID NO: 67 |
| 19) SEQ ID NO: 175 * SEQ ID NO: 81 | 59) SEQ ID NO: 101 * SEQ ID NO: 94 |
| 20) SEQ ID NO: 175 * SEQ ID NO: 96 | 60) SEQ ID NO: 101 * SEQ ID NO: 139 |
| 21) SEQ ID NO: 175 * SEQ ID NO: 62 | 61) SEQ ID NO: 149 * SEQ ID NO: 139 |
| 22) SEQ ID NO: 175 * SEQ ID NO: 39 | 62) SEQ ID NO: 69 * SEQ ID NO: 144 |
| 23) SEQ ID NO: 175 * SEQ ID NO: 246 | 63) SEQ ID NO: 69 * SEQ ID NO: 73 |
| 24) SEQ ID NO: 175 * SEQ ID NO: 93 | 64) SEQ ID NO: 69 * SEQ ID NO: 91 |
| 25) SEQ ID NO: 175 * SEQ ID NO: 252 | 65) SEQ ID NO: 69 * SEQ ID NO: 158 |
| 26) SEQ ID NO: 175 * SEQ ID NO: 17 | 66) SEQ ID NO: 69 * SEQ ID NO: 155 |
| 27) SEQ ID NO: 175 * SEQ ID NO: 194 | 67) SEQ ID NO: 69 * SEQ ID NO: 206 |
| 28) SEQ ID NO: 175 * SEQ ID NO: 110 | 68) SEQ ID NO: 69 * SEQ ID NO: 99 |
| 29) SEQ ID NO: 175 * SEQ ID NO: 58 | 69) SEQ ID NO: 84 * SEQ ID NO: 139 |
| 30) SEQ ID NO: 175 * SEQ ID NO: 11 | 70) SEQ ID NO: 205 * SEQ ID NO: 67 |
| 31) SEQ ID NO: 175 * SEQ ID NO: 211 | 71) SEQ ID NO: 205 * SEQ ID NO: 185 |
| 32) SEQ ID NO: 175 * SEQ ID NO: 141 | 72) SEQ ID NO: 205 * SEQ ID NO: 139 |
| 33) SEQ ID NO: 175 * SEQ ID NO: 47 | 73) SEQ ID NO: 250 * SEQ ID NO: 67 |
| 34) SEQ ID NO: 175 * SEQ ID NO: 159 | 74) SEQ ID NO: 70 * SEQ ID NO: 144 |
| 35) SEQ ID NO: 175 * SEQ ID NO: 155 | 75) SEQ ID NO: 70 * SEQ ID NO: 73 |
| 36) SEQ ID NO: 175 * SEQ ID NO: 232 | 76) SEQ ID NO: 70 * SEQ ID NO: 139 |
| 37) SEQ ID NO: 175 * SEQ ID NO: 204 | 77) SEQ ID NO: 70 * SEQ ID NO: 91 |
| 38) SEQ ID NO: 175 * SEQ ID NO: 228 | 78) SEQ ID NO: 55 * SEQ ID NO: 104 |
| 39) SEQ ID NO: 175 * SEQ ID NO: 187 | 79) SEQ ID NO: 55 * SEQ ID NO: 143 |
| 40) SEQ ID NO: 175 * SEQ ID NO: 199 | 80) SEQ ID NO: 186 * SEQ ID NO: 228 |

**[Table 3-2]**

| | |
|---|---|
| 81) SEQ ID NO: 186 * SEQ ID NO: 163 | 121) SEQ ID NO: 67 * SEQ ID NO: 142 |
| 82) SEQ ID NO: 186 * SEQ ID NO: 235 | 122) SEQ ID NO: 67 * SEQ ID NO: 244 |
| 83) SEQ ID NO: 186 * SEQ ID NO: 215 | 123) SEQ ID NO: 67 * SEQ ID NO: 38 |
| 84) SEQ ID NO: 75 * SEQ ID NO: 8 | 124) SEQ ID NO: 67 * SEQ ID NO: 265 |
| 85) SEQ ID NO: 75 * SEQ ID NO: 180 | 125) SEQ ID NO: 67 * SEQ ID NO: 195 |
| 86) SEQ ID NO: 234 * SEQ ID NO: 120 | 126) SEQ ID NO: 67 * SEQ ID NO: 170 |
| 87) SEQ ID NO: 227 * SEQ ID NO: 67 | 127) SEQ ID NO: 67 * SEQ ID NO: 231 |
| 88) SEQ ID NO: 227 * SEQ ID NO: 180 | 128) SEQ ID NO: 67 * SEQ ID NO: 213 |
| 89) SEQ ID NO: 243 * SEQ ID NO: 67 | 129) SEQ ID NO: 67 * SEQ ID NO: 150 |
| 90) SEQ ID NO: 243 * SEQ ID NO: 180 | 130) SEQ ID NO: 67 * SEQ ID NO: 92 |
| 91) SEQ ID NO: 85 * SEQ ID NO: 139 | 131) SEQ ID NO: 67 * SEQ ID NO: 135 |
| 92) SEQ ID NO: 249 * SEQ ID NO: 67 | 132) SEQ ID NO: 67 * SEQ ID NO: 229 |
| 93) SEQ ID NO: 113 * SEQ ID NO: 67 | 133) SEQ ID NO: 67 * SEQ ID NO: 46 |
| 94) SEQ ID NO: 113 * SEQ ID NO: 180 | 134) SEQ ID NO: 67 * SEQ ID NO: 259 |
| 95) SEQ ID NO: 201 * SEQ ID NO: 67 | 135) SEQ ID NO: 67 * SEQ ID NO: 174 |
| 96) SEQ ID NO: 201 * SEQ ID NO: 180 | 136) SEQ ID NO: 67 * SEQ ID NO: 169 |
| 97) SEQ ID NO: 107 * SEQ ID NO: 67 | 137) SEQ ID NO: 67 * SEQ ID NO: 267 |
| 98) SEQ ID NO: 107 * SEQ ID NO: 139 | 138) SEQ ID NO: 67 * SEQ ID NO: 31 |
| 99) SEQ ID NO: 13 * SEQ ID NO: 67 | 139) SEQ ID NO: 67 * SEQ ID NO: 103 |
| 100) SEQ ID NO: 13 * SEQ ID NO: 73 | 140) SEQ ID NO: 67 * SEQ ID NO: 49 |
| 101) SEQ ID NO: 230 * SEQ ID NO: 67 | 141) SEQ ID NO: 67 * SEQ ID NO: 236 |
| 102) SEQ ID NO: 253 * SEQ ID NO: 67 | 142) SEQ ID NO: 67 * SEQ ID NO: 14 |
| 103) SEQ ID NO: 136 * SEQ ID NO: 139 | 143) SEQ ID NO: 67 * SEQ ID NO: 224 |
| 104) SEQ ID NO: 189 * SEQ ID NO: 67 | 144) SEQ ID NO: 67 * SEQ ID NO: 258 |
| 105) SEQ ID NO: 189 * SEQ ID NO: 180 | 145) SEQ ID NO: 67 * SEQ ID NO: 181 |
| 106) SEQ ID NO: 35 * SEQ ID NO: 67 | 146) SEQ ID NO: 67 * SEQ ID NO: 43 |
| 107) SEQ ID NO: 35 * SEQ ID NO: 180 | 147) SEQ ID NO: 67 * SEQ ID NO: 252 |
| 108) SEQ ID NO: 233 * SEQ ID NO: 67 | 148) SEQ ID NO: 67 * SEQ ID NO: 161 |
| 109) SEQ ID NO: 241 * SEQ ID NO: 67 | 149) SEQ ID NO: 67 * SEQ ID NO: 172 |
| 110) SEQ ID NO: 67 * SEQ ID NO: 251 | 150) SEQ ID NO: 67 * SEQ ID NO: 188 |
| 111) SEQ ID NO: 67 * SEQ ID NO: 197 | 151) SEQ ID NO: 67 * SEQ ID NO: 73 |
| 112) SEQ ID NO: 67 * SEQ ID NO: 111 | 152) SEQ ID NO: 67 * SEQ ID NO: 194 |
| 113) SEQ ID NO: 67 * SEQ ID NO: 86 | 153) SEQ ID NO: 67 * SEQ ID NO: 273 |
| 114) SEQ ID NO: 67 * SEQ ID NO: 266 | 154) SEQ ID NO: 67 * SEQ ID NO: 248 |
| 115) SEQ ID NO: 67 * SEQ ID NO: 261 | 155) SEQ ID NO: 67 * SEQ ID NO: 82 |
| 116) SEQ ID NO: 67 * SEQ ID NO: 218 | 156) SEQ ID NO: 67 * SEQ ID NO: 239 |
| 117) SEQ ID NO: 67 * SEQ ID NO: 191 | 157) SEQ ID NO: 67 * SEQ ID NO: 238 |
| 118) SEQ ID NO: 67 * SEQ ID NO: 100 | 158) SEQ ID NO: 67 * SEQ ID NO: 203 |
| 119) SEQ ID NO: 67 * SEQ ID NO: 127 | 159) SEQ ID NO: 67 * SEQ ID NO: 257 |
| 120) SEQ ID NO: 67 * SEQ ID NO: 168 | 160) SEQ ID NO: 67 * SEQ ID NO: 242 |

**[Table 3-3]**

| | |
|---|---|
| 161) SEQ ID NO: 67 * SEQ ID NO: 106 | 201) SEQ ID NO: 105 * SEQ ID NO: 212 |
| 162) SEQ ID NO: 67 * SEQ ID NO: 177 | 202) SEQ ID NO: 105 * SEQ ID NO: 206 |
| 163) SEQ ID NO: 67 * SEQ ID NO: 25 | 203) SEQ ID NO: 27 * SEQ ID NO: 73 |
| 164) SEQ ID NO: 67 * SEQ ID NO: 196 | 204) SEQ ID NO: 86 * SEQ ID NO: 38 |
| 165) SEQ ID NO: 67 * SEQ ID NO: 58 | 205) SEQ ID NO: 86 * SEQ ID NO: 170 |
| 166) SEQ ID NO: 67 * SEQ ID NO: 226 | 206) SEQ ID NO: 86 * SEQ ID NO: 66 |
| 167) SEQ ID NO: 67 * SEQ ID NO: 183 | 207) SEQ ID NO: 86 * SEQ ID NO: 166 |
| 168) SEQ ID NO: 67 * SEQ ID NO: 269 | 208) SEQ ID NO: 19 * SEQ ID NO: 131 |
| 169) SEQ ID NO: 67 * SEQ ID NO: 21 | 209) SEQ ID NO: 19 * SEQ ID NO: 128 |
| 170) SEQ ID NO: 67 * SEQ ID NO: 119 | 210) SEQ ID NO: 19 * SEQ ID NO: 139 |
| 171) SEQ ID NO: 67 * SEQ ID NO: 78 | 211) SEQ ID NO: 147 * SEQ ID NO: 94 |
| 172) SEQ ID NO: 67 * SEQ ID NO: 184 | 212) SEQ ID NO: 147 * SEQ ID NO: 73 |
| 173) SEQ ID NO: 67 * SEQ ID NO: 225 | 213) SEQ ID NO: 147 * SEQ ID NO: 91 |
| 174) SEQ ID NO: 67 * SEQ ID NO: 200 | 214) SEQ ID NO: 147 * SEQ ID NO: 158 |
| 175) SEQ ID NO: 67 * SEQ ID NO: 63 | 215) SEQ ID NO: 100 * SEQ ID NO: 166 |
| 176) SEQ ID NO: 67 * SEQ ID NO: 128 | 216) SEQ ID NO: 127 * SEQ ID NO: 121 |
| 177) SEQ ID NO: 67 * SEQ ID NO: 254 | 217) SEQ ID NO: 168 * SEQ ID NO: 180 |
| 178) SEQ ID NO: 67 * SEQ ID NO: 171 | 218) SEQ ID NO: 142 * SEQ ID NO: 180 |
| 179) SEQ ID NO: 67 * SEQ ID NO: 211 | 219) SEQ ID NO: 244 * SEQ ID NO: 180 |
| 180) SEQ ID NO: 67 * SEQ ID NO: 64 | 220) SEQ ID NO: 38 * SEQ ID NO: 180 |
| 181) SEQ ID NO: 67 * SEQ ID NO: 263 | 221) SEQ ID NO: 265 * SEQ ID NO: 180 |
| 182) SEQ ID NO: 67 * SEQ ID NO: 192 | 222) SEQ ID NO: 170 * SEQ ID NO: 139 |
| 183) SEQ ID NO: 67 * SEQ ID NO: 36 | 223) SEQ ID NO: 231 * SEQ ID NO: 135 |
| 184) SEQ ID NO: 67 * SEQ ID NO: 256 | 224) SEQ ID NO: 231 * SEQ ID NO: 14 |
| 185) SEQ ID NO: 67 * SEQ ID NO: 268 | 225) SEQ ID NO: 231 * SEQ ID NO: 139 |
| 186) SEQ ID NO: 67 * SEQ ID NO: 219 | 226) SEQ ID NO: 24 * SEQ ID NO: 143 |
| 187) SEQ ID NO: 67 * SEQ ID NO: 139 | 227) SEQ ID NO: 15 * SEQ ID NO: 139 |
| 188) SEQ ID NO: 67 * SEQ ID NO: 209 | 228) SEQ ID NO: 30 * SEQ ID NO: 73 |
| 189) SEQ ID NO: 67 * SEQ ID NO: 207 | 229) SEQ ID NO: 30 * SEQ ID NO: 158 |
| 190) SEQ ID NO: 67 * SEQ ID NO: 1 | 230) SEQ ID NO: 94 * SEQ ID NO: 81 |
| 191) SEQ ID NO: 67 * SEQ ID NO: 220 | 231) SEQ ID NO: 94 * SEQ ID NO: 11 |
| 192) SEQ ID NO: 67 * SEQ ID NO: 193 | 232) SEQ ID NO: 94 * SEQ ID NO: 200 |
| 193) SEQ ID NO: 67 * SEQ ID NO: 221 | 233) SEQ ID NO: 167 * SEQ ID NO: 73 |
| 194) SEQ ID NO: 67 * SEQ ID NO: 222 | 234) SEQ ID NO: 81 * SEQ ID NO: 143 |
| 195) SEQ ID NO: 67 * SEQ ID NO: 180 | 235) SEQ ID NO: 81 * SEQ ID NO: 144 |
| 196) SEQ ID NO: 67 * SEQ ID NO: 121 | 236) SEQ ID NO: 81 * SEQ ID NO: 20 |
| 197) SEQ ID NO: 67 * SEQ ID NO: 146 | 237) SEQ ID NO: 81 * SEQ ID NO: 73 |
| 198) SEQ ID NO: 67 * SEQ ID NO: 223 | 238) SEQ ID NO: 81 * SEQ ID NO: 66 |
| 199) SEQ ID NO: 67 * SEQ ID NO: 166 | 239) SEQ ID NO: 81 * SEQ ID NO: 91 |
| 200) SEQ ID NO: 2 * SEQ ID NO: 147 | 240) SEQ ID NO: 81 * SEQ ID NO: 77 |

**[Table 3-4]**

| | |
|---|---|
| 241) SEQ ID NO: 81 * SEQ ID NO: 210 | 281) SEQ ID NO: 31 * SEQ ID NO: 206 |
| 242) SEQ ID NO: 96 * SEQ ID NO: 104 | 282) SEQ ID NO: 31 * SEQ ID NO: 115 |
| 243) SEQ ID NO: 96 * SEQ ID NO: 144 | 283) SEQ ID NO: 5 * SEQ ID NO: 73 |
| 244) SEQ ID NO: 96 * SEQ ID NO: 126 | 284) SEQ ID NO: 49 * SEQ ID NO: 180 |
| 245) SEQ ID NO: 96 * SEQ ID NO: 73 | 285) SEQ ID NO: 4 * SEQ ID NO: 8 |
| 246) SEQ ID NO: 96 * SEQ ID NO: 56 | 286) SEQ ID NO: 4 * SEQ ID NO: 180 |
| 247) SEQ ID NO: 92 * SEQ ID NO: 73 | 287) SEQ ID NO: 148 * SEQ ID NO: 8 |
| 248) SEQ ID NO: 98 * SEQ ID NO: 143 | 288) SEQ ID NO: 148 * SEQ ID NO: 180 |
| 249) SEQ ID NO: 104 * SEQ ID NO: 51 | 289) SEQ ID NO: 179 * SEQ ID NO: 185 |
| 250) SEQ ID NO: 104 * SEQ ID NO: 166 | 290) SEQ ID NO: 51 * SEQ ID NO: 91 |
| 251) SEQ ID NO: 216 * SEQ ID NO: 180 | 291) SEQ ID NO: 51 * SEQ ID NO: 210 |
| 252) SEQ ID NO: 216 * SEQ ID NO: 206 | 292) SEQ ID NO: 51 * SEQ ID NO: 232 |
| 253) SEQ ID NO: 135 * SEQ ID NO: 120 | 293) SEQ ID NO: 51 * SEQ ID NO: 206 |
| 254) SEQ ID NO: 112 * SEQ ID NO: 139 | 294) SEQ ID NO: 145 * SEQ ID NO: 139 |
| 255) SEQ ID NO: 62 * SEQ ID NO: 144 | 295) SEQ ID NO: 7 * SEQ ID NO: 139 |
| 256) SEQ ID NO: 62 * SEQ ID NO: 89 | 296) SEQ ID NO: 198 * SEQ ID NO: 139 |
| 257) SEQ ID NO: 62 * SEQ ID NO: 73 | 297) SEQ ID NO: 198 * SEQ ID NO: 210 |
| 258) SEQ ID NO: 62 * SEQ ID NO: 139 | 298) SEQ ID NO: 20 * SEQ ID NO: 73 |
| 259) SEQ ID NO: 62 * SEQ ID NO: 91 | 299) SEQ ID NO: 20 * SEQ ID NO: 215 |
| 260) SEQ ID NO: 62 * SEQ ID NO: 158 | 300) SEQ ID NO: 153 * SEQ ID NO: 93 |
| 261) SEQ ID NO: 62 * SEQ ID NO: 155 | 301) SEQ ID NO: 153 * SEQ ID NO: 252 |
| 262) SEQ ID NO: 124 * SEQ ID NO: 166 | 302) SEQ ID NO: 153 * SEQ ID NO: 171 |
| 263) SEQ ID NO: 118 * SEQ ID NO: 139 | 303) SEQ ID NO: 153 * SEQ ID NO: 141 |
| 264) SEQ ID NO: 143 * SEQ ID NO: 17 | 304) SEQ ID NO: 153 * SEQ ID NO: 139 |
| 265) SEQ ID NO: 143 * SEQ ID NO: 273 | 305) SEQ ID NO: 153 * SEQ ID NO: 255 |
| 266) SEQ ID NO: 143 * SEQ ID NO: 28 | 306) SEQ ID NO: 153 * SEQ ID NO: 232 |
| 267) SEQ ID NO: 143 * SEQ ID NO: 171 | 307) SEQ ID NO: 153 * SEQ ID NO: 204 |
| 268) SEQ ID NO: 143 * SEQ ID NO: 158 | 308) SEQ ID NO: 153 * SEQ ID NO: 228 |
| 269) SEQ ID NO: 143 * SEQ ID NO: 155 | 309) SEQ ID NO: 153 * SEQ ID NO: 199 |
| 270) SEQ ID NO: 143 * SEQ ID NO: 116 | 310) SEQ ID NO: 153 * SEQ ID NO: 48 |
| 271) SEQ ID NO: 143 * SEQ ID NO: 99 | 311) SEQ ID NO: 153 * SEQ ID NO: 116 |
| 272) SEQ ID NO: 143 * SEQ ID NO: 160 | 312) SEQ ID NO: 153 * SEQ ID NO: 146 |
| 273) SEQ ID NO: 143 * SEQ ID NO: 215 | 313) SEQ ID NO: 153 * SEQ ID NO: 235 |
| 274) SEQ ID NO: 144 * SEQ I17 NO: 51 | 314) SEQ ID NO: 153 * SEQ ID NO: 202 |
| 275) SEQ ID NO: 40 * SEQ ID NO: 139 | 315) SEQ ID NO: 153 * SEQ ID NO: 215 |
| 276) SEQ ID NO: 39 * SEQ ID NO: 139 | 316) SEQ ID NO: 153 * SEQ ID NO: 166 |
| 277) SEQ ID NO: 50 * SEQ ID NO: 206 | 317) SEQ ID NO: 93 * SEQ ID NO: 73 |
| 278) SEQ ID NO: 246 * SEQ ID NO: 22 | 318) SEQ ID NO: 93 * SEQ ID NO: 22 |
| 279) SEQ ID NO: 31 * SEQ ID NO: 158 | 319) SEQ ID NO: 93 * SEQ ID NO: 139 |
| 280) SEQ ID NO: 31 * SEQ ID NO: 159 | 320) SEQ ID NO: 93 * SEQ ID NO: 77 |

**[Table 3-5]**

| | |
|---|---|
| 321) SEQ ID NO: 52 * SEQ ID NO: 139 | 361) SEQ ID NO: 22 * SEQ ID NO: 164 |
| 322) SEQ ID NO: 252 * SEQ ID NO: 165 | 362) SEQ ID NO: 22 * SEQ ID NO: 166 |
| 323) SEQ ID NO: 252 * SEQ ID NO: 180 | 363) SEQ ID NO: 110 * SEQ ID NO: 139 |
| 324) SEQ ID NO: 89 * SEQ ID NO: 139 | 364) SEQ ID NO: 109 * SEQ ID NO: 139 |
| 325) SEQ ID NO: 126 * SEQ ID NO: 139 | 365) SEQ ID NO: 109 * SEQ ID NO: 99 |
| 326) SEQ ID NO: 172 * SEQ ID NO: 139 | 366) SEQ ID NO: 8 * SEQ ID NO: 130 |
| 327) SEQ ID NO: 65 * SEQ ID NO: 139 | 367) SEQ ID NO: 8 * SEQ ID NO: 171 |
| 328) SEQ ID NO: 73 * SEQ ID NO: 109 | 368) SEQ ID NO: 8 * SEQ ID NO: 77 |
| 329) SEQ ID NO: 73 * SEQ ID NO: 200 | 369) SEQ ID NO: 8 * SEQ ID NO: 158 |
| 330) SEQ ID NO: 73 * SEQ ID NO: 277 | 370) SEQ ID NO: 8 * SEQ ID NO: 132 |
| 331) SEQ ID NO: 73 * SEQ ID NO: 268 | 371) SEQ ID NO: 8 * SEQ ID NO: 206 |
| 332) SEQ ID NO: 73 * SEQ ID NO: 141 | 372) SEQ ID NO: 8 * SEQ ID NO: 99 |
| 333) SEQ ID NO: 73 * SEQ ID NO: 139 | 373) SEQ ID NO: 8 * SEQ ID NO: 115 |
| 334) SEQ ID NO: 73 * SEQ ID NO: 132 | 374) SEQ ID NO: 8 * SEQ ID NO: 163 |
| 335) SEQ ID NO: 73 * SEQ ID NO: 88 | 375) SEQ ID NO: 8 * SEQ ID NO: 215 |
| 336) SEQ ID NO: 194 * SEQ ID NO: 180 | 376) SEQ ID NO: 8 * SEQ ID NO: 123 |
| 337) SEQ ID NO: 273 * SEQ ID NO: 139 | 377) SEQ ID NO: 203 * SEQ ID NO: 206 |
| 338) SEQ ID NO: 56 * SEQ ID NO: 139 | 378) SEQ ID NO: 196 * SEQ ID NO: 139 |
| 339) SEQ ID NO: 56 * SEQ ID NO: 158 | 379) SEQ ID NO: 133 * SEQ ID NO: 139 |
| 340) SEQ ID NO: 156 * SEQ ID NO: 139 | 380) SEQ ID NO: 76 * SEQ ID NO: 139 |
| 341) SEQ ID NO: 22 * SEQ ID NO: 139 | 381) SEQ ID NO: 269 * SEQ ID NO: 139 |
| 342) SEQ ID NO: 22 * SEQ ID NO: 255 | 382) SEQ ID NO: 28 * SEQ ID NO: 121 |
| 343) SEQ ID NO: 22 * SEQ ID NO: 158 | 383) SEQ ID NO: 79 * SEQ ID NO: 210 |
| 344) SEQ ID NO: 22 * SEQ ID NO: 159 | 384) SEQ ID NO: 122 * SEQ ID NO: 139 |
| 345) SEQ ID NO: 22 * SEQ ID NO: 155 | 385) SEQ ID NO: 78 * SEQ ID NO: 180 |
| 346) SEQ ID NO: 22 * SEQ ID NO: 232 | 386) SEQ ID NO: 130 * SEQ ID NO: 180 |
| 347) SEQ ID NO: 22 * SEQ ID NO: 204 | 387) SEQ ID NO: 6 * SEQ ID NO: 210 |
| 348) SEQ ID NO: 22 * SEQ ID NO: 228 | 388) SEQ ID NO: 128 * SEQ ID NO: 139 |
| 349) SEQ ID NO: 22 * SEQ ID NO: 187 | 389) SEQ ID NO: 128 * SEQ ID NO: 210 |
| 350) SEQ ID NO: 22 * SEQ ID NO: 190 | 390) SEQ ID NO: 128 * SEQ ID NO: 158 |
| 351) SEQ ID NO: 22 * SEQ ID NO: 199 | 391) SEQ ID NO: 128 * SEQ ID NO: 187 |
| 352) SEQ ID NO: 22 * SEQ ID NO: 206 | 392) SEQ ID NO: 128 * SEQ ID NO: 132 |
| 353) SEQ ID NO: 22 * SEQ ID NO: 116 | 393) SEQ ID NO: 128 * SEQ ID NO: 190 |
| 354) SEQ ID NO: 22 * SEQ ID NO: 162 | 394) SEQ ID NO: 128 * SEQ ID NO: 88 |
| 355) SEQ ID NO: 22 * SEQ ID NO: 117 | 395) SEQ ID NO: 128 * SEQ ID NO: 117 |
| 356) SEQ ID NO: 22 * SEQ ID NO: 99 | 396) SEQ ID NO: 128 * SEQ ID NO: 115 |
| 357) SEQ ID NO: 22 * SEQ ID NO: 173 | 397) SEQ ID NO: 128 * SEQ ID NO: 163 |
| 358) SEQ ID NO: 22 * SEQ ID NO: 146 | 398) SEQ ID NO: 128 * SEQ ID NO: 137 |
| 359) SEQ ID NO: 22 * SEQ ID NO: 160 | 399) SEQ ID NO: 128 * SEQ ID NO: 129 |
| 360) SEQ ID NO: 22 * SEQ ID NO: 215 | 400) SEQ ID NO: 120 * SEQ ID NO: 141 |

**[Table 3-6]**

| |
|---|
| 401) SEQ ID NO: 254 * SEQ ID NO: 180 |
| 402) SEQ ID NO: 171 * SEQ ID NO: 180 |
| 403) SEQ ID NO: 211 * SEQ ID NO: 180 |
| 404) SEQ ID NO: 64 * SEQ ID NO: 180 |
| 405) SEQ ID NO: 277 * SEQ ID NO: 232 |
| 406) SEQ ID NO: 277 * SEQ ID NO: 116 |
| 407) SEQ ID NO: 80 * SEQ ID NO: 91 |
| 408) SEQ ID NO: 80 * SEQ ID NO: 158 |
| 409) SEQ ID NO: 80 * SEQ ID NO: 206 |
| 410) SEQ ID NO: 185 * SEQ ID NO: 121 |
| 411) SEQ ID NO: 139 * SEQ ID NO: 41 |
| 412) SEQ ID NO: 139 * SEQ ID NO: 193 |
| 413) SEQ ID NO: 139 * SEQ ID NO: 121 |
| 414) SEQ ID NO: 139 * SEQ ID NO: 91 |
| 415) SEQ ID NO: 139 * SEQ ID NO: 158 |
| 416) SEQ ID NO: 139 * SEQ ID NO: 116 |
| 417) SEQ ID NO: 165 * SEQ ID NO: 166 |
| 418) SEQ ID NO: 193 * SEQ ID NO: 212 |
| 419) SEQ ID NO: 212 * SEQ ID NO: 163 |
| 420) SEQ ID NO: 180 * SEQ ID NO: 255 |
| 421) SEQ ID NO: 180 * SEQ ID NO: 48 |
| 422) SEQ ID NO: 180 * SEQ ID NO: 173 |
| 423) SEQ ID NO: 180 * SEQ ID NO: 163 |
| 424) SEQ ID NO: 180 * SEQ ID NO: 202 |
| 425) SEQ ID NO: 180 * SEQ ID NO: 123 |
| 426) SEQ ID NO: 180 * SEQ ID NO: 166 |
| 427) SEQ ID NO: 83 * SEQ ID NO: 166 |
| 428) SEQ ID NO: 121 * SEQ ID NO: 206 |
| 429) SEQ ID NO: 87 * SEQ ID NO: 206 |
| 430) SEQ ID NO: 77 * SEQ ID NO: 158 |
| 431) SEQ ID NO: 77 * SEQ ID NO: 173 |
| 432) SEQ ID NO: 77 * SEQ ID NO: 163 |
| 433) SEQ ID NO: 210 * SEQ ID NO: 232 |
| 434) SEQ ID NO: 206 * SEQ ID NO: 166 |

### Example 2

23 operatively harvested specimens (9 specimens with lymph node metastasis and 14 specimens without lymph node metastasis) different from the specimens used in Example 1 were used to prepare CAGE libraries and extract transcription start sites differing in activity between a group with lymph node metastasis and a group without lymph node metastasis, in the same way as in Example 1. As a result, the transcriptional activity levels of the transcription start sites shown in SEQ ID NOs: 35, 36, 63, 73, 140, 161, 189, 193, 205, and 219 were confirmed to significantly differ between the groups with and without metastasis (Table 4).

**[Table 4]**

| SEQ ID NO | Expression ratio [with metastasis/without metastasis] | Average expression | Pvalue | FDR |
|---|---|---|---|---|
| 35 | 95.83 | 3.46 | 5.88E-06 | 8.40E-03 |
| 36 | 39.30 | 0.72 | 1.11E-04 | 4.13E-02 |
| 63 | 71.61 | 0.83 | 8.00E-05 | 3.66E-02 |
| 73 | 11.81 | 9.30 | 7.64E-08 | 7.46E-04 |
| 140 | 4.35 | 19.33 | 3.42E-05 | 2.11E-02 |
| 161 | 40.26 | 1.34 | 6.49E-06 | 9.04E-03 |
| 189 | 75.83 | 0.70 | 1.36E-04 | 4.59E-02 |
| 193 | 5.38 | 9.46 | 6.64E-06 | 9.04E-03 |
| 205 | 70.95 | 5.40 | 2.75E-09 | 8.05E-05 |
| 219 | 5.68 | 14.37 | 2.08E-06 | 4.87E-03 |

### Example 3 Discrimination between endometrial cancer metastatic to lymph nodes and endometrial cancer without metastasis using TACC2 as marker

### (1) Specimen

The specimens used were operatively harvested specimens (5 mm each of endometrial tumor lesions collected from patients with endometrial cancer (well-differentiated adenocarcinoma) having 1/2 or less myometrial invasion) of patients under their comprehensive consent before operation which were already diagnosed as having lymph node metastasis or having no lymph node metastasis.

### (2) Detection of protein by immunochemical staining

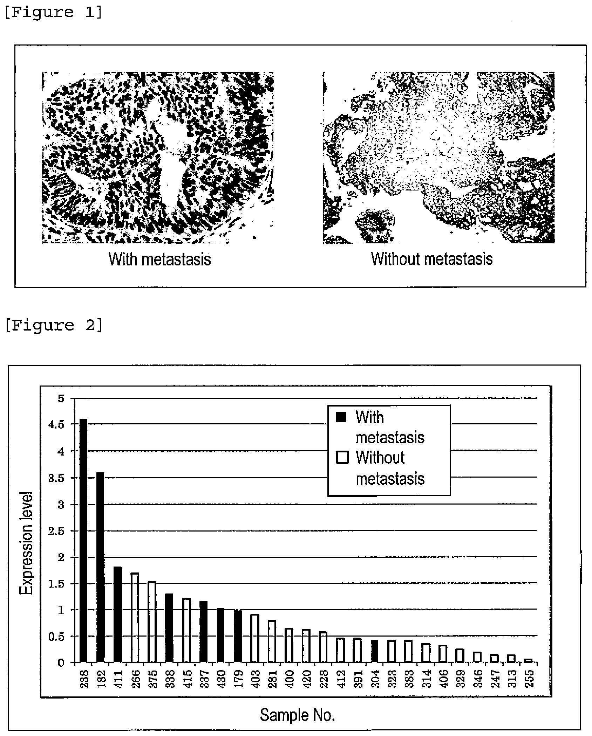

The biological sample isolated from each patient was fixed in formalin by a routine method, then embedded in paraffin, and sliced into a tissue section, which was attached to slide glass. The resultant was used as a section sample. Subsequently, the section sample was heat-treated (121°C, 10 min) for antigen retrieval. Subsequently, an anti-TACC2 antibody (EMD Millipore, "Polyclonal Rabbit anti-TACC2 antibody") (primary antibody; concentration: 0.5 µg/mL) was reacted therewith overnight (4°C). After thorough washing with a buffer solution, Anti-Rabbit Immunoglobulins (DAKO #E432) was used as a secondary antibody and reacted therewith for 30 minutes (20°C). After thorough washing with a buffer solution, a labeling reagent Peroxidase-conjugated streptavidin (DAKO #P0397) was used and reacted therewith for 30 minutes (20°C) (ABC method). After thorough washing with a buffer solution, color was developed using DAB. The positivity or negativity of the preparation was observed under an optical microscope. One example thereof is shown in Figure 1.

As a result, the staining pattern differed between the groups with and without lymph node metastasis, demonstrating that TACC2 can be used as a marker for discrimination therebetween.

### (3) Measurement of RNA level by qRT-PCR

Total RNA was extracted from each tissue specimen using an RNA extraction kit (Qiagen N.V., RAase Plus Mini Kit (#74134)). Reverse transcription reaction from RNA to cDNA was performed using TAKARA PrimeScript II 1^{st} strand cDNA Synthesis Kit (#6210A). Real-time PCR was carried out using ABI Fast SYBR Green Master Mix (#4385612), the following primers, and ABI 7500 Fast Real Time PCR System.
Primer F: CCAgTTgCTgAAgggCAgAA (SEQ ID NO: 278)
Primer R: gCggACCTTggAgTCTgAg (SEQ ID NO: 279)

The expression level of the TACC2 gene was corrected with the housekeeping gene SUDS4 and calculated as a relative variation from the control. The results are shown in Figure 2.

As seen from Figure 2, the TACC2 expression level obtained in the real-time PCR using these primers differed between the groups with and without lymph node metastasis, suggesting that TACC2 can be used as a marker for discrimination therebetween.

### Industrial Applicability

According to the present invention, the determination of the presence or absence of lymph node metastasis of endometrial cancer or the prediction of its occurrence can be objectively performed by examining primary endometrial lesions collected before or during operation. This can avoid unnecessary lymph node dissection and thus allows complications such as postoperative lymphedema to be decreased.

## Claims

1. A method for assessing lymph node metastasis or lymph node metastatic potential of endometrial cancer, comprising a step of measuring an expression level of an expression product of at least one DNA in a biological sample derived from a cancer tissue isolated from an endometrial cancer patient, wherein
the DNA comprises a region, represented by SEQ ID NO: 264.

2. The method according to claim 1, further comprising a step of comparing the expression level of the expression product of the DNA with a control level.

3. The method according to claim 1 or 2, further comprising a step of comparing the expression level of the expression product of the DNA with a threshold level.

4. The method according to any one of claims 1 to 3, wherein measurement of the expression level of the expression product is performed by measuring an amount of a transcription product or an amount of a translation product.

5. Use of a testing kit for assessing lymph node metastasis or lymph node metastatic potential of endometrial cancer in a method according to any one of claims 1 to 4, the testing kit comprising an oligonucleotide specifically hybridizing to a transcription product of the DNA, or an antibody recognizing a translation product of the DNA.

6. Use of an expression product of the DNA of claim 1 as a marker for assessing lymph node metastasis or lymph node metastatic potential of endometrial cancer.

7. A method for assessing lymph node metastasis or lymph node metastatic potential of endometrial cancer, comprising a step of measuring an expression level of TACC2 RNA or TACC2 protein in a biological sample derived from a cancer tissue isolated from an endometrial cancer patient.

8. Use of a testing kit for assessing lymph node metastasis or lymph node metastatic potential of endometrial cancer in a method according to claim 7, the testing kit comprising an oligonucleotide specifically hybridizing to TACC2 RNA, or an antibody recognizing TACC2 protein.

9. Use of TACC2 RNA or TACC2 protein as a marker for assessing lymph node metastasis or lymph node metastatic potential of endometrial cancer.

## Patentansprüche

1. Verfahren zur Beurteilung von Lymphknotenmetastase oder dem metastatischen Potenzial von Lymphknoten bei einem Endometriumkarzinom, umfassend einen Schritt des Messens des Expressionsniveaus eines Expressionsprodukts wenigstens einer DNA in einer biologischen Probe, die von einem Krebsgewebe stammt, das von einem Patienten mit einem Endometriumkarzinom isoliert wurde, wobei die DNA eine Region umfasst, die durch die SEQ ID Nr. 264 wiedergegeben wird.

2. Verfahren nach Anspruch 1, ferner einen Schritt des Vergleichens des Expressionsniveaus des Expressionsprodukts der DNA mit einem Kontrollwert umfassend.

3. Verfahren nach Anspruch 1 oder 2, ferner einen Schritt des Vergleichens des Expressionsniveaus des Expressionsprodukts der DNA mit einem Schwellenwert umfassend.

4. Verfahren nach einem der Ansprüche 1 bis 3, wobei die Messung des Expressionsniveaus des Expressionsprodukts durch Messen der Menge eines Transkriptionsprodukts oder der Menge eines Translationsprodukts durchgeführt wird.

5. Verwendung eines Testkits, zur Beurteilung von Lymphknotenmetastase oder dem metastatischen Potenzial von Lymphknoten bei einem Endometriumkarzinom in dem Verfahren nach einem der Ansprüche 1 bis 4, wobei der Testkit ein Oligonukleotid umfasst, das spezifisch an ein Transkriptionsprodukt der DNA hybridisiert, oder einen Antikörper, der das Translationsprodukt der DNA erkennt.

6. Verwendung eines Expressionsprodukts der DNA nach Anspruch 1, als Marker zur Beurteilung von Lymphknotenmetastase oder dem metastatischen Potenzial von Lymphknoten bei einem Endometriumkarzinom.

7. Verfahren zur Beurteilung von Lymphknotenmetastase oder dem metastatischen Potenzial von Lymphknoten bei einem Endometriumkarzinom, umfassend einen Schritt des Messens des Expressionsniveaus von TACC2-RNA oder dem TACC2-Protein in einer biologischen Probe, die von einem Krebsgewebe stammt, das von einem Patienten mit einem Endometriumkarzinom isoliert wurde.

8. Verwendung eines Testkits zur Beurteilung von Lymphknotenmetastase oder dem metastatischen Potenzial von Lymphknoten bei einem Endometriumkarzinom in dem Verfahren nach Anspruch 7, wobei der Testkit ein Oligonukleotid umfasst, das spezifisch an die TACC2-RNA hybridisiert oder einen Antikörper, der das TACC2-Protein erkennt.

9. Verwendung von TACC2-RNA oder des TACC2-Proteins als Marker, zur Beurteilung von Lymphknotenmetastase oder dem metastatischen Potenzial von Lymphknoten bei einem Endometriumkarzinom.

## Revendications

1. Procédé pour déterminer une métastase dans les ganglions lymphatiques ou un potentiel métastasique dans les ganglions lymphatiques d'un cancer de l'endomètre, comprenant une étape de mesure du niveau d'expression d'un produit d'expression d'au moins un ADN dans un échantillon biologique dérivé d'un tissu cancéreux isolé auprès d'une patiente souffrant d'un cancer de l'endomètre, dans lequel l'ADN comprend une région représentée par la SEQ ID NO : 264.

2. Procédé selon la revendication 1, comprenant en outre une étape de comparaison du niveau d'expression du produit d'expression de l'ADN avec un niveau témoin.

3. Procédé selon la revendication 1 ou 2, comprenant en outre une étape de comparaison du niveau d'expression du produit d'expression de l'ADN avec un niveau de seuil.

4. Procédé selon l'une quelconque des revendications 1 à 3, dans lequel la mesure du niveau d'expression du produit d'expression est effectuée par mesure de la quantité d'un produit de transcription ou de la quantité d'un produit de traduction.

5. Utilisation d'un kit de test pour déterminer une métastase dans les ganglions lymphatiques ou un potentiel métastasique dans les ganglions lymphatiques d'un cancer de l'endomètre dans un procédé selon l'une quelconque des revendications 1 à 4, le kit de test comprenant un oligonucléotide s'hybridant spécifiquement à un produit de transcription de l'ADN, ou un anticorps reconnaissant un produit de traduction de l'ADN.

6. Utilisation d'un produit d'expression de l'ADN de la revendication 1 en tant que marqueur pour déterminer une métastase dans les ganglions lymphatiques ou un potentiel métastasique dans les ganglions lymphatiques d'un cancer de l'endomètre.

7. Procédé pour déterminer une métastase dans les ganglions lymphatiques ou un potentiel métastasique dans les ganglions lymphatiques d'un cancer de l'endomètre, comprenant une étape de mesure du niveau d'expression d'ARN de TACC2 ou de protéine TACC2 dans un échantillon biologique dérivé d'un tissu cancéreux isolé auprès d'une patiente souffrant d'un cancer de l'endomètre.

8. Utilisation d'un kit de test pour déterminer une métastase dans les ganglions lymphatiques ou un potentiel métastasique dans les ganglions lymphatiques d'un cancer de l'endomètre dans un procédé selon la revendication 7, le kit de test comprenant un oligonucléotide s'hybridant spécifiquement à l'ARN de TACC2, ou un anticorps reconnaissant la protéine TACC2.

9. Utilisation d'ARN de TACC2 ou de protéine TACC2 en tant que marqueur pour déterminer une métastase dans les ganglions lymphatiques ou un potentiel métastasique dans les ganglions lymphatiques d'un cancer de l'endomètre.


### SEQUENCE LISTING

<110> JUNTENDO EDUCATIONAL FOUNDATION RIKEN
<120> METHOD FOR EVALUATING LYMPH NODE METASTASIS ABILITY IN UTERINE CANCER
<130> JUSM0008
<150> JP 2014-003191
   <151> 2014-01-10
<160> 279
<170> PatentIn version 3.5
<210> 1
   <211> 104
   <212> DNA
   <213> Homo sapiens
<220>
   <221> Transcript Start Site
   <222> (1)..(4)
<400> 1
   gggccttcac agcctccata ccagtgttgg ccctgtggac ccaccttatg tactcttaac 60
   ttgtcttttc tcattccttt gactcctccg gacttcgtag cccc 104
<210> 2
   <211> 114
   <212> DNA
   <213> Homo sapiens
<220>
   <221> Transcript Start Site
   <222> (1)..(14)
<400> 2
   attgcttaca tttgcttctg acacaactgt gttcactagc aacctcaaac agacaccatg 60
   gtgcatctga ctcctgagga gaagtctgcc gttactgccc tgtggggcaa ggtg 114
<210> 3
   <211> 116
   <212> DNA
   <213> Homo sapiens
<220>
   <221> Transcript Start Site
   <222> (1)..(16)
<400> 3
   cataaggcac aaactttcag agacagcaga gcacacaagc ttctaggaca agagccagga 60
   agaaaccacc ggaaggaacc atctcactgt gtgtaaacat gacttccaag ctggcc 116
<210> 4
   <211> 131
   <212> DNA
   <213> Homo sapiens
<220>
   <221> Transcript Start Site
   <222> (1)..(31)
<400> 4
   ctcttagtaa gattacacat gcaagcatcc ccattccagt gagttcaccc tctaaatcac 60
   cacgatgatt tagaggtaag gactaaaggg aaatcaaagg tagggggagg tttccaactg 120
   tgaatgggaa g 131
<210> 5
   <211> 116
   <212> DNA
   <213> Homo sapiens
<220>
   <221> Transcript Start Site
   <222> (1)..(16)
<400> 5
   cgcaatggca gtccactgcc ttgctgcagt cacagaatgg aaatctgcag aggcctccgc 60
   agtcacctaa tcactctcct cctcttcctg ttccattcag agacgatctg ccgacc 116
<210> 6
   <211> 124
   <212> DNA
   <213> Homo sapiens
<220>
   <221> Transcript Start Site
   <222> (1)..(24)
<400> 6
   cggtggtggc gagagtcagt ctttcagagc acaggatgga acaagaactc cagccactga       60
   ctgtctcatg tatctgcaag ggccgaggaa attaatgacc caaggaggct atgatatggt      120
   ccaa                                                                   124
<210> 7
   <211> 114
   <212> DNA
   <213> Homo sapiens
<220>
   <221> Transcript Start Site
   <222> (1)..(14)
<400> 7
   gctaggcact tctccttctt gccaccttgt gaagaaggtg tcttgctttc tcttcgcttt       60
   ctaccatgat tgtaagtttc ctgaaggctc cccagccatg ctgaacggtg agtc            114
<210> 8
   <211> 116
   <212> DNA
   <213> Homo sapiens
<220>
   <221> Transcript Start Site
   <222> (1)..(16)
<400> 8
   gggagccgcc gcgcccgtcc gcccgccgct ccgcgctcca cccagcgcac ccgggccccg       60
   cgcccccaac tgcctccggc ggccgcccag tcccgagggc gccatgagga gcctgt          116
<210> 9
   <211> 114
   <212> DNA
   <213> Homo sapiens
<220>
   <221> Transcript Start Site
   <222> (1)..(14)
<400> 9
   tgaggtgcaa ggagggttga gagccactgg cacaggggca gaagaggcta tttcttccat 60
   tccgccccct ctttgttgcg gaggcaaact gaggttcctg gagtcagaat ctat 114
<210> 10
   <211> 115
   <212> DNA
   <213> Homo sapiens
<220>
   <221> Transcript Start Site
   <222> (1)..(15)
<400> 10
   aaagcggtgc cagcggcgga gtctccaact gggagagctg cagctgccga gaggaggaga 60
   acgctgaggt cggtcggacc aacggacgcg ctgaccgctg ccaactgcag ctcgc 115
<210> 11
   <211> 119
   <212> DNA
   <213> Homo sapiens
<220>
   <221> Transcript Start Site
   <222> (1)..(19)
<400> 11
   aggggtgttc cacagggtag ctgggagttg gaagagccaa gaacgcctcc gagctctgga 60
   tttgagcttc tctgcccatg ggtgaagcgc ccatgctcag cttgtgagct tcttcccgg 119
<210> 12
   <211> 147
   <212> DNA
   <213> Homo sapiens
<220>
   <221> Transcript Start Site
   <222> (1)..(47)
<400> 12
   aactcagtca agctcagtga gcattctcag cacattgcct caacagcttc aaggtgagcc 60
   agctcaagac tttgctctcc accaggcaga agatgacaga ctgtgaattt ggatatattt 120
   acaggctggc tcaggactat ctgcagt 147
<210> 13
   <211> 114
   <212> DNA
   <213> Homo sapiens
<220>
   <221> Transcript Start Site
   <222> (1)..(14)
<400> 13
   cgcgctcgcc tccctcgctc cacgcgcgcc cggactcggc ggccaggctt gcgcgcggtt 60
   cccctcccgg tggtgagtcg cggccggggc ctcggggccg gcggcgggat gggc 114
<210> 14
   <211> 105
   <212> DNA
   <213> Homo sapiens
<220>
   <221> Transcript Start Site
   <222> (1)..(5)
<400> 14
   tggcaacctg cttgggtctc cttccacacc ttgaaaggtt tgttctttag ctctttgcac 60
   taagtcttgc tgctgctcac tctttgggtc cgcactgcct ttatg 105
<210> 15
   <211> 115
   <212> DNA
   <213> Homo sapiens
<220>
   <221> Transcript Start Site
   <222> (1)..(15)
<400> 15
   cccggtatgc tcttgcgacc actccaggca cgattggcac ccttctgcag aagtaaattg 60
   ccttgctgag aaagcttttt gccggagtgc tgattcttct ttgcggcacc aagca 115
<210> 16
   <211> 115
   <212> DNA
   <213> Homo sapiens
<220>
   <221> Transcript Start Site
   <222> (1)..(15)
<400> 16
   ggctcccagc cgagaggtgt cacccccagc gggcgcgggc cggagcacgg gcacccagca 60
   tgggggtact gctcacacag aggacgctgc tcagtaagta cttccttcct ccccg 115
<210> 17
   <211> 136
   <212> DNA
   <213> Homo sapiens
<220>
   <221> Transcript Start Site
   <222> (1)..(36)
<400> 17
   ggggatgggg cagtgtgaga tgacgggacc agagcacgtt cctggctgca gaggccacaa 60
   gtcacgctgt ctctgagagg tatggggaca ggggcgcaga ggaagaatga tcgcgagtcc 120
   tcagccctgt ttggct 136
<210> 18
   <211> 112
   <212> DNA
   <213> Homo sapiens
<220>
   <221> Transcript Start Site
   <222> (1)..(12)
<400> 18
   tcaaaggctt ggcccgttgc cttttccttc ccggcaatac tcggttcaat taggtcttgt 60
   cccctcatta tctgtgagga ctgaattcca cccccgcttt tcaacgcagg ct 112
<210> 19
   <211> 127
   <212> DNA
   <213> Homo sapiens
<220>
   <221> Transcript Start Site
   <222> (1)..(27)
<400> 19
   acgttcacca tacagacaca cgccactttg ctggagcatt cactaggcga ggcgctccat 60
   cggactcact agctgcactc atgaatcggc accatctgca ggatcacttt ctggaaatag 120
   acaagaa 127
<210> 20
   <211> 119
   <212> DNA
   <213> Homo sapiens
<220>
   <221> Transcript Start Site
   <222> (1)..(19)
<400> 20
   cggctgctgc tctgctccgg gttctgtcac tgtgtcggcg gtgcccagct cactggcccc      60
   ctccctctct tgtcgagcgt ggttgccaga gaggctccct cagccctgct ccgcggggt      119
<210> 21
   <211> 106
   <212> DNA
   <213> Homo sapiens
<220>
   <221> Transcript Start Site
   <222> (1)..(6)
<400> 21
   tggtaacctg ctcgggtccc cttccacatc atggaagctt tgttcttttg ctcttagcaa      60
   taaatcttgc tgctgctcac tctttgggtc tgcactacct ttctga                    106
<210> 22
   <211> 117
   <212> DNA
   <213> Homo sapiens
<220>
   <221> Transcript Start Site
   <222> (1)..(17)
<400> 22
   agagaatgtt ggtgaagatc ttaacaccac gccttgagca agtcgcaaga gcgggaggac      60
   acagaccagg aaccgagaag ggacaagcac atggaagcca gcccagcatc cgggccc       117
<210> 23
   <211> 127
   <212> DNA
   <213> Homo sapiens
<220>
   <221> Transcript Start Site
   <222> (1)..(27)
<400> 23
   tgggccccat tctactcatc tggcccagaa agtgaacacc ttgggggcca ctaaggcagc      60
   cctgctaggg gagacgctcc aacctgtctt ctctctgtct cctggcagct ctcttggcct     120
   cctagtt                                                               127
<210> 24
   <211> 113
   <212> DNA
   <213> Homo sapiens
<220>
   <221> Transcript Start Site
   <222> (1)..(13)
<400> 24
   ggcagcagca acctgctctg gttcccttcc acgctgtgga agctttgttc ttttggtctt       60
   catgataaat cttgctgctg ctcactcgtt gggtccgtgc cacctttaag agc             113
<210> 25
   <211> 124
   <212> DNA
   <213> Homo sapiens
<220>
   <221> Transcript Start Site
   <222> (1)..(24)
<400> 25
   aacagggaaa agagacaaga gatctaaaat atctaggttg actcctgtac ctgagcgacc       60
   tgcgatctcc accgccgccg cgacgaattg gcctctgggc ggggactttt agccaaaagg      120
   aaaa                                                                   124
<210> 26
   <211> 131
   <212> DNA
   <213> Homo sapiens
<220>
   <221> Transcript Start Site
   <222> (1)..(31)
<400> 26
   gctgtcccgg gagccctcag cagcagttgg agctggtgca caggaaggat gaggaagacc       60
   aggctctggg ggctgctgtg gatgctcttt gtctcaggta agacaaggaa gaggactgag      120
   caggggaggc g                                                           131
<210> 27
   <211> 139
   <212> DNA
   <213> Homo sapiens
<220>
   <221> Transcript Start Site
   <222> (1)..(39)
<400> 27
   gagcttcctc gctgtcccca ctctccctcg gctagcagcc tgggcacacg gacagacgga 60
   ctgacggact ctcgagcgga cagcgcagct agcggggcgc gggcgctggg cgtcgacggc 120
   cagccccagc cttccccgc 139
<210> 28
   <211> 174
   <212> DNA
   <213> Homo sapiens
<220>
   <221> Transcript Start Site
   <222> (1)..(74)
<400> 28
   ccctcttttg cagggttgtg gacttcctgc tgaaggccat catgcgcacc atgtggttcg 60
   ccggcggctt ccaccgggtg gccgtgaagg ggcggcaggc gctgcccacc gaggcggcca 120
   tcctcacgct cgcgcctcac tcgtcctact tcgacgccat ccctgtgacc atga 174
<210> 29
   <211> 123
   <212> DNA
   <213> Homo sapiens
<220>
   <221> Transcript Start Site
   <222> (1)..(23)
<400> 29
   gagggccaga cagccacaga gctcctggcg tgggcaaggc tggccaagga tggcgacgcc 60
   caggggcctg ggggccctgc tcctgctcct cctgctcccg acctcaggtg agtggctggc 120
   acc 123
<210> 30
   <211> 116
   <212> DNA
   <213> Homo sapiens
<220>
   <221> Transcript Start Site
   <222> (1)..(16)
<400> 30
   gcccgttcgg ttccgggcgt taccatcgtc cgtgcgcacc gcccggcgtc caggtgagtc 60
   tcccatctgc agagacgcgg acgcgccggc ccgcagttgg cctgcggagc gcggtg 116
<210> 31
   <211> 115
   <212> DNA
   <213> Homo sapiens
<220>
   <221> Transcript Start Site
   <222> (1)..(15)
<400> 31
   gctcctgctg gaggtgaaag tctggcctgg cagccttccc caggtgagca gcaacaaggt      60
   gagagaatgc agagctgcag gtaagaggct ttggagagag agagagaaag agaga          115
<210> 32
   <211> 133
   <212> DNA
   <213> Homo sapiens
<220>
   <221> Transcript Start Site
   <222> (1)..(33)
<400> 32
   ggaaaggctg tttactccct cctgtctagt cggcttggtc cctttagggc tccggatatc      60
   tttggtgact tgtccactcc agtgtggcat catgtggcag ctgctcctcc caactgctct     120
   gctacttcta ggt                                                        133
<210> 33
   <211> 104
   <212> DNA
   <213> Homo sapiens
<220>
   <221> Transcript Start Site
   <222> (1)..(4)
<400> 33
   cagatccgag ccgggctggc tgcagagaaa ccgcagggag agcctcactg ctgagcgccc      60
   ctcgacggcg gagcggcagc agcctccgtg gcctccagca tccg                      104
<210> 34
   <211> 105
   <212> DNA
   <213> Homo sapiens
<220>
   <221> Transcript Start Site
   <222> (1)..(5)
<400> 34
   agtaggttca gcggacgcgc gcggcctcga tctctggact cgtcacctgc ccctccccct 60
   cccgccgccg tcacccagga aaccggccgc aatcgccggc cgacc 105
<210> 35
   <211> 117
   <212> DNA
   <213> Homo sapiens
<220>
   <221> Transcript Start Site
   <222> (1)..(17)
<400> 35
   ggcagccaga cactctccaa aaagcagaga cagcaggaag aggggagtgg aggcagccca 60
   ttcacctggg gaaatgactg ggttgtcgat ggacggtggt gagtgtgctg gtgtgcg 117
<210> 36
   <211> 116
   <212> DNA
   <213> Homo sapiens
<220>
   <221> Transcript Start Site
   <222> (1)..(16)
<400> 36
   agcgagcatc ggccaccgcc atcccatcca gcgagcatct gccgccgcgc cgccgccacc 60
   ctcccagaga gcactggcca ccgctccacc atcacttgcc cagagtttgg gccacc 116
<210> 37
   <211> 115
   <212> DNA
   <213> Homo sapiens
<220>
   <221> Transcript Start Site
   <222> (1)..(15)
<400> 37
   aaagattaag ctgcaggctc cctgcccata aaacagggtg tgaaaggcat ctcagcggct 60
   gccccaccat ggctacctgg gccctcctgc tccttgcagc catgctcctg ggcaa 115
<210> 38
   <211> 114
   <212> DNA
   <213> Homo sapiens
<220>
   <221> Transcript Start Site
   <222> (1)..(14)
<400> 38
   ggctttggac tcttctcctc ctccacctcc tcctcctcct cccgcgccgc cgcctcctcc       60
   tcctcttcct ctccgcgcct tcgctacgcg cccggccgcc cgaggcagat ccag             114
<210> 39
   <211> 135
   <212> DNA
   <213> Homo sapiens
<220>
   <221> Transcript Start Site
   <222> (1)..(35)
<400> 39
   tatctgtcat cctgctaatg tgcattgaaa ggagagcaac ggggctgagg ccgtgtcagc       60
   acgatggacc ccaaacagac caccctcctg tgtcttggtg agtttcagag taaaagtggg      120
   ttagagggga agata                                                        135
<210> 40
   <211> 116
   <212> DNA
   <213> Homo sapiens
<220>
   <221> Transcript Start Site
   <222> (1)..(16)
<400> 40
   agagccggtg ttgctccaca ggaagccaag aagcacacag gaaaaggagc ttagctgctg       60
   gtaagttggg gactgacaat ttaaaattta tcaatggaac tcctgctctc tgacaa          116
<210> 41
   <211> 185
   <212> DNA
   <213> Homo sapiens
<220>
   <221> Transcript Start Site
   <222> (1)..(85)
<400> **41**
   tttttgtcca agaaaacttt tcgtgtttgc ctgtagctga tgtgcagttg tttctgctcc 60
   caaatcaaca gggtttaatg aaaaccctag aaagtagaaa cccgtgggag agaaaagaaa 120
   aaagacaagt tcacaaggta tgtttagtgt ataaaaaagg agaaggacta gaaagcctga 180
   gatac 185
<210> 42
   <211> 167
   <212> DNA
   <213> Homo sapiens
<220>
   <221> Transcript Start Site
   <222> (1)..(67)
<400> 42
   agtccaagag agagcagctc cattccaggc gtttgggagg atgtgggtcc gctggtgctt 60
   tcaggtgaag gtgttgaggt cagcgcttcc caggctggcg tgtggaggct cccagctggg 120
   agcagagctg ggagggggct ctggggggta taggggttct ggggtcc 167
<210> 43
   <211> 115
   <212> DNA
   <213> Homo sapiens
<220>
   <221> Transcript Start Site
   <222> (1)..(15)
<400> 43
   cacagctggg acagtcctgg cccactgcac tgggaatcta ggatgggggc cttggccaga 60
   gccctgccgt ccatactgct ggcattgctg cttacgtcca ccccagaggc tctgg 115
<210> 44
   <211> 119
   <212> DNA
   <213> Homo sapiens
<220>
   <221> Transcript Start Site
   <222> (1)..(19)
<400> 44
   agctgggata ttggagcagc aagaggctgg gaagccatca cttaccttgc actgagaaag 60
   aagacaaagg ccagtatgca cagctttcct ccactgctgc tgctgctgtt ctggggtgt 119
<210> 45
   <211> 116
   <212> DNA
   <213> Homo sapiens
<220>
   <221> Transcript Start Site
   <222> (1)..(16)
<400> 45
   ccaggccact gggctccgcg gagccagcga gaggtctgcg cggagtctga gcggcgctcg        60
   tcccgtccca aggccgacgc cagcacgccg tcatggcccc cgcagcggcg acgggg            116
<210> 46
   <211> 105
   <212> DNA
   <213> Homo sapiens
<220>
   <221> Transcript Start Site
   <222> (1)..(5)
<400> 46
   cggcagccct gggcggtccc gggaatcgca caggacccgg ctcccctcgc ctaagctgct        60
   ggggggcgcc atgggccgct accgcatccg cgtggccacc ggggc                        105
<210> 47
   <211> 119
   <212> DNA
   <213> Homo sapiens
<220>
   <221> Transcript Start Site
   <222> (1)..(19)
<400> 47
   gccccacgct ttgaccgggt cgtggcagcc ggagtcgtct tcgggacgcg cctgctcttc        60
   gcctttcgct gcagtccgtc ggtgagtgcg cgcccagtgg gtgccccagc gaaccggtc         119
<210> 48
   <211> 120
   <212> DNA
   <213> Homo sapiens
<220>
   <221> Transcript Start Site
   <222> (1)..(20)
<400> 48
   gtttgttaag atggcagagc ccggtaatcg cataaaactt aaaactttac agtcagaggt 60
   tcaattcctc ttcttaacaa catacccatg gccaacctcc tactcctcat tgtacccatt 120
<210> 49
   <211> 105
   <212> DNA
   <213> Homo sapiens
<220>
   <221> Transcript Start Site
   <222> (1)..(5)
<400> 49
   tggcgactcg ctggggttcc cttaccgagc tgtgggagct ttgttctttc gctttttgca 60
   ataaatcttg ctgctgctca ctctttgggt ccacactgcc tttat 105
<210> 50
   <211> 136
   <212> DNA
   <213> Homo sapiens
<220>
   <221> Transcript Start Site
   <222> (1)..(36)
<400> 50
   tggaagtgga ggaaatctac aagcaccagg aagtcaagat gcaagcacca gccttcaggg 60
   acaagaaaca gggggtctca gccaagaatc aaggttaggg aactcgaggt ggaagggagg 120
   ggttaagaag gagaga 136
<210> 51
   <211> 171
   <212> DNA
   <213> Homo sapiens
<220>
   <221> Transcript Start Site
   <222> (1)..(71)
<400> 51
   gggccagaag agctgtgaga cttagaaact tccccccagt agccccactt ggctcttccc 60
   tccttcactg ctgaggagct gctcattggt ccgaggcagc tgggactctg ggcagagcct 120
   tcttccggac agctggatgg agggtccccc tcttagcaga gctgctctaa c 171
<210> 52
   <211> 121
   <212> DNA
   <213> Homo sapiens
<220>
   <221> Transcript Start Site
   <222> (1)..(21)
<400> 52
   gcctcattgt gcaaacacac ctgcttcaag gtcccaggca cagaaggctc agatccgtgc      60
   actgccttca gctctgggtt gaacttacag ctggactaga ggatgggaag gcatcaaaag     120
   g                                                                     121
<210> 53
   <211> 116
   <212> DNA
   <213> Homo sapiens
<220>
   <221> Transcript Start Site
   <222> (1)..(16)
<400> 53
   tttgcagatg gattcgagac ggaagcgggc tgggaggcgt cggcggcggc agcgcacgtg      60
   gtgacgtgcg agggggtgcg gcgcgagcgg tcggcggcgg cggaggcagt gtctcc         116
<210> 54
   <211> 145
   <212> DNA
   <213> Homo sapiens
<220>
   <221> Transcript Start Site
   <222> (1)..(45)
<400> 54
   gcatcctcat catgtagcgc cagccacacc aggaacagaa gggtgccggg taccttccgc      60
   atgcttggta ttctccccgc ggggctctga ccgctgccgc tctcaggcac ctgtctttcc     120
   tctccgtccc agaatggagc caaga                                           145
<210> 55
   <211> 116
   <212> DNA
   <213> Homo sapiens
<220>
   <221> Transcript Start Site
   <222> (1)..(16)
<400> 55
   tcttcctcag aggaagtttc ttggtagatc accgacacct catccaggcg gggggttggg 60
   gggaaacttg gcaccagcca tcccaggcag agcaccactg tgatttgttc tcctgg 116
<210> 56
   <211> 131
   <212> DNA
   <213> Homo sapiens
<220>
   <221> Transcript Start Site
   <222> (1)..(31)
<400> 56
   aaggcacaca atgccagaca ctatggcctt caggcctccc tgccacgggg atgctgcctt 60
   ttctgctccg ggtgtttcca cgaggcaggc atggaatctt ccctggacaa gcgacatacc 120
   gtggagagac a 131
<210> 57
   <211> 106
   <212> DNA
   <213> Homo sapiens
<220>
   <221> Transcript Start Site
   <222> (1)..(6)
<400> 57
   tggtaaccag ctgcagtact ttttgtgtgg tgtggaagtg tttgtttttt gcaatagatt 60
   tttgttgcag tttaggtttg tattttcttt gttttgtagt gcttat 106
<210> 58
   <211> 116
   <212> DNA
   <213> Homo sapiens
<220>
   <221> Transcript Start Site
   <222> (1)..(16)
<400> 58
   gcgctcagtc ctgtgtccgg gccccgaggc acagccaggg caccaggtgg agcaccagct 60
   acgcgtggcg cagcgcagcg tccctagcac cgagcctccc gcagccgccg agatgc 116
<210> 59
   <211> 116
   <212> DNA
   <213> Homo sapiens
<220>
   <221> Transcript Start Site
   <222> (1)..(16)
<400> 59
   aggacccggg ctgtcccctc ttcccgggcc acgtcagcca ggcccgcgtg atttggagag 60
   gaagagagga ccagcttggt tcctcagaac tgctgcttcc tcgctttccg gcttac 116
<210> 60
   <211> 118
   <212> DNA
   <213> Homo sapiens
<220>
   <221> Transcript Start Site
   <222> (1)..(18)
<400> 60
   ctgctgctgc tactgccgcc gccgccgctg ttgccgctgg tgccgctgcc gccgccgctg 60
   ctcatgatca ttattttacc ttttaattct ttttttttcc gctcttgcca aatgcttt 118
<210> 61
   <211> 116
   <212> DNA
   <213> Homo sapiens
<220>
   <221> Transcript Start Site
   <222> (1)..(16)
<400> 61
   tcgcagcaca gtgcggagac cgcagccccg gagcccgggc cagggtccac ctgtccccgc 60
   agcgccggct cgcgccctcc tgccgcagcc accggtgagt gccgcggtcc tgagat 116
<210> 62
   <211> 168
   <212> DNA
   <213> Homo sapiens
<220>
   <221> Transcript Start Site
   <222> (1)..(68)
<400> 62
   ctccgccccg cccgccggag gagcccaggc tgggcagatg ctgcggctcg ccgcgcggct 60
   ccagctgggg cgcggtggtc ccgacggtca cccgaatcgc agggagtcgc cgctggagag 120
   gggctcggcg cccggcgggg actgcaggtg tgtgactggg cggccagg 168
<210> 63
   <211> 147
   <212> DNA
   <213> Homo sapiens
<220>
   <221> Transcript Start Site
   <222> (1)..(47)
<400> 63
   tctcgttttt ctcttccttt ctctttctct ctcaccctct ctgtgcaact ctcttggtca 60
   ttctcctctc tctgtgcaac tctttcctat ccagtatttt gctctgcaaa ttccatccac 120
   ctttgcctct gaattctcaa ctccgtc 147
<210> 64
   <211> 119
   <212> DNA
   <213> Homo sapiens
<220>
   <221> Transcript Start Site
   <222> (1)..(19)
<400> 64
   agcttaaggg caaaggctgg aagcagagcg aactgggagc agagcacaca ggtgcgttct 60
   gatgggtatt cgggagagtc aagaaggggc tgacctggag gacagaaaag cagtggaag 119
<210> 65
   <211> 122
   <212> DNA
   <213> Homo sapiens
<220>
   <221> Transcript Start Site
   <222> (1)..(22)
<400> 65
   ggacgcggac acccacacag gaacggaccc ggggagaaca acacatcgtc tctgcgccag 60
   ggagagaggc gtccggggaa cccagccgtg cccacgcatt tatctccgcc ttccggctca 120
   ga 122
<210> 66
   <211> 124
   <212> DNA
   <213> Homo sapiens
<220>
   <221> Transcript Start Site
   <222> (1)..(24)
<400> 66
   cccgcagtgt ccgacccagt cgtcccgcgc cggagccggc cccgtagcgt gccatggcct 60
   gctacatcta ccagctgccc tcctgggtgc tggacgacct gtgccgcaac atggacgcgc 120
   tcag 124
<210> 67
   <211> 161
   <212> DNA
   <213> Homo sapiens
<220>
   <221> Transcript Start Site
   <222> (1)..(61)
<400> 67
   cccgggacac cccctcccgg tcccctggcg gggcagcgtc ggctctggca gcactggagg 60
   cggcggcggc ccgagggcga cttgcggggc gcgcaggccg ccgtgcaccc gggacgcttc 120
   cccctcgggg accctccgcg ggcttctccg ccgcgccgtc c 161
<210> 68
   <211> 115
   <212> DNA
   <213> Homo sapiens
<220>
   <221> Transcript Start Site
   <222> (1)..(15)
<400> 68
   ggagaggggt tggacccagg gctgaggcag gcccccccct ccctcccgcc tcagtggatc 60
   atgcccaggg cggcagcggc ggcggttgcg ggggggaagt gactgggcgg tgccg 115
<210> 69
   <211> 136
   <212> DNA
   <213> Homo sapiens
<220>
   <221> Transcript Start Site
   <222> (1)..(36)
<400> 69
   gcagcacccc agttctcccc gcacgccggc actcgcggct gctggagccc cggctggctc 60
   accccggggc cgggcagaat tgggctccag gtaagcgaca gcgtcgggtg gggactgggc 120
   aggtcaagca gtgccc 136
<210> 70
   <211> 118
   <212> DNA
   <213> Homo sapiens
<220>
   <221> Transcript Start Site
   <222> (1)..(18)
<400> 70
   tttgcagcag atgtgtgacc tggcgttccc ctgagtgctc ggaaaatggc cttgtctccg    60
   gtcgttcccg ttcaggagca ggtacctggg gtgctccggg gatcccgttg tctaggat     118
<210> 71
   <211> 122
   <212> DNA
   <213> Homo sapiens
<220>
   <221> Transcript Start Site
   <222> (1)..(22)
<400> 71
   tgctttcaga cgcttcagag atcctctgga ggcctggggg agcttttgag tactttattt    60
   cagttggtcc ctgagctcgg tgagtggggc gggtagagcc accaggggaa tcaacagtgg   120
   tt                                                                  122
<210> 72
   <211> 134
   <212> DNA
   <213> Homo sapiens
<220>
   <221> Transcript Start Site
   <222> (1)..(34)
<400> 72
   ctgagatgcc agagcctctc ttaacactgt cctgtagctc agcaactcca aattagcaca    60
   gctccacaca tccaaagcct ggaaagggtg gaagatgaat atgaacggac agatccctca   120
   tgctcaccct tggt                                                     134
<210> 73
   <211> 118
   <212> DNA
   <213> Homo sapiens
<220>
   <221> Transcript Start Site
   <222> (1)..(18)
<400> 73
   accgccgtcc cgccatggct ctgaggcgcg ccctgcccgc gctgcgcccc tgcattcccc        60
   gcttcgtccc gctgtccacg gcgccggcct cccgcgagca gcccgcagcg ggcccagc         118
<210> 74
   <211> 155
   <212> DNA
   <213> Homo sapiens
<220>
   <221> Transcript Start Site
   <222> (1)..(55)
<400> 74
   gtgaggggtg gctttggagt tgcagttgat gtgtgatagt tgaaggttga ttgctgtact        60
   tgcttgtaag catggggggg gggggtttga tgtgggttgg gtttttatgt actacaggtg       120
   gtcaagtatt tatggtaccg tacaatattc atggt                                  155
<210> 75
   <211> 107
   <212> DNA
   <213> Homo sapiens
<220>
   <221> Transcript Start Site
   <222> (1)..(7)
<400> 75
   tggcaaccac ctggggttct tttcacactg tggaaggttt gttctttagc tctttgcaat        60
   aaatcttgct actgctcact ctttgggtct acgctgcttt tatgagc                     107
<210> 76
   <211> 113
   <212> DNA
   <213> Homo sapiens
<220>
   <221> Transcript Start Site
   <222> (1)..(13)
<400> 76
   ctgtcgctca taacgttctt gtttcttgca gtttccaaca tggctagatc catcagaaac        60
   tgaagccgtg gagaacgctc tcggggcctt tgccacttct tggagtagaa gcc              113
<210> 77
   <211> 284
   <212> DNA
   <213> Homo sapiens
<220>
   <221> Transcript Start Site
   <222> (1)..(184)
<400> 77
   atgggggctt cgacatgggc tttagggagt cataagtgga gtccgtaaag aggtatcttt 60
   actataaaag ctattgtgta agctagtcat attaagttgt tggctcagga gtttgatagt 120
   tcttgggcag tgagagtgag tagtagaatg tttagtgagc ctagggtgtt gtgagtgtaa 180
   attagtgcga tgagtagggg aagggagcct actagggtgt agaataggaa gtatgtgcct 240
   gcgttcaggc gttctggctg gttgcctcat cgggtgatga tagc 284
<210> 78
   <211> 177
   <212> DNA
   <213> Homo sapiens
<220>
   <221> Transcript Start Site
   <222> (1)..(77)
<400> 78
   atcagaatat gtagtctaga aaccattctc ataagcccat gtgcatgctg aggaggcctc 60
   ccgtcatttt aggctgttgt gtatcagtta gcagagctac tttgtttccc gcttggagga 120
   cctggagaag gggagctcag ccacgtgctg cacgccggag gctggagcct tgcagca 177
<210> 79
   <211> 116
   <212> DNA
   <213> Homo sapiens
<220>
   <221> Transcript Start Site
   <222> (1)..(16)
<400> 79
   gtggtaaagg aaactagctg agtcgttttc tgagaagaga ccatatttgt tcgcagagga 60
   agccgttgct ttctgggatc tggctacgcc agaaaagaca tcggctccaa cagggg 116
<210> 80
   <211> 116
   <212> DNA
   <213> Homo sapiens
<220>
   <221> Transcript Start Site
   <222> (1)..(16)
<400> 80
   ctggagcaga aagccgcgca cctcctcccg ccaggcgctt tctcggacgc cttgcccagc 60
   gggccgcccg accccctgca ccatggaccc cgctcgcccc ctggggctgt cgattc 116
<210> 81
   <211> 145
   <212> DNA
   <213> Homo sapiens
<220>
   <221> Transcript Start Site
   <222> (1)..(45)
<400> 81
   ctctctgccc acttgcttcc tcagtacctt ggtccagctc ttcctgcaac ggcccaggag 60
   ctcagagctc cacatctgac cttctagtca tgaccaggac cagggcagca ctcctcctgt 120
   tcacaggtga gcctggaccc caatg 145
<210> 82
   <211> 115
   <212> DNA
   <213> Homo sapiens
<220>
   <221> Transcript Start Site
   <222> (1)..(15)
<400> 82
   cactcgccag cctgtgcttt gccacctgag cgccgctccc tctcaggatg aaggtgacgg 60
   tggtgagtac ccagacccca tgcagagagg ctctgctgac tgctctcctg gtggc 115
<210> 83
   <211> 160
   <212> DNA
   <213> Homo sapiens
<220>
   <221> Transcript Start Site
   <222> (1)..(60)
<400> 83
   gagctggttt cctctgccct gttagagctg ggggactctt cagagtcaaa ggccagagag 60
   catggagctg agtggagcca ccatggcccg ggggctcgct gtcctgctag tcttgttcct 120
   gcatatcaag aacctgcctg cccaggctgc ggacacatgt 160
<210> 84
   <211> 122
   <212> DNA
   <213> Homo sapiens
<220>
   <221> Transcript Start Site
   <222> (1)..(22)
<400> 84
   gagagaagct ggactgcagc tggtttcagg aacttctctt gacgagaaga gagaccaagg 60
   aggccaagca ggggctgggc cagaggtgcc aacgtgagta ggggatccct agagaggggc 120
   tg 122
<210> 85
   <211> 157
   <212> DNA
   <213> Homo sapiens
<220>
   <221> Transcript Start Site
   <222> (1)..(57)
<400> 85
   ggaagtagca ttaagctgag accagaaggc tgagtggtgg tgacctacaa gaagaagaag 60
   ggaaggtcat aacagtaatg acttggagtg gagaggccct gcaggttgga gaagttcagt 120
   gtggctcacc atggagtgtc agagtgaggg agatgac 157
<210> 86
   <211> 184
   <212> DNA
   <213> Homo sapiens
<220>
   <221> Transcript Start Site
   <222> (1)..(84)
<400> 86
   ggcgtcgggc ggggcggccc agcgggtaaa agctgcgcgg ccgcaagctc ggcactcacg 60
   gctctgaggg ctccgacggc actgacggcc atggcgcgtt cgaacctccc gctggcgctg 120
   ggcctggccc tggtcgcatt ctgcctcctg gcgctgccac gcgacgcccg ggcccggccg 180
   cagg 184
<210> 87
   <211> 129
   <212> DNA
   <213> Homo sapiens
<220>
   <221> Transcript Start Site
   <222> (1)..(29)
<400> 87
   tggttaaaaa atagtagggg gatgatgcta ataattaggc tgtgggtggt tgtgttgatt 60
   caaattatgt gctttttgga aagtcatgtc agtggtagta atataattgt tgggacgatt 120
   agttttagc 129
<210> 88
   <211> 207
   <212> DNA
   <213> Homo sapiens
<220>
   <221> Transcript Start Site
   <222> (1)..(107)
<400> 88
   gggggtcatc catggggacg agaagggatt tgactgtaat gtgctatgta cggtaaatga 60
   ctttatgtac tatgtactgt taagggtggg taggtttgtt ggtatcctag tgggtgaggg 120
   gtggctttgg agttgcagtt gatgtgtgat agttgaaggt tgattgctgt acttgcttgt 180
   aagcatgggg ggggggggtt tgatgtg 207
<210> 89
   <211> 121
   <212> DNA
   <213> Homo sapiens
<220>
   <221> Transcript Start Site
   <222> (1)..(21)
<400> 89
   tttgtttata cattcagcag ctgatgggca tttgggttgc atccactttt tggctgttct 60
   gaagagtgtt actgtgaaca ttcatgcaca aatttttatg tggaggtatg tttttatttc 120
   t 121
<210> 90
   <211> 135
   <212> DNA
   <213> Homo sapiens
<220>
   <221> Transcript Start Site
   <222> (1)..(35)
<400> 90
   ctgtcagagg ctgagccatt aacgacagag ctcggggaga gaagctggac tgcagctggt 60
   ttcaggaact tctcttgacg agaagagaga ccaaggaggc caagcagggg ctgggccaga 120
   ggtgccaacg tgagt 135
<210> 91
   <211> 146
   <212> DNA
   <213> Homo sapiens
<220>
   <221> Transcript Start Site
   <222> (1)..(46)
<400> 91
   ggaggggggt tgttaggggg tcggaggaaa aggttgggga acagctaaat aggttgttgt 60
   tgatttggtt aaaaaatagt agggggatga tgctaataat taggctgtgg gtggttgtgt 120
   tgattcaaat tatgtgcttt ttggaa 146
<210> 92
   <211> 130
   <212> DNA
   <213> Homo sapiens
<220>
   <221> Transcript Start Site
   <222> (1)..(30)
<400> 92
   gagctgccaa ctgttgcgct ccaccacgcc ctccacgtgt tccactgcct cttctcttct 60
   cgcttgggaa ctccagtctc acctcggctt gcaatggacc ccaactgctc ctgcgaggct 120
   ggtaaggaac 130
<210> 93
   <211> 166
   <212> DNA
   <213> Homo sapiens
<220>
   <221> Transcript Start Site
   <222> (1)..(66)
<400> 93
   cttagtcatt tccccgctcc agcctgctcc cgcccgagcg cgcactcacg gccgctctcc 60
   ctcctcgctc cgcagccgcg gcccatggag cccgccggcc cggcccccgg ccgcctcggg 120
   ccgctgctct gcctgctgct cgccgcgtcc tgcgcctggt caggta 166
<210> 94
   <211> 126
   <212> DNA
   <213> Homo sapiens
<220>
   <221> Transcript Start Site
   <222> (1)..(26)
<400> 94
   tatgatgaag atgctcaggt agtggaagac gaggaggacg aggatgagga ggaggaaggt 60
   gaagaggagg acgtgagtgg agaggaggag gtaaggaggc tgggctgggc agggccaccg 120
   tctgcc 126
<210> 95
   <211> 108
   <212> DNA
   <213> Homo sapiens
<220>
   <221> Transcript Start Site
   <222> (1)..(8)
<400> 95
   gtgcatgctt tcagtttcag cttacacaag aagggaggag agaaaagcca tggccggtga 60
   gtcttttact gtcccaaaaa caggcccctg ggaggaaaaa gttgtgga 108
<210> 96
   <211> 122
   <212> DNA
   <213> Homo sapiens
<220>
   <221> Transcript Start Site
   <222> (1)..(22)
<400> 96
   ggcccaggag ctcagagctc cacatctgac cttctagtca tgaccaggac cagggcagca 60
   ctcctcctgt tcacaggtga gcctggaccc caatgaagta gggctgggga cccaggccca 120
   ag 122
<210> 97
   <211> 143
   <212> DNA
   <213> Homo sapiens
<220>
   <221> Transcript Start Site
   <222> (1)..(43)
<400> 97
   gcgcgggtaa aggggccgcc tcgagcgcgg tccgagcgtt cagcggacgc gcgcggcctc 60
   gatctctgga ctcgtcacct gcccctcccc ctcccgccgc cgtcacccag gaaaccggcc 120
   gcaatcgccg gccgacctga agc 143
<210> 98
   <211> 122
   <212> DNA
   <213> Homo sapiens
<220>
   <221> Transcript Start Site
   <222> (1)..(22)
<400> 98
   ggcaggccag ctcagcagag cctggggcca gagggccaga cagccacaga gctcctggcg 60
   tgggcaaggc tggccaagga tggcgacgcc caggggcctg ggggccctgc tcctgctcct 120
   cc 122
<210> 99
   <211> 157
   <212> DNA
   <213> Homo sapiens
<220>
   <221> Transcript Start Site
   <222> (1)..(57)
<400> 99
   tttacgttgt tagatatggg gagtagtgtg attgaggtgg agtagattag gcgtaggtag 60
   aagtagaggt taaggagggt gatggtggct atgatggtgg ggatgatgag gctattgttt 120
   tttgtgaatt cttcgataat ggcccatttg ggcaaaa 157
<210> 100
   <211> 116
   <212> DNA
   <213> Homo sapiens
<220>
   <221> Transcript Start Site
   <222> (1)..(16)
<400> 100
   agacccagag atgctgtaat ggtaagactt tggatccttc ctgaggacgt ggagaaaact 60
   tgctgctgag aaggacattt tgaaggtttt gttggctgaa aaagctgttt ctggaa 116
<210> 101
   <211> 159
   <212> DNA
   <213> Homo sapiens
<220>
   <221> Transcript Start Site
   <222> (1)..(59)
<400> 101
   ttcctatcct aacctactgg ggccctttat accaggtgcc aaacttcatt atccacccca 60
   ccaccttgcc cgggagggag atctgtgaaa gcgaaacgtg atttaagctg ggatctggca 120
   gcaacttcgt ggactctgaa gggcacgtgg gaaaggagt 159
<210> 102
   <211> 112
   <212> DNA
   <213> Homo sapiens
<220>
   <221> Transcript Start Site
   <222> (1)..(12)
<400> 102
   gtgcaggggc gtatctgggc gcctgagcgc ggcgtgggag ccttgggagc cgccgcagca 60
   gggggcacac ccggaaccgg cctgagcgcc cgggaccatg aacggggagg cc 112
<210> 103
   <211> 113
   <212> DNA
   <213> Homo sapiens
<220>
   <221> Transcript Start Site
   <222> (1)..(13)
<400> 103
   ggggaaatgg aaggttggct ctaggaccag atgcatcagg cttcaggtag gctcctgctt 60
   atttggtgaa ctcccttttc cttcctcctt agcctgagat ttaatttctg cat 113
<210> 104
   <211> 170
   <212> DNA
   <213> Homo sapiens
<220>
   <221> Transcript Start Site
   <222> (1)..(70)
<400> 104
   agggtgcagt gtttgttgat ggggaccctg gctttggctg aggctgacta acctctccac 60
   cttcaggacc tcttcaacga cctgattgct tgtgcgttcc ttgtgggagc cgtggtcttt 120
   gctgtgagaa gtcggcgatc catgaatctc cactacttac ttgctgtggt 170
<210> 105
   <211> 167
   <212> DNA
   <213> Homo sapiens
<220>
   <221> Transcript Start Site
   <222> (1)..(67)
<400> 105
   gactgggagg ggctgcgaga ggcgcgaatg cgaacctggc ccgtgcggaa agggcgcgga 60
   gagccccggc gcggagcagg cgggtagggc gaagggtccc ctttcgggcg ccatggggcg 120
   ccgagcgcgg cctggcccct cgggctcctc tgcggggagg gcaggcc 167
<210> 106
   <211> 139
   <212> DNA
   <213> Homo sapiens
<220>
   <221> Transcript Start Site
   <222> (1)..(39)
<400> 106
   gcaactttgt ctaatgtgat cattaacctt cctgcaaaac acagctggca gttctctgag 60
   gtttgtcact agaatgtgaa gacagccaca cagatattgc acagactatt tacagatcgt 120
   ttggtttaca ttgagagtc 139
<210> 107
   <211> 121
   <212> DNA
   <213> Homo sapiens
<220>
   <221> Transcript Start Site
   <222> (1)..(21)
<400> 107
   tgcgcgggac tcggccttct gggcgcgcgc gacgtcagtt tgagttctgt gttctccccg 60
   cccgtgtccc gcccgacccg cgcccgcgat gctggcgctg cgctgcggct cccgctggct 120
   c 121
<210> 108
   <211> 155
   <212> DNA
   <213> Homo sapiens
<220>
   <221> Transcript Start Site
   <222> (1)..(55)
<400> 108
   ctcctcctcc tttctcttgc taatcccgcc tcctcctaag acttaggaag actggtggat 60
   gcgtttgggt tgtagctagg ctttttcttt tctttctctt ttaaaacaca tctagacaag 120
   gaaaaaacaa gcctcggatc tgatttttca ctcct 155
<210> 109
   <211> 115
   <212> DNA
   <213> Homo sapiens
<220>
   <221> Transcript Start Site
   <222> (1)..(15)
<400> 109
   cctaggtgtg tctgcctgcc agacacccaa tcttgcaaga ccgcctttga aagtctcgca 60
   tctgctgttt ttcaggtctc caagtccatt ctttgtgttt ggactggtga gtgtt 115
<210> 110
   <211> 106
   <212> DNA
   <213> Homo sapiens
<220>
   <221> Transcript Start Site
   <222> (1)..(6)
<400> 110
   cagcactttt ccttgctgcc gccatgagaa gaagtatgtg ttttcttccc cttctgacat 60
   gattgttaag tttcctgagg cctccccagc catgctgaac tgtgag 106
<210> 111
   <211> 111
   <212> DNA
   <213> Homo sapiens
<220>
   <221> Transcript Start Site
   <222> (1)..(11)
<400> 111
   cggacagacg gactgacgga ctctcgagcg gacagcgcag ctagcggggc gcgggcgctg 60
   ggcgtcgacg gccagcccca gccttccccg ccccgtcgcg ccccgccccg t 111
<210> 112
   <211> 122
   <212> DNA
   <213> Homo sapiens
<220>
   <221> Transcript Start Site
   <222> (1)..(22)
<400> 112
   tggaggccca gattgcgatc gtgacggaga accaggccct gcagcagcag cttcaccagg 60
   ttggtcgcag caagtgggat ttcccagggc gctgagggtg tggctggggg cccaggctca 120
   cc 122
<210> 113
   <211> 139
   <212> DNA
   <213> Homo sapiens
<220>
   <221> Transcript Start Site
   <222> (1)..(39)
<400> 113
   acgacagcga ctgaagcgca gctggagtgg taagatggcg gacgagcagc tgcagcagct 60
   ccgtgcggtg ctacaggggg aggggaaggt ggtttctcct gacacaagct ggaggccgga 120
   gcctgtggca ccctggctt 139
<210> 114
   <211> 126
   <212> DNA
   <213> Homo sapiens
<220>
   <221> Transcript Start Site
   <222> (1)..(26)
<400> 114
   ctggtgagtc ctctctcttg ctcttgcttg cttgcactga tgaagtaagc tgccacgttg 60
   tgagatgctc aacgaagagg cccatgaggc agggcaccga gggaggcttc tggccaacag 120
   ctcctg 126
<210> 115
   <211> 135
   <212> DNA
   <213> Homo sapiens
<220>
   <221> Transcript Start Site
   <222> (1)..(35)
<400> 115
   agtgtgggga ggaatggggt gggttttgta tgttcaaact gtcattttat ttttacgttg 60
   ttagatatgg ggagtagtgt gattgaggtg gagtagatta ggcgtaggta gaagtagagg 120
   ttaaggaggg tgatg 135
<210> 116
   <211> 143
   <212> DNA
   <213> Homo sapiens
<220>
   <221> Transcript Start Site
   <222> (1)..(43)
<400> 116
   gggagggggg ttcatagtag aagagcgatg gtgagagcta aggtcggggc ggtgatgtag 60
   agggtgatgg tagatgtggc gggttttagg ggctctttgg tgaagagttt tatggcgtca 120
   gcgaagggtt gtagtagccc gta 143
<210> 117
   <211> 178
   <212> DNA
   <213> Homo sapiens
<220>
   <221> Transcript Start Site
   <222> (1)..(78)
<400> 117
   tttggtttaa tccacctcaa ctgcctgcta tgatggataa gattgagaga gtgaggagaa        60
   ggcttacgtt tagtgaggga gagatttggt atatgattga gatgggggct agtttttgtc       120
   atgtgagaag aagcaggccg gatgtcagag gggtgccttg ggtaacctct gggactca         178
<210> 118
   <211> 114
   <212> DNA
   <213> Homo sapiens
<220>
   <221> Transcript Start Site
   <222> (1)..(14)
<400> 118
   gggtcagaga ggagatgagg gtgtgagagg gtgagcaggt agcgccctca ccgtgctggg        60
   cacaagacaa ggacttcaga gctgattatt ttggttttgg ggttttttgt ttgt             114
<210> 119
   <211> 107
   <212> DNA
   <213> Homo sapiens
<220>
   <221> Transcript Start Site
   <222> (1)..(7)
<400> 119
   tggcaactgg ctcaggttct cttccacgct cacgctatgg gagttttgtt tttttgatct        60
   ttgagataac ttgctgctgc tcattctttg ggtacacact gagttta                     107
<210> 120
   <211> 105
   <212> DNA
   <213> Homo sapiens
<220>
   <221> Transcript Start Site
   <222> (1)..(5)
<400> 120
   tggcaatcct ctccggtccc cttccgggct gtgggagctt tgttcttttg ctctttgcaa 60
   taaatcttgc tgctgctcgc tctttgggtc cacattgcct ttgtg 105
<210> 121
   <211> 112
   <212> DNA
   <213> Homo sapiens
<220>
   <221> Transcript Start Site
   <222> (1)..(12)
<400> 121
   aaagcagtct ggagcccaga agggacacac cagcacagtc tggtaggcta cagcagcaag 60
   tctctaaaga aaggctgaga acacccagaa caggagagtt caggtccagg at 112
<210> 122
   <211> 154
   <212> DNA
   <213> Homo sapiens
<220>
   <221> Transcript Start Site
   <222> (1)..(54)
<400> 122
   aaaggaaatg aacatgtgtt ctgctttccc agggtccatg gcaggagggc tgcagcggcc 60
   tcatttattc atttgtgcca ggcaccctgt taatcatgga gataccacgc tgacctgcct 120
   tcaaggagac catattctag taggagagct gagc 154
<210> 123
   <211> 150
   <212> DNA
   <213> Homo sapiens
<220>
   <221> Transcript Start Site
   <222> (1)..(50)
<400> 123
   accacgcgct ttgcactctg gggcccacgc acttccctga agagtctaga agctgctcct 60
   catttccaga ctttccgggc gcccaccagg ctcccgggct ctcaccggag accacaggga 120
   ggagcttcaa ggtaccccaa gtccctgcgg 150
<210> 124
   <211> 145
   <212> DNA
   <213> Homo sapiens
<220>
   <221> Transcript Start Site
   <222> (1)..(45)
<400> 124
   tttcttgagc taggaaaggt ggttggctta cggcacagta gagagcttcc agggctggct        60
   ggcgtgggat acccgtacca cagaaatgca gggaccattg cttcttccag gtatgtgtga       120
   ctgtaacaat gactgaggct taacc                                             145
<210> 125
   <211> 115
   <212> DNA
   <213> Homo sapiens
<220>
   <221> Transcript Start Site
   <222> (1)..(15)
<400> 125
   gccggccgca gcctcgcagc gctctctccg cgcccgcgtc gctgactgac cgcccggccg        60
   gccggccgag ggagcagccc ccgcccggac ccgcagccgc ctcccgaccc cggtg            115
<210> 126
   <211> 159
   <212> DNA
   <213> Homo sapiens
<220>
   <221> Transcript Start Site
   <222> (1)..(59)
<400> 126
   gagtcagttc cccagatctc ttcatcactt ccccagatct ctgggatctt cagctgagat        60
   tgggccccag cacctggact caaggtaagg ccccttagga tgcttgtagc tgatcaatca       120
   gggtagtgca aaggaagagg ggaaaaagcc acccagacc                              159
<210> 127
   <211> 132
   <212> DNA
   <213> Homo sapiens
<220>
   <221> Transcript Start Site
   <222> (1)..(32)
<400> 127
   ttcaagattc attctccctg aatcttacca acaaaacact cctgaggaga aagaaagaga     60
   gggagggaga gaaaaagaga gagagagaaa caaaaaacca aagagagaga aaaaatgaat    120
   tcatctaaat ca                                                        132
<210> 128
   <211> 130
   <212> DNA
   <213> Homo sapiens
<220>
   <221> Transcript Start Site
   <222> (1)..(30)
<400> 128
   gggaaagttg tttactgaaa cttagaagca agggggtgaa gagaaccagg aagctaaact     60
   ttaaaatgga gaatcaaaga ataagagagc tgaacatact gacatactga ttctttgaag    120
   agaaacttgg                                                           130
<210> 129
   <211> 151
   <212> DNA
   <213> Homo sapiens
<220>
   <221> Transcript Start Site
   <222> (1)..(51)
<400> 129
   attgtgaaga tgataagtgt agagggaagg ttaatggttg atattgctag ggtggcgctt     60
   ccaattaggt gcatgagtag gtggcctgca gtaatgttag cggttaggcg tacggccagg    120
   gctattggtt gaatgagtag gctgatggtt t                                   151
<210> 130
   <211> 144
   <212> DNA
   <213> Homo sapiens
<220>
   <221> Transcript Start Site
   <222> (1)..(44)
<400> 130
   tggcaggatc cttactccta cctctaccca cagccagtgc ctttggcgca ctgaggtgca     60
   cagggtccct tagccgggcg cagggcgcgc agcccaggct gagatccgcg gcttccgtag    120
   aagtgagcat ggctgggcag cgag                                           144
<210> 131
   <211> 113
   <212> DNA
   <213> Homo sapiens
<220>
   <221> Transcript Start Site
   <222> (1)..(13)
<400> 131
   aacttcagtt tgttggctgc ggcagcaggt agcaaagtga cgccgagggc ctgagtgctc      60
   cagtagccac cgcatctgga gaaccagcgg ttaccatgga ggggatcagt gta            113
<210> 132
   <211> 120
   <212> DNA
   <213> Homo sapiens
<220>
   <221> Transcript Start Site
   <222> (1)..(20)
<400> 132
   tagggggttg tttgatcccg tttcgtgcaa gaataggagg tggagtgctg ctagggctgc      60
   aataatgaag ggtaagatga agtgaaaggt aaagaatcgt gtgagggtgg gactgtctac     120
<210> 133
   <211> 114
   <212> DNA
   <213> Homo sapiens
<220>
   <221> Transcript Start Site
   <222> (1)..(14)
<400> 133
   cggggcaggg agtgggctgc agtgacagcc ggcggcggag cggccggtcc acggaggagg      60
   tgagtgcgcc cgccgtgcgg agccgcgcct gctcccgcgg cggtgcccgg gccc           114
<210> 134
   <211> 117
   <212> DNA
   <213> Homo sapiens
<220>
   <221> Transcript Start Site
   <222> (1)..(17)
<400> 134
   ctcctcagag cagagcctga catctcccca ccacagacgt ttgaacagag caggctcctg 60
   aggtctccag gatgcctgtc ccagcctcct ggccccaccc tccttgtcct ttcctgc 117
<210> 135
   <211> 123
   <212> DNA
   <213> Homo sapiens
<220>
   <221> Transcript Start Site
   <222> (1)..(23)
<400> 135
   ggagctggca gcatttgatt gtggcttggg acgcgaggag aggcgcgcag cgaccgcctg 60
   acggcaggca atggtgtaag cgcctctcgg cctccccctc cccccagacg cggccgggtc 120
   ctc 123
<210> 136
   <211> 154
   <212> DNA
   <213> Homo sapiens
<220>
   <221> Transcript Start Site
   <222> (1)..(54)
<400> 136
   cgggctgccc tgagggagga agcgccagag cggcggccgg tcccgcgcgg agcccggcgc 60
   ccctccagcc cgagccagga cgccgccggc cccggtcccg gccccgggca cgcagcgagc 120
   cagggatgtg agcggcgccc cgcggcatgg cagc 154
<210> 137
   <211> 135
   <212> DNA
   <213> Homo sapiens
<220>
   <221> Transcript Start Site
   <222> (1)..(35)
<400> 137
   ctatcatttg ttttgaggtt agtttgatta gtcattgttg ggtggtgatt agtcggttgt 60
   tgatgagata tttggaggtg gggatcaata gagggggaaa tagaatgatc agtactgcgg 120
   cgggtaggcc tagga 135
<210> 138
   <211> 106
   <212> DNA
   <213> Homo sapiens
<220>
   <221> Transcript Start Site
   <222> (1)..(6)
<400> 138
   tggcaaccgg ctcaggtccc cttctattgt ggaagctttg ttctttctct ctttgcaata        60
   aatcctgctg ctgctcactc tttgggtcca cactgccttt atgagc                      106
<210> 139
   <211> 103
   <212> DNA
   <213> Homo sapiens
<220>
   <221> Transcript Start Site
   <222> (1)..(3)
<400> 139
   tgcctgcctg tgtgtgctgg aggtggcggt ggatctttta ttacccaaca ctccacttgc        60
   ctttttagcc tttcctctcc ctcttctctc ctggcctccc agt                         103
<210> 140
   <211> 117
   <212> DNA
   <213> Homo sapiens
<220>
   <221> Transcript Start Site
   <222> (1)..(17)
<400> 140
   ttccctcatt cgagtgctcc ggcgcacaga cccgcgcccc gccgtctgcg agcctcccga        60
   gagccgtccc ttcgtccggc cctggagcat tgcgtttgtc gccggtgtcg cagtgcg          117
<210> 141
   <211> 242
   <212> DNA
   <213> Homo sapiens
<220>
   <221> Transcript Start Site
   <222> (1)..(142)
<400> 141
   ttaacttcta ggccaggcac tactctgtcc caacaataag ccctgtacat tgggaaaggt 60
   gccgagacat gaactttggt cttctctgca atccatctgg agcattcact gacaacatcg 120
   actttgaagt tgcactgacc tggccagccc tgccacttac caggttgggt aagttattca 180
   tttctttatt aattcattca ttcattcaat ttatgggtat tttgtgtcat acacttgcta 240
   gg 242
<210> 142
   <211> 123
   <212> DNA
   <213> Homo sapiens
<220>
   <221> Transcript Start Site
   <222> (1)..(23)
<400> 142
   cggctgctgc cttcatctgg ggaaattcgt ggccactgca agtttactac gcgaggcgca 60
   gccaatgcca agcgccgagg ccgaggaggg ctaaacactg cggccgcggc tccgaacaat 120
   aac 123
<210> 143
   <211> 121
   <212> DNA
   <213> Homo sapiens
<220>
   <221> Transcript Start Site
   <222> (1)..(21)
<400> 143
   cctcaccatt ttttcctgca tggcatggac agaagcaggg gctaaagctc tgttcctctc 60
   tcctggaagc ttgcagacct cccttcagaa ccaatcccaa gaagccacct atccggaaca 120
   a 121
<210> 144
   <211> 130
   <212> DNA
   <213> Homo sapiens
<220>
   <221> Transcript Start Site
   <222> (1)..(30)
<400> 144
   aaggacagca gagcctaagt cacagtagcc ctgactacag cattcctgga gcccaggctc 60
   ttttccacag aggaggaaag agcaggcagc agagaccatg gggcccccct cagcctctcc 120
   ccacagagaa 130
<210> 145
   <211> 178
   <212> DNA
   <213> Homo sapiens
<220>
   <221> Transcript Start Site
   <222> (1)..(78)
<400> 145
   acatgagtat tttgcaatta gcaataacca gtgcacgggg tttcgcatag acgcctgccc 60
   attcggcccg gcagtgtgtg aggcgccttt caaaccggag ggattggcaa ggtaatgcca 120
   cttgagacaa gtttcttggc ctgtccgctc cttggtgtgg tggtggttgg gtggggga 178
<210> 146
   <211> 141
   <212> DNA
   <213> Homo sapiens
<220>
   <221> Transcript Start Site
   <222> (1)..(41)
<400> 146
   gggtcgaaga aggtggtgtt gaggttgcgg tctgttagta gtatagtgat gccagcagct 60
   aggactggga gagataggag aagtaggact gctgtgatta ggacggatca gacgaagagg 120
   ggcgtttggt attgggttat g 141
<210> 147
   <211> 117
   <212> DNA
   <213> Homo sapiens
<220>
   <221> Transcript Start Site
   <222> (1)..(17)
<400> 147
   agtggctctg gaacgaaagg gcctggttcg caaagaagct gacttcagag ggggaaactt 60
   tcttctttta ggaggcggtt agccctgttc cacgaaccca ggagaactgc tggccag 117
<210> 148
   <211> 104
   <212> DNA
   <213> Homo sapiens
<220>
   <221> Transcript Start Site
   <222> (1)..(4)
<400> 148
   attagctctt agtaagatta cacatgcaag catccccatt ccagtgagtt caccctctaa       60
   atcaccacga tgatttagag gtaaggacta aagggaaatc aaag                        104
<210> 149
   <211> 121
   <212> DNA
   <213> Homo sapiens
<220>
   <221> Transcript Start Site
   <222> (1)..(21)
<400> 149
   agcacgctcc gactgtgtga gctagggggc ccggcgcggg tgccatcgca gcggggcgcg       60
   tgggaaggcc agtgtgcacg cgaaggtccc actccaggcg agcgcagggg aggacccgga      120
   g                                                                       121
<210> 150
   <211> 110
   <212> DNA
   <213> Homo sapiens
<220>
   <221> Transcript Start Site
   <222> (1)..(10)
<400> 150
   attcaatcta tgaggtggag ggccagttag caggactcca tgtccctatg ctgagttctt       60
   tttctttttc cttttcaccc agtaaattct gctcctaacc cttctatgta                 110
<210> 151
   <211> 117
   <212> DNA
   <213> Homo sapiens
<220>
   <221> Transcript Start Site
   <222> (1)..(17)
<400> 151
   atgtttcagt cacatttcag ccactgctct gagaatttgt gagcagcccc taacaggctg 60
   ttacttcact acaactgacg atatgatcat cttaatttac ttatttctct tgctatg 117
<210> 152
   <211> 141
   <212> DNA
   <213> Homo sapiens
<220>
   <221> Transcript Start Site
   <222> (1)..(41)
<400> 152
   ttcctccccc tccctctccc tcacccctcg gacaggagcg caccgcctgc cccctgttcc 60
   tcgggaaggg aggaatttag cggaaaaaaa agtttcccag aagtttggat cacagggagg 120
   acgggaaggc agagaggaag a 141
<210> 153
   <211> 147
   <212> DNA
   <213> Homo sapiens
<220>
   <221> Transcript Start Site
   <222> (1)..(47)
<400> 153
   atgccgtgtc attgggagac tccgcagccg gagcattaga ttacagctcg acggagctcg 60
   ggaagggcgg cgggggtgga agatgagcag aagcccctgt tctcggaacg ccggctgaca 120
   agcggggtga gcgcagccgg ggcgggg 147
<210> 154
   <211> 155
   <212> DNA
   <213> Homo sapiens
<220>
   <221> Transcript Start Site
   <222> (1)..(55)
<400> 154
   gagattgaga ctagtagggc taggcccacc gctgcttcgc aggcggcaaa gactagtatg 60
   gcaataggca caatattggc taagagggag tgggtgttga gggttatgag agtagctata 120
   atgaacagcg atagtattat tccttctagg catag 155
<210> 155
   <211> 158
   <212> DNA
   <213> Homo sapiens
<220>
   <221> Transcript Start Site
   <222> (1)..(58)
<400> 155
   atggggttta gtagggtggg gttattttcg ttaatgttag taagggtggg gaagcgaggt      60
   tgacctgtta gggtgagaag aattattcga gtgctatagg cgcttgtcag ggaggtagcg     120
   atgagagtaa tagatagggc tcaggcgttt gtgtatga                             158
<210> 156
   <211> 204
   <212> DNA
   <213> Homo sapiens
<220>
   <221> Transcript Start Site
   <222> (1)..(104)
<400> 156
   aggttccttt ctgtcctgtc actgtctgtg tgtgcttatg tgtctgtgtg tgtctgtgtc      60
   tttgccacca gaaggagttg ggcctgtttg ctcatgagca gctgtcgagg aggcccactg     120
   tgtaccacat atgcggctcc tgaggtcctg aggtggaagg tccctactgc atccaccacc     180
   ccgcggggca gagaaggagc ccag                                            204
<210> 157
   <211> 115
   <212> DNA
   <213> Homo sapiens
<220>
   <221> Transcript Start Site
   <222> (1)..(15)
<400> 157
   ggctccactc cgccttccac gtgcacccac tgcctcttcc cttctcgctt gggaactcta      60
   gtctcgcctc gggttgcaat ggaccccaac tgctcctgtg ccgctggtaa gggac          115
<210> 158
   <211> 137
   <212> DNA
   <213> Homo sapiens
<220>
   <221> Transcript Start Site
   <222> (1)..(37)
<400> 158
   agtaggttaa tagtgggggg taaggcgagg ttagcgaggc ttgctagaag tcatcaaaaa 60
   gctattagtg ggagtagagt ttgaagtcct tgagagagga ttatgatgcg actgtgagtg 120
   cgttcgtagt ttgagtt 137
<210> 159
   <211> 166
   <212> DNA
   <213> Homo sapiens
<220>
   <221> Transcript Start Site
   <222> (1)..(66)
<400> 159
   ttgtggggaa gagactgata ataaaggtgg atgcgacaat ggattttaca taatgggggt 60
   atgagttttt tttgttaggg ttaacgaggg tggtaaggat ggggggaatt agggaagtca 120
   gggttagggt ggttatagta gtgtgcatgg ttattacttt tatttg 166
<210> 160
   <211> 117
   <212> DNA
   <213> Homo sapiens
<220>
   <221> Transcript Start Site
   <222> (1)..(17)
<400> 160
   agatcaggtt cgtcctttag tgttgtgtat ggctatcatt tgttttgagg ttagtttgat 60
   tagtcattgt tgggtggtga ttagtcggtt gttgatgaga tatttggagg tggggat 117
<210> 161
   <211> 196
   <212> DNA
   <213> Homo sapiens
<220>
   <221> Transcript Start Site
   <222> (1)..(96)
<400> 161
   cgggcggagc cggcgcggcc gcgggccagg cgccgaggtg cgcgcggagc gaggtggccg        60
   cagcgtctcc gcgcgcggcc caagcccggc aggagtgcgg aaccgccgcc tcggccatgc       120
   ggctcccggc cggggggcct gggctggggc ccgcgccgcc ccccgcgctc cgcccccgct      180
   gagcctgagc ccgacc                                                       196
<210> 162
   <211> 134
   <212> DNA
   <213> Homo sapiens
<220>
   <221> Transcript Start Site
   <222> (1)..(34)
<400> 162
   cttcggcaag gtcgaagggg gttcggttgg tctctgctag tgtggagata aatcatatta        60
   tggccaaggg tcatgatggc aggagtaatc agaggtgttc ttgtgttgtg ataagggtgg       120
   agaggttaaa ggag                                                         134
<210> 163
   <211> 111
   <212> DNA
   <213> Homo sapiens
<220>
   <221> Transcript Start Site
   <222> (1)..(11)
<400> 163
   tgtagcttac ctcctcaaag caatacactg aaaatgttta gacgggctca catcacccca        60
   taaacaaata ggtttggtcc tagcctttct attagctctt agtaagatta c                111
<210> 164
   <211> 189
   <212> DNA
   <213> Homo sapiens
<220>
   <221> Transcript Start Site
   <222> (1)..(89)
<400> 164
   cggcggcttc gaagccaaag tgatgtttgg atgtaaagtg aaatattagt tggcggatga 60
   agcagatagt gaggaaagtt gagccaataa tgacgtgaag tccgtggaag cctgtggcta 120
   caaaaaatgt tgagccgtag atgccgtcgg aaatggtgaa gggagactcg aagtactctg 180
   aggcttgta 189
<210> 165
   <211> 180
   <212> DNA
   <213> Homo sapiens
<220>
   <221> Transcript Start Site
   <222> (1)..(80)
<400> 165
   aagtgaagtt ttgcgcccct ccccctccct gcccacctcc tgcagcctcc tgcgccccgc 60
   cgagctggcg gatggagctg cgcagcggga gcgtgggcag ccaggcggtg gcgcggagga 120
   tggatgggga cagccgagat ggcggcggcg gcaaggacgc caccgggtcg gaggactacg 180
<210> 166
   <211> 106
   <212> DNA
   <213> Homo sapiens
<220>
   <221> Transcript Start Site
   <222> (1)..(6)
<400> 166
   gcagaacttc tggacagctg agctggtgaa ggcttgcagg aaggtgaaca agaattcacc 60
   catgtgttgg gatgatggtg caccccaact ccacgggaat agaagc 106
<210> 167
   <211> 120
   <212> DNA
   <213> Homo sapiens
<220>
   <221> Transcript Start Site
   <222> (1)..(20)
<400> 167
   agaggccagc tcagacctcc cggctcgaca ggcggcgcgg gcggcggtga gtgcggcgcg 60
   gggacgccgg ggcgcgggga ccagcgggag acagcggggg gccggtggcg ccagcacctg 120
<210> 168
   <211> 150
   <212> DNA
   <213> Homo sapiens
<220>
   <221> Transcript Start Site
   <222> (1)..(50)
<400> 168
   cctcctcctc ctcctgccct ctcgggctac accaccacca acagtggcgg cggcggcagc        60
   agcggcaaag gccacagcag ggacttcgtc ctccggaggg acctttccgc cacggccccc       120
   gcggcggcca tgcacggggc cccgctcgga                                        150
<210> 169
   <211> 124
   <212> DNA
   <213> Homo sapiens
<220>
   <221> Transcript Start Site
   <222> (1)..(24)
<400> 169
   cagacagtct ggccacagcg tgtttctctg tggccccgtg cggggcgacc ttgctaccca        60
   ggcccacagt aggctgtatg cgaagagcct gcgcctctgc ctccaagaag gcctccaggt       120
   ctgc                                                                    124
<210> 170
   <211> 150
   <212> DNA
   <213> Homo sapiens
<220>
   <221> Transcript Start Site
   <222> (1)..(50)
<400> 170
   gggtgcgctt ccgccgtcgc ctgttcccgc cgcggagacc cggcagttgg gggatgccga        60
   cgcctgggcc ttgaggatgc tgcggaagct caccatcgag cagatcaacg actggttcac       120
   catcggcaag acggtgacca atgtggagct                                        150
<210> 171
   <211> 132
   <212> DNA
   <213> Homo sapiens
<220>
   <221> Transcript Start Site
   <222> (1)..(32)
<400> 171
   gctctactca ccacagcccc tgtccagatg gcagctcccg aggtgacgag gagggtgtag      60
   tcgcggatgt agaaaaatcc tcctaggact gcactcaaga tccccagcac gatctgcatc     120
   acctgaaaga ca                                                         132
<210> 172
   <211> 136
   <212> DNA
   <213> Homo sapiens
<220>
   <221> Transcript Start Site
   <222> (1)..(36)
<400> 172
   gcacagcagg cctgactggg tctcggtgct gtgcctgtcc catggcagag tcggatcagt      60
   gcgtcatgga ggtctcagac cgaagaaact gtggctaccc gggcatcagc cccgaggaat     120
   gcgcctctcg gaagtg                                                     136
<210> 173
   <211> 121
   <212> DNA
   <213> Homo sapiens
<220>
   <221> Transcript Start Site
   <222> (1)..(21)
<400> 173
   ttgcaattca atatgaaaat cacctcggag ctggtaaaaa gaggcctaac ccctgtcttt      60
   agatttacag tccaatgctt cactcagcca ttttacctca cccccactga tgttcgccga     120
   c                                                                     121
<210> 174
   <211> 105
   <212> DNA
   <213> Homo sapiens
<220>
   <221> Transcript Start Site
   <222> (1)..(5)
<400> 174
   cagcaacccg ctcgggtccc tttccatgct gtggaagctc tgttcttttg ctcttcgcaa      60
   taaatcttgc tgctgctcac tctttgggtt tacactacct ttatg                     105
<210> 175
   <211> 136
   <212> DNA
   <213> Homo sapiens
<220>
   <221> Transcript Start Site
   <222> (1)..(36)
<400> 175
   taaaaccttc cttggctcag tgcccttcac cactgctggc tttttgctgt agctccacat 60
   tcctgtgcat tgaggggtaa gtgcattttc cttggatggg ttcttaggtg gtccacaggg 120
   tagtgactgc aggtga 136
<210> 176
   <211> 127
   <212> DNA
   <213> Homo sapiens
<220>
   <221> Transcript Start Site
   <222> (1)..(27)
<400> 176
   gggcgcgctc aggcccggat cctggcggcc tgggcggccg caccatggac tcgggaaccg 60
   aggagtacga gctcaacggc ggcctgcctc cgggcacacc cggctccccg gacgcctcgg 120
   taagacc 127
<210> 177
   <211> 114
   <212> DNA
   <213> Homo sapiens
<220>
   <221> Transcript Start Site
   <222> (1)..(14)
<400> 177
   aaggctacag tgagcaaggc tacctcacca gagagcagag caggagaatg gctgcgagca 60
   acatttctaa caccaatcat cgtaaacaag tccaaggagg cattgacata tatc 114
<210> 178
   <211> 130
   <212> DNA
   <213> Homo sapiens
<220>
   <221> Transcript Start Site
   <222> (1)..(30)
<400> 178
   agttcagtgg acacagtctg tgagcgcctg ccccggtggg tgggaagcag gactcgggcg 60
   ctcatgcagc gagcgggcgg cgctcggggc gctagttcct agcagctggg ccagcgtagg 120
   ggcgcaggtc 130
<210> 179
   <211> 113
   <212> DNA
   <213> Homo sapiens
<220>
   <221> Transcript Start Site
   <222> (1)..(13)
<400> 179
   tgggtgtgcg gtgttctgtc tccgctcccg tttcgctgtc acagcccgtt ccttcccgga 60
   gcccgggaca ggctgggcgc gcgcccgtgt gagtgagcgg gactcagggc aga 113
<210> 180
   <211> 118
   <212> DNA
   <213> Homo sapiens
<220>
   <221> Transcript Start Site
   <222> (1)..(18)
<400> 180
   ccttcactca acactcagca ctcaacacag attgatgaga ggttccctgt gccagcccct 60
   gtgatgcatt ccaaggagac agaaaacagt aagtcccagc ccctactgga ccgatgac 118
<210> 181
   <211> 145
   <212> DNA
   <213> Homo sapiens
<220>
   <221> Transcript Start Site
   <222> (1)..(45)
<400> 181
   gagagactga ctgtatctgg ggattctggc aaggtgaaga gctggtctgt ttggcaggac 60
   cttcccatct gggatccatc tgtgtttccc tggaatggga caggcagctc tagtcagtga 120
   gaaatcgcag atgtgagaga gctga 145
<210> 182
   <211> 114
   <212> DNA
   <213> Homo sapiens
<220>
   <221> Transcript Start Site
   <222> (1)..(14)
<400> 182
   atacctgctg gcacaaacac acaagcccag aacaccaggt gctgttctca acaacgtccg       60
   gcgcttgacc agtgcattga gaaggttggt ccccaataac atctatctca agac            114
<210> 183
   <211> 111
   <212> DNA
   <213> Homo sapiens
<220>
   <221> Transcript Start Site
   <222> (1)..(11)
<400> 183
   tgcttgttca gcgggctact ccgcggggct cgcgtccagt tactatgtgc ccagatgtaa       60
   aggcagcagt cgcttgaaag tcacccatct gcagtggcag tgtgtctttc c               111
<210> 184
   <211> 118
   <212> DNA
   <213> Homo sapiens
<220>
   <221> Transcript Start Site
   <222> (1)..(18)
<400> 184
   ggatctgtgc tacttacgct agccatggga tgattagaac aagataagag acatgaggaa       60
   gacttcttca aggaggtgct caagccatgt tttggaaaag agaactcaag gtctggca        118
<210> 185
   <211> 131
   <212> DNA
   <213> Homo sapiens
<220>
   <221> Transcript Start Site
   <222> (1)..(31)
<400> 185
   cggaggaggt gctggcggcg gcactgagcg gcggcggcgc agggcgcgcg ggccttccgc 60
   gccgactcca tcgacccaag gggcggcggc ggtggcggcg gctgagcgac cctgggccgg 120
   gcgcgtgatg a 131
<210> 186
   <211> 120
   <212> DNA
   <213> Homo sapiens
<220>
   <221> Transcript Start Site
   <222> (1)..(20)
<400> 186
   gctccccaag actgcgagct ccccgcaccc cctcgcactc cctctggccg gcccagggcg 60
   ccttcagccc aacctcccca gccccacggg cgccacggaa cccgctcgat ctcgccgcca 120
<210> 187
   <211> 170
   <212> DNA
   <213> Homo sapiens
<220>
   <221> Transcript Start Site
   <222> (1)..(70)
<400> 187
   gggtggggag gtcgatgaat gagtggttaa ttaattttat tagggggtta attttgcgta 60
   ttggggtcat tggtgttctt gtagttgaaa tacaacgatg gtttttcata tcattggtcg 120
   tggttgtagt ccgtgcgaga ataatgatgt atgctttgtt tctgttgagt 170
<210> 188
   <211> 104
   <212> DNA
   <213> Homo sapiens
<220>
   <221> Transcript Start Site
   <222> (1)..(4)
<400> 188
   gtagggatcc accatctcat ctcgttgcca cctgagacac agccatggct tctctttgta 60
   agtccgtatg aaacatttct aagaaactca atgtgtcagc ttct 104
<210> 189
   <211> 111
   <212> DNA
   <213> Homo sapiens
<220>
   <221> Transcript Start Site
   <222> (1)..(11)
<400> 189
   aggggagtgg aggcagccca ttcacctggg gaaatgactg ggttgtcgat ggacggtggt        60
   gagtgtgctg gtgtgcgtgc ccgggcgtgt gcaatgctgg gtggctctga t                111
<210> 190
   <211> 185
   <212> DNA
   <213> Homo sapiens
<220>
   <221> Transcript Start Site
   <222> (1)..(85)
<400> 190
   tgtggggagg ggtgtttaag gggttggcta gggtataatt gtctgggtcg cctaggaggt        60
   ctggtgagaa tagtgttaat gtcattaagg agagaaggaa gagaagtaag ccgagggcgt       120
   ctttgattgt gtagtaaggg tggaaggtga ttttatcgga atgggaggtg attcctaggg       180
   ggttg                                                                   185
<210> 191
   <211> 117
   <212> DNA
   <213> Homo sapiens
<220>
   <221> Transcript Start Site
   <222> (1)..(17)
<400> 191
   ccggccttca aggcacggct ttttattcct tcggctggtc ggcctctcgc ccttcagcta        60
   cctgtgcgtc cctccgtccc gtcccgtccc ggggtcaccc cggagcctgt ccgctat          117
<210> 192
   <211> 139
   <212> DNA
   <213> Homo sapiens
<220>
   <221> Transcript Start Site
   <222> (1)..(39)
<400> 192
   cttctagtct gtcggtcaga ccaagggcac tcagactgga aaggttgttg ctgggcttgg 60
   gtgtccgtcc tttcctgtct ccttttcttc ctcttttcac ttaaatccac tgagtctctc 120
   gccaagtcag tgatttagg 139
<210> 193
   <211> 117
   <212> DNA
   <213> Homo sapiens
<220>
   <221> Transcript Start Site
   <222> (1)..(17)
<400> 193
   cccgcggaca cacagtgcag gcgcccaagc cgccgccgcc agatcggtgc cgattcctgc 60
   cctgccccga ccgccagcgc gaccatgtcc catcactggg ggtacggcaa acacaac 117
<210> 194
   <211> 109
   <212> DNA
   <213> Homo sapiens
<220>
   <221> Transcript Start Site
   <222> (1)..(9)
<400> 194
   gaatcaggcc attaccgggc tctcctgtgt ctgcataccc tgatgttcgg tgcccagctt 60
   agagaaggag aagggtaaga cgcctgctgg atgcggtagg cttgagcgt 109
<210> 195
   <211> 114
   <212> DNA
   <213> Homo sapiens
<220>
   <221> Transcript Start Site
   <222> (1)..(14)
<400> 195
   gacccggagc cgagagcggg cggcggagcc tgagctggac gcggccacgc cggcgcggcg 60
   gtgagcgcag cccggtctgg cgcctgcagc tgcggggagc gcgccaggca gagg 114
<210> 196
   <211> 114
   <212> DNA
   <213> Homo sapiens
<220>
   <221> Transcript Start Site
   <222> (1)..(14)
<400> 196
   ccatttgatt taaaagaatg aaaaggcgga tctggtcatt cgtctccgcc cggggtctct 60
   gtaaggacgg cggagagagg cgacctccgc ccaataccag gcgcagcctt gccg 114
<210> 197
   <211> 129
   <212> DNA
   <213> Homo sapiens
<220>
   <221> Transcript Start Site
   <222> (1)..(29)
<400> 197
   gtattggagt atttattccc cacatttgct gccagcggcc atgatcatcc tgcctcttaa 60
   tgcacctcac aaataactct agtctacctt tccctttccc gcaggcctcc tcttccagca 120
   gcctctatc 129
<210> 198
   <211> 134
   <212> DNA
   <213> Homo sapiens
<220>
   <221> Transcript Start Site
   <222> (1)..(34)
<400> 198
   gcggggagat ggtgagtcgg ggataaacac tccgcggcgg tggcggctgc tgctctgctc 60
   cgggttctgt cactgtgtcg gcggtgccca gctcactggc cccctccctc tcttgtcgag 120
   cgtggttgcc agag 134
<210> 199
   <211> 122
   <212> DNA
   <213> Homo sapiens
<220>
   <221> Transcript Start Site
   <222> (1)..(22)
<400> 199
   ttcagtgttt tgatctgacg caggcttatg cggaggagaa tgttttcatg ttacttatac 60
   taacattagt tcttctatag ggtgatagat tggtccaatt gggtgtgagg agttcagtta 120
   ta 122
<210> 200
   <211> 124
   <212> DNA
   <213> Homo sapiens
<220>
   <221> Transcript Start Site
   <222> (1)..(24)
<400> 200
   gcagcagcca gccccgcagt cctggacggc gccgggccgc ccaagcgcgc cgaggagagg 60
   gggcggcaaa cttcgggccg agcggcgccg gggcccgcgc gcggagtctc cctccgggca 120
   ccac 124
<210> 201
   <211> 121
   <212> DNA
   <213> Homo sapiens
<220>
   <221> Transcript Start Site
   <222> (1)..(21)
<400> 201
   agcagcctcc tcagatccgt tctctgcgct gccagctcag gtgagccctc gccaaggtga 60
   cctcgcaggt gagcaggcgt ccactttggg gctgctgggt gaagggggtg ggtgcccaag 120
   a 121
<210> 202
   <211> 135
   <212> DNA
   <213> Homo sapiens
<220>
   <221> Transcript Start Site
   <222> (1)..(35)
<400> 202
   catgcaccta attggaagcg ccaccctagc aatatcaacc attaaccttc cctctacact 60
   tatcatcttc acaattctaa ttctactgac tatcctagaa atcgctgtcg ccttaatcca 120
   agcctacgtt ttcac 135
<210> 203
   <211> 122
   <212> DNA
   <213> Homo sapiens
<220>
   <221> Transcript Start Site
   <222> (1)..(22)
<400> 203
   ctcagttaag tctacatgaa aaggatggtt tcttggagct tccacaaact taaaaccatg 60
   aaacatctat tattgctact attgtgtgtt tttctagtta agtcccaagg tgtcaacgac 120
   aa 122
<210> 204
   <211> 155
   <212> DNA
   <213> Homo sapiens
<220>
   <221> Transcript Start Site
   <222> (1)..(55)
<400> 204
   gggttgaggt cttggtgagt gttttagtgg ggttagcgat ggaggtagga ttggtgctgt 60
   gggtgaaaga gtatgatggg gtggtggttg tggtaaactt taatagtgta ggaagctgaa 120
   taatttatga aggagagggg tcagggttga ttcgg 155
<210> 205
   <211> 113
   <212> DNA
   <213> Homo sapiens
<220>
   <221> Transcript Start Site
   <222> (1)..(13)
<400> 205
   tcgcgcgtag atggcagcgg aggcggcggc gcgggccggg gtgaccaggt acgagcgggc 60
   ggggctggcg ggccgagccg ggagtagtgc gggacagtcg ctgcagggag gac 113
<210> 206
   <211> 147
   <212> DNA
   <213> Homo sapiens
<220>
   <221> Transcript Start Site
   <222> (1)..(47)
<400> 206
   gggggggttt ggtggaaatt ttttgttatg atgtctgtgt ggaaagcggc tgtgcagaca 60
   ttcaattgtt attattatgt cctacaagca ttaattaatt aacacacttt agtaggtatg 120
   ttcgcctgta atattgaacg taggtgc 147
<210> 207
   <211> 113
   <212> DNA
   <213> Homo sapiens
<220>
   <221> Transcript Start Site
   <222> (1)..(13)
<400> 207
   gcgccaggtg cggagcgggc gttagaagtt gctggcagtc agaggcaggg gagctgtcac 60
   tcgcggcgag ccgggcggcg gccagggcgc aaagttgaga gcagtctcta gtc 113
<210> 208
   <211> 122
   <212> DNA
   <213> Homo sapiens
<220>
   <221> Transcript Start Site
   <222> (1)..(22)
<400> 208
   gcctccagac tcggtgtgac cgtagccgtg cccggtgctc ccctcgcgga ggcgttaggg 60
   cggcaggttt atctcacttg agaccattcc ttcctttttc atcttctccg ggctccggcg 120
   ca 122
<210> 209
   <211> 120
   <212> DNA
   <213> Homo sapiens
<220>
   <221> Transcript Start Site
   <222> (1)..(20)
<400> 209
   ctccctagtc gtccagacgc tagctgccgc gggcgttcct ggttcgcgct cccgccccct 60
   ccgccccgcc gactcgccgc ttcctccgga gccccagcgt cccgaaccag ggactgcatt 120
<210> 210
   <211> 123
   <212> DNA
   <213> Homo sapiens
<220>
   <221> Transcript Start Site
   <222> (1)..(23)
<400> 210
   gtgaagcttc agggggtttg gatgagaatg gctgttacta cgagggctat gtggctgatt 60
   gaagagtatg caatgagcga ttttaggtct gtttgtcgta ggcagatgga gcttgttata 120
   att 123
<210> 211
   <211> 114
   <212> DNA
   <213> Homo sapiens
<220>
   <221> Transcript Start Site
   <222> (1)..(14)
<400> 211
   cccgcccaag ctgtgtgctg gggaagggcc tgcatggagg ctctctcgtg ggagaggagt 60
   tgcatttcct gtactaagcc tgctcctggc tcaatctctc cccaggctgt gctg 114
<210> 212
   <211> 114
   <212> DNA
   <213> Homo sapiens
<220>
   <221> Transcript Start Site
   <222> (1)..(14)
<400> 212
   cggggagctc gagtcccggg tgactgctgg agggaggagg tgaccgcgca gctctcgtgc 60
   acggacggac ttcgccgccc gcggggggcg cgaacccgag cccgggccca gctc 114
<210> 213
   <211> 111
   <212> DNA
   <213> Homo sapiens
<220>
   <221> Transcript Start Site
   <222> (1)..(11)
<400> 213
   cccacatcct ggcctctcct ctccacggtc gcctgtgccc gggcaccccg gagctgcaaa 60
   ctgcagagcc caggcaaccg ctgggctgtg cgccccgccg gcgccggtag g 111
<210> 214
   <211> 114
   <212> DNA
   <213> Homo sapiens
<220>
   <221> Transcript Start Site
   <222> (1)..(14)
<400> 214
   ggagaagcag cggctggcgt gggccatccg gggaatgggc gccctcgtga cctagtgttg 60
   cggggcaaaa agggtcttgc cggcctcgct cgtgcagggg cgtatctggg cgcc 114
<210> 215
   <211> 129
   <212> DNA
   <213> Homo sapiens
<220>
   <221> Transcript Start Site
   <222> (1)..(29)
<400> 215
   aaatcgctgt cgccttaatc caagcctacg ttttcacact tctagtaagc ctctacctgc 60
   acgacaacac ataatgaccc accaatcaca tgcctatcat atagtaaaac ccagcccatg 120
   acccctaac 129
<210> 216
   <211> 107
   <212> DNA
   <213> Homo sapiens
<220>
   <221> Transcript Start Site
   <222> (1)..(7)
<400> 216
   gcggtaaccc gctggggtcc cgttccacac cgtaagagct ttgttctttt gctctttgca 60
   ataaatcttg ctactgctca ctctttgggt ccacactgct tttatga 107
<210> 217
   <211> 146
   <212> DNA
   <213> Homo sapiens
<220>
   <221> Transcript Start Site
   <222> (1)..(46)
<400> 217
   cagccggcgc ttcccggccg gtgtcgctcc gcggcgggcc atggccaacg tctctaagaa 60
   ggtgtcctgg tccggccggg accgggacga cgaggaggcg gcgccgctgc tgcggaggac 120
   ggcgcggccc ggcgggggga cgccgc 146
<210> 218
   <211> 121
   <212> DNA
   <213> Homo sapiens
<220>
   <221> Transcript Start Site
   <222> (1)..(21)
<400> 218
   tggacagggc tggggagggc gctccaggga agccgaccta gcgtcgattc ctgcagttgc 60
   ggcccgggtc cccgcgcctg gagaggaggc gacgcgtggc cggcggcttt gtggcgcgcg 120
   g 121
<210> 219
   <211> 123
   <212> DNA
   <213> Homo sapiens
<220>
   <221> Transcript Start Site
   <222> (1)..(23)
<400> 219
   gtgagcgcct gtctctttaa atgccacggg ctgcgctccc gcccgggagc ccggagccgg 60
   atctacaatc ccgtccctcc agctccggtg cttgttgctc gcccggccca agcgggagct 120
   gtt 123
<210> 220
   <211> 113
   <212> DNA
   <213> Homo sapiens
<220>
   <221> Transcript Start Site
   <222> (1)..(13)
<400> 220
   aagcccagaa caccaggtgc tgttctcaac aacgtccggc gcttgaccag tgcattgaga 60
   aggttggtcc ccaataacat ctatctcaag accactttcc acagcctttt aat 113
<210> 221
   <211> 114
   <212> DNA
   <213> Homo sapiens
<220>
   <221> Transcript Start Site
   <222> (1)..(14)
<400> 221
   aaagcattgc gcatgcggga aacccacgct gagggaagaa tcatgggaaa gaggtgagcc 60
   tgtaaagttt taggatcaag gttaaaggcc cttttttgct ctcttctctc ttgg 114
<210> 222
   <211> 111
   <212> DNA
   <213> Homo sapiens
<220>
   <221> Transcript Start Site
   <222> (1)..(11)
<400> 222
   agaacactct gagcctcagt ctccttcaat ggaggcactg gacagcagga agtcagagaa 60
   gactttcagt gctaactgct cagtttttat caaagcaact gtgaattgta g 111
<210> 223
   <211> 116
   <212> DNA
   <213> Homo sapiens
<220>
   <221> Transcript Start Site
   <222> (1)..(16)
<400> 223
   ttttttctca ctttgccttg tgttttccac cctgaaagaa tgttgtggct gctctttttt 60
   ctggtgactg ccattcatgc tgaactctgt caaccaggta tgtagccttg aaattt 116
<210> 224
   <211> 117
   <212> DNA
   <213> Homo sapiens
<220>
   <221> Transcript Start Site
   <222> (1)..(17)
<400> 224
   aacccgcttt ctgggcacgg acaagagcgc ggggtagatc gctctgcgga gccttcacgg 60
   ggctcttccc gctcggaggg ccggtgctgc agccgggtca ggggtgcgcg ggggaaa 117
<210> 225
   <211> 114
   <212> DNA
   <213> Homo sapiens
<220>
   <221> Transcript Start Site
   <222> (1)..(14)
<400> 225
   accttgtaca atagagctta taggctaatg agaagacagg aagaaacaac aggaacacat 60
   atagctggaa tgcagatggg atctgtgcta cttacgctag ccatgggatg atta 114
<210> 226
   <211> 118
   <212> DNA
   <213> Homo sapiens
<220>
   <221> Transcript Start Site
   <222> (1)..(18)
<400> 226
   cgggacaaag cagtggctat cctgctgctg gctgcatcac tgcttgttca gcgggctact 60
   ccgcggggct cgcgtccagt tactatgtgc ccagatgtaa aggcagcagt cgcttgaa 118
<210> 227
   <211> 118
   <212> DNA
   <213> Homo sapiens
<220>
   <221> Transcript Start Site
   <222> (1)..(18)
<400> 227
   cgggtgccag atactgggat cagccactgc agctccctga gcactctcta cagagacgcg 60
   gaccccagac atgaggaggt aatgccacca tccctgagcc ccaatcccca ccccaccc 118
<210> 228
   <211> 166
   <212> DNA
   <213> Homo sapiens
<220>
   <221> Transcript Start Site
   <222> (1)..(66)
<400> 228
   tgggggaggt tatatgggtt taatagtttt tttaatttat ttagggggaa tgatggttgt 60
   ctttggatat actacagcga tggctattga ggagtatcct gaggcatggg ggtcaggggt 120
   tgaggtcttg gtgagtgttt tagtggggtt agcgatggag gtagga 166
<210> 229
   <211> 107
   <212> DNA
   <213> Homo sapiens
<220>
   <221> Transcript Start Site
   <222> (1)..(7)
<400> 229
   actcataaac taataagcaa gacttggaga cgatgcagga tgccagttca agaccctagg 60
   taaatgagct gatcttgaac agggggaaga accaactgtt ctaaagc 107
<210> 230
   <211> 113
   <212> DNA
   <213> Homo sapiens
<220>
   <221> Transcript Start Site
   <222> (1)..(13)
<400> 230
   tgaggcagaa tcgttcgaat ccaggaagtg gaggctgcag tgagttgaga tcttgccatt 60
   gcactccagc ctgggcaaca agagcgaaac tccatctcaa ggaaaaacaa caa 113
<210> 231
   <211> 115
   <212> DNA
   <213> Homo sapiens
<220>
   <221> Transcript Start Site
   <222> (1)..(15)
<400> 231
   gctggagtca gataaatttg aaatgagaga tttgaaatat cattgctcgg aggggccaca        60
   tggaaagcat gagaatggaa tatgggcagc taggagcaaa gactggatcc cactt            115
<210> 232
   <211> 121
   <212> DNA
   <213> Homo sapiens
<220>
   <221> Transcript Start Site
   <222> (1)..(21)
<400> 232
   tgcgggggaa atgttgttag taatgagaaa tcctgcgaat aggcttccgg ctgccaggcg        60
   tttaatgggg tttagtaggg tggggttatt ttcgttaatg ttagtaaggg tggggaagcg       120
   a                                                                       121
<210> 233
   <211> 112
   <212> DNA
   <213> Homo sapiens
<220>
   <221> Transcript Start Site
   <222> (1)..(12)
<400> 233
   cctggagcag aggctgcttc tccgcccaag cactccctgg gtgagtccat actcctggcg        60
   cgagtacgat cctctcctcc agggaaggcc gggtccctcg ctggggttcg ac              112
<210> 234
   <211> 145
   <212> DNA
   <213> Homo sapiens
<220>
   <221> Transcript Start Site
   <222> (1)..(45)
<400> 234
   tgcttgaggt gtgtcagggc ctctgcttag aactcgcgca attctgaacg gaggaggaga        60
   aagtccagtt gctgaagggc agaatttcca tgcgcccgca gcttccaagt ccagaccacc       120
   cccgaatgta agtaggaggc agaga                                             145
<210> 235
   <211> 188
   <212> DNA
   <213> Homo sapiens
<220>
   <221> Transcript Start Site
   <222> (1)..(88)
<400> 235
   ccggacgtct aaaccaaacc actttcaccg ctacacgacc gggggtatac tacggtcaat      60
   gctctgaaat ctgtggagca aaccacagtt tcatgcccat cgtcctagaa ttaattcccc     120
   taaaaatctt tgaaataggg cccgtattta ccctatagca ccccctctac cccctctaga     180
   gcccactg                                                              188
<210> 236
   <211> 139
   <212> DNA
   <213> Homo sapiens
<220>
   <221> Transcript Start Site
   <222> (1)..(39)
<400> 236
   gcccttccag tttcccatac cttcgtgttg agttgctcag tctctggctt agaggaccag      60
   gaatgggaaa tccccagctg taggctggag cgtgcaacac tgcggcccac ctcgctgcgg     120
   ttggtggtgg caacagaga                                                  139
<210> 237
   <211> 160
   <212> DNA
   <213> Homo sapiens
<220>
   <221> Transcript Start Site
   <222> (1)..(60)
<400> 237
   tcaagggtag ctgggactga agtccctatg acctttccac tagacgctag cattctggag      60
   gacagttggg acttggtaag gtcagaagct ctccctgtct tcctttgtat tttccctttc     120
   ttctttcctg ttctgcattt ttcactcaca atctcttttc                           160
<210> 238
   <211> 113
   <212> DNA
   <213> Homo sapiens
<220>
   <221> Transcript Start Site
   <222> (1)..(13)
<400> 238
   ccaaccagtg ccctgagttc tgaggcagag aggaggacag aagaaacaag aggctggaga 60
   ttgtcaaatt cagtatccca gttggctctt gattcttggt gaaaccatcc ctc 113
<210> 239
   <211> 118
   <212> DNA
   <213> Homo sapiens
<220>
   <221> Transcript Start Site
   <222> (1)..(18)
<400> 239
   cagagccatt taacctttgg gaacaaggca actagcgtct ggcagcagga atccaaccag 60
   tgccctgagt tctgaggcag agaggaggac agaagaaaca agaggctgga gattgtca 118
<210> 240
   <211> 122
   <212> DNA
   <213> Homo sapiens
<220>
   <221> Transcript Start Site
   <222> (1)..(22)
<400> 240
   gcgtgttcat cagtcgctgt ttgggacgct gggtgtgcgg tgttctgtct ccgctcccgt 60
   ttcgctgtca cagcccgttc cttcccggag cccgggacag gctgggcgcg cgcccgtgtg 120
   ag 122
<210> 241
   <211> 142
   <212> DNA
   <213> Homo sapiens
<220>
   <221> Transcript Start Site
   <222> (1)..(42)
<400> 241
   caggacagac aattcctcag tgacccagga gctgacacac tatggcgcac gtccgaggct 60
   tgcagctgcc tggctgcctg gccctggctg ccctgtgtag ccttgtgcac agccagcatg 120
   gtaagggagt gcttgcaggc tg 142
<210> 242
   <211> 113
   <212> DNA
   <213> Homo sapiens
<220>
   <221> Transcript Start Site
   <222> (1)..(13)
<400> 242
   gcttgctttc cttccacgct gtaaggacac aatgagaagc tggcagtctt caacctgaac 60
   caaccatgct ggcaccctga tctttaactt ctagcttcca gaactgtgag aaa 113
<210> 243
   <211> 136
   <212> DNA
   <213> Homo sapiens
<220>
   <221> Transcript Start Site
   <222> (1)..(36)
<400> 243
   tcagccactg cagctccctg agcactctct acagagacgc ggaccccaga catgaggagg 60
   taatgccacc atccctgagc cccaatcccc accccaccca aacctggagg cctggccctt 120
   agccagaggg ggccca 136
<210> 244
   <211> 121
   <212> DNA
   <213> Homo sapiens
<220>
   <221> Transcript Start Site
   <222> (1)..(21)
<400> 244
   cctttaatac actttggttc tccgcctggc tttggactct tctcctcctc cacctcctcc 60
   tcctcctccc gcgccgccgc ctcctcctcc tcttcctctc cgcgccttcg ctacgcgccc 120
   g 121
<210> 245
   <211> 171
   <212> DNA
   <213> Homo sapiens
<220>
   <221> Transcript Start Site
   <222> (1)..(71)
<400> 245
   agcgcgcagg cgcaggtccc agtaaccgcc ggttggaggc ggccgaaccg cagtagggaa        60
   agacccaggc tgcgggacgc ggtgcaggct gcggcgctga cggcctctgc tccttccgcg       120
   ggtttccgac tccctgccct agattttctg cttagcgact tggggtcccc t                171
<210> 246
   <211> 105
   <212> DNA
   <213> Homo sapiens
<220>
   <221> Transcript Start Site
   <222> (1)..(5)
<400> 246
   gcaacccact caggttccct tccacgctgt agaagctctg ttctttcact cttcgcaata        60
   aatcttgctg ctgctcactc tttgggtccg caccaccttt atgag                       105
<210> 247
   <211> 106
   <212> DNA
   <213> Homo sapiens
<220>
   <221> Transcript Start Site
   <222> (1)..(6)
<400> 247
   tggcaaccca ctcacgtccc cttccatgct atggaagctc tgttcttttg ctctttgcaa        60
   taaatcttgc tgctgctcac tctttgggtc cacactgcct ttatga                      106
<210> 248
   <211> 112
   <212> DNA
   <213> Homo sapiens
<220>
   <221> Transcript Start Site
   <222> (1)..(12)
<400> 248
   cccctggcta cccacctttg tgcccgggtt caccaccact ggtttctgcg ttccctggac        60
   tctgccctgc acgggccgca cctcccccgc cggccctcgc ccatgctcca gc               112
<210> 249
   <211> 111
   <212> DNA
   <213> Homo sapiens
<220>
   <221> Transcript Start Site
   <222> (1)..(11)
<400> 249
   accccagacc ggttagatca caagtggaga agctggggcc ccagcattgg cagttgttaa       60
   agctccctgg gtggacccac cgtgcggaag gtgggagaag cagcaaacgc a               111
<210> 250
   <211> 114
   <212> DNA
   <213> Homo sapiens
<220>
   <221> Transcript Start Site
   <222> (1)..(14)
<400> 250
   gtggtcagag agaggtaggg gcactcagag atccagcagg tgctgcacca tgagtgtctc       60
   tgtgctgagc cccagcagac tcctgggtga tgtctctgga atcctccaag cggc            114
<210> 251
   <211> 109
   <212> DNA
   <213> Homo sapiens
<220>
   <221> Transcript Start Site
   <222> (1)..(9)
<400> 251
   cggccatgat catcctgcct cttaatgcac ctcacaaata actctagtct acctttccct       60
   ttcccgcagg cctcctcttc cagcagcctc tatctttatt ctacaggat                  109
<210> 252
   <211> 134
   <212> DNA
   <213> Homo sapiens
<220>
   <221> Transcript Start Site
   <222> (1)..(34)
<400> 252
   atctgtgaac acgtattgag aggctggata aggctgcgcc catgtgagtg ctgggcttgt 60
   acgtgcattt ttgcctgagt gagcattagt ggcagtgtcc ccagcctacc cctttcctga 120
   atcccaggct cata 134
<210> 253
   <211> 121
   <212> DNA
   <213> Homo sapiens
<220>
   <221> Transcript Start Site
   <222> (1)..(21)
<400> 253
   cccgcagttg tcttactaag aagagaagcc ttcaatggat ccagctgtgg ctctggtgct 60
   ctgtctctcc tgtttgtttc tcctttcact ctggaggcag agctctggaa gagggaggct 120
   c 121
<210> 254
   <211> 107
   <212> DNA
   <213> Homo sapiens
<220>
   <221> Transcript Start Site
   <222> (1)..(7)
<400> 254
   tagcacagat tcctccttgg gctgcagtga gtatgtctaa gaccatacag ttttgtaata 60
   cagtcttctc atgacagtaa cttcattatt taataatgag acattca 107
<210> 255
   <211> 132
   <212> DNA
   <213> Homo sapiens
<220>
   <221> Transcript Start Site
   <222> (1)..(32)
<400> 255
   gggttggaga ccggaggagt agacggcaga ggcgcccccg caggtaggcg caggtgggtc 60
   cggagggtcc tggccgacgc gggtgggggt cgctcgccgc agttcctggg gcgctggttc 120
   agcgcgggcc tc 132
<210> 256
   <211> 118
   <212> DNA
   <213> Homo sapiens
<220>
   <221> Transcript Start Site
   <222> (1)..(18)
<400> 256
   tttggccact ctgcaagaag gcaatcctct aaggatcacc agcttctacc tataagactt 60
   caactcaaga tgcaggcaag taaatgatat gtatataata ttagactatt ccatgcag 118
<210> 257
   <211> 116
   <212> DNA
   <213> Homo sapiens
<220>
   <221> Transcript Start Site
   <222> (1)..(16)
<400> 257
   agtggcagca atcctttctt tcagctggag tgctcctcag gagccagccc cacccttaga 60
   aaagatgttt tccatgagga tcgtctgcct ggtcctaagt gtggtgggca cagcat 116
<210> 258
   <211> 154
   <212> DNA
   <213> Homo sapiens
<220>
   <221> Transcript Start Site
   <222> (1)..(54)
<400> 258
   agccagcagt caccggcctc acccagccac cctgccgcca ttaacagcac taggcaggca 60
   gtgcgcagag ctgagaactt gccccaggtt acccgcctcc cagctgctaa acaaaaggga 120
   ggcttttcag ctggttcaaa ttgttacaaa gttc 154
<210> 259
   <211> 104
   <212> DNA
   <213> Homo sapiens
<220>
   <221> Transcript Start Site
   <222> (1)..(4)
<400> 259
   ccaacctgct ctgatcccct tccgctgtgg gaggtttgtt cttttgctct ttgcaataaa 60
   tcttgctgct gctcactcct tgggtccgca ctacctttat aagc 104
<210> 260
   <211> 184
   <212> DNA
   <213> Homo sapiens
<220>
   <221> Transcript Start Site
   <222> (1)..(84)
<400> 260
   ccggggggag ggccggggct gcgagagagg aagctctttc gcggcgctac ggcgttggca 60
   ccagtctcta gaaaagaagt cagctctggt tcggagaagc agcggctggc gtgggccatc 120
   cggggaatgg gcgccctcgt gacctagtgt tgcggggcaa aaagggtctt gccggcctcg 180
   ctcg 184
<210> 261
   <211> 127
   <212> DNA
   <213> Homo sapiens
<220>
   <221> Transcript Start Site
   <222> (1)..(27)
<400> 261
   gcgtcgattc ctgcagttgc ggcccgggtc cccgcgcctg gagaggaggc gacgcgtggc 60
   cggcggcttt gtggcgcgcg ggttccagcg cgggggcggg gagagggcga gctgagggcc 120
   tcgagtc 127
<210> 262
   <211> 121
   <212> DNA
   <213> Homo sapiens
<220>
   <221> Transcript Start Site
   <222> (1)..(21)
<400> 262
   cctgcagttg tctcacagaa gacggatcgg gaggcctgag gaggccgctc tttcctgata 60
   cgaggttaca gctggggtct gatgtcaggt ggggggcatg gtggggtctg ctttctctgc 120
   t 121
<210> 263
   <211> 116
   <212> DNA
   <213> Homo sapiens
<220>
   <221> Transcript Start Site
   <222> (1)..(16)
<400> 263
   acatcttctt gtatttgaat atccgttaac tgtacgcata gtggtcctac ttggtggttt 60
   ggaaaacagt gattctgtat ttggtggatg gtttaaagta tactgaagtg tctaac 116
<210> 264
   <211> 125
   <212> DNA
   <213> Homo sapiens
<220>
   <221> Transcript Start Site
   <222> (1)..(25)
<400> 264
   acggaggagg agaaagtcca gttgctgaag ggcagaattt ccatgcgccc gcagcttcca 60
   agtccagacc acccccgaat gtaagtagga ggcagagatg tgtgcacctt cggttctgtc 120
   ttggg 125
<210> 265
   <211> 113
   <212> DNA
   <213> Homo sapiens
<220>
   <221> Transcript Start Site
   <222> (1)..(13)
<400> 265
   ccgaggcaga tccaggcggc ggcggaggcg gcgggcgcag gagagcggct cccagggctg 60
   aagtggccgc caccaccgcc gcctgcgcct ggagcccggt ggccgccgga cgc 113
<210> 266
   <211> 120
   <212> DNA
   <213> Homo sapiens
<220>
   <221> Transcript Start Site
   <222> (1)..(20)
<400> 266
   gcccagagga acggcctgcg gagccaggta tacgctgtga atggcgacta ctatgtgggc      60
   gagtggaagg acaacgtgaa acacggtgag tgggactccg ggacgggcgg ccggagtggg     120
<210> 267
   <211> 105
   <212> DNA
   <213> Homo sapiens
<220>
   <221> Transcript Start Site
   <222> (1)..(5)
<400> 267
   tggcaatctg ctagtatagt gatttgagac tgtaaagggt ttgttctttt gctctttgca      60
   attaaatctt gtgagtgttc actgtttgga tccccagtgc tttta                     105
<210> 268
   <211> 104
   <212> DNA
   <213> Homo sapiens
<220>
   <221> Transcript Start Site
   <222> (1)..(4)
<400> 268
   ttgtactcct ctctttctct aggagaagat catgtttcaa atgctgttgg ttcaaagagg      60
   atgacagaca tgtagagcct atctgaatcc agctcagtca acct                      104
<210> 269
   <211> 105
   <212> DNA
   <213> Homo sapiens
<220>
   <221> Transcript Start Site
   <222> (1)..(5)
<400> 269
   tggcaacctg ttagggtctg cttttgtcct ctgggagctt tccttgtttg gttttcgtag 60
   taaatcttac tgctgctcag tgtttgtgtc tgtgctgcgt ttgtg 105
<210> 270
   <211> 113
   <212> DNA
   <213> Homo sapiens
<220>
   <221> Transcript Start Site
   <222> (1)..(13)
<400> 270
   ccccgctctg cgcggccgtt ctagaggtcc gggtgggagc tggaggagga aagcgggagg 60
   aggcggcgag gccgcggggc ggtatgaggg ggcgccggcc gcctctcgga ttg 113
<210> 271
   <211> 104
   <212> DNA
   <213> Homo sapiens
<220>
   <221> Transcript Start Site
   <222> (1)..(4)
<400> 271
   cagatcttcg cagagcggag cagcggccgg agcgtttggc ggactctgcg tggacttgga 60
   gctcacagcg tcttgcgact tggaagcgga ttcagaggac agga 104
<210> 272
   <211> 103
   <212> DNA
   <213> Homo sapiens
<220>
   <221> Transcript Start Site
   <222> (1)..(3)
<400> 272
   tggcaaccca cttgggttcc ctgcgatgtt gtggaagggt tgttgttttg ctgtttccag 60
   taaatgttgg tgttgctcag tttttgggtc cacactgtgt tta 103
<210> 273
   <211> 106
   <212> DNA
   <213> Homo sapiens
<220>
   <221> Transcript Start Site
   <222> (1)..(6)
<400> 273
   cattgcagat tctttagtgt ttgcctcctc agcggctctt gcccggctta gaggaggaag 60
   aggcgaaagg aaagacacct ccctcactct ggcctaaggc accacg 106
<210> 274
   <211> 134
   <212> DNA
   <213> Homo sapiens
<220>
   <221> Transcript Start Site
   <222> (1)..(34)
<400> 274
   ggccaaaact cttcggaatc agcagccaga gaggctctga gggcgcccgc ggggcggagg 60
   ggagcggggc agagggccgc cggggcgagg gccgaggagg ggtccgagac gcgccaaggg 120
   gctggtagct aggc 134
<210> 275
   <211> 106
   <212> DNA
   <213> Homo sapiens
<220>
   <221> Transcript Start Site
   <222> (1)..(6)
<400> 275
   tgacatcccg ctctggtcat tttccataga gtggaaagtt tgttatttcc gtttttgcaa 60
   taaattttat tgctatttgt tctttgggtc cacactactt ttatga 106
<210> 276
   <211> 132
   <212> DNA
   <213> Homo sapiens
<220>
   <221> Transcript Start Site
   <222> (1)..(32)
<400> 276
   gggaagggct aagggagcca cagaacctca gtggatctca gagagagccc cagactgagg 60
   gaagcatgga tggatggaga aggatgcctc gctggggact gctgctgctg ctctggggct 120
   cctgtacctt tg 132
<210> 277
   <211> 190
   <212> DNA
   <213> Homo sapiens
<220>
   <221> Transcript Start Site
   <222> (1)..(90)
<400> 277
   gaggcacgtg gaccagaaat acttcctaga aacagcaagt acgctgctca accctgcctg 60
   aactttcctg taaacagcca ctgacagact gcatccctgc tgggtggatc ctgcgactcc 120
   tagagggaat ggcacttctt gtttttaatt agcaaggtga aaggtgaatt aaaactagca 180
   gtttggcaag 190
<210> 278
   <211> 20
   <212> DNA
   <213> Artificial Sequence
<220>
   <223> Synthetic oligonucleotide
<400> 278
   ccagttgctg aagggcagaa 20
<210> 279
   <211> 19
   <212> DNA
   <213> Artificial Sequence
<220>
   <223> Synthetic oligonucleotide
<400> 279
   gcggaccttg gagtctgag 19